(19) Europäisches Patentamt
European Patent Office
Office européen des brevets

(11) **EP 2 488 479 B1**

(12) **EUROPÄISCHE PATENTSCHRIFT**

(45) Veröffentlichungstag und Bekanntmachung des Hinweises auf die Patenterteilung:
**04.09.2013 Patentblatt 2013/36**

(21) Anmeldenummer: **10768740.2**

(22) Anmeldetag: **14.10.2010**

(51) Int Cl.:
**C07C 57/07** *(2006.01)*

(86) Internationale Anmeldenummer:
**PCT/EP2010/065373**

(87) Internationale Veröffentlichungsnummer:
**WO 2011/045356 (21.04.2011 Gazette 2011/16)**

(54) **VERFAHREN DER INBETRIEBNAHME EINES TRENNVERFAHRENS ZUR REINIGENDEN ABTRENNUNG VON ACRYLSÄUREKRISTALLEN AUS EINER SUSPENSION S IHRER KRISTALLE IN MUTTERLAUGE**

PROCESS FOR STARTING UP A SEPARATING PROCESS FOR PURIFICATIVE REMOVAL OF ACRYLIC ACID CRYSTALS FROM A SUSPENSION S OF CRYSTALS THEREOF IN MOTHER LIQUOR

PROCÉDÉ POUR LA MISE EN OEUVRE D'UN PROCÉDÉ DE SÉPARATION DESTINÉ À LA SÉPARATION LAVANTE DE CRISTAUX D'ACIDE ACRYLIQUE CONTENUS DANS UNE SUSPENSION S DE CRISTAUX D'ACIDE ACRYLIQUE DANS UNE LESSIVE MÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.10.2009 DE 102009045767**
**18.06.2010 DE 102010030279**
**18.06.2010 US 356078 P**
**16.10.2009 US 252181 P**

(43) Veröffentlichungstag der Anmeldung:
**22.08.2012 Patentblatt 2012/34**

(73) Patentinhaber: **BASF SE**
**67056 Ludwigshafen (DE)**

(72) Erfinder:
• **HEILEK, Jörg**
**69245 Bammental (DE)**
• **LITTERS, Dirk**
**68623 Lampertheim (DE)**
• **MÜLLER-ENGEL, Klaus Joachim**
**76297 Stutensee (DE)**

(56) Entgegenhaltungen:
**WO-A1-01/77056      WO-A2-2006/111565**

**Beschreibung**

Vorliegende Erfindung betrifft ein Verfahren der Inbetriebnahme eines Trennverfahrens zur reinigenden Abtrennung von Acrylsäurekristallen aus einer Suspension S ihrer Kristalle in Mutterlauge mit einer Vorrichtung, die eine hydraulische Waschkolonne umfasst, die einen zu seiner von oben nach unten verlaufenden Längsachse rotationssymmetrischen Prozessraum aufweist, der von einer zylindrischen Mantelwand und zwei auf der Symmetrieachse einander gegenüberliegenden Enden begrenzt wird, wobei

- sich vom oberen Ende des Prozessraums parallel zu dessen Längsachse ein oder mehrere Filterrohre durch den Prozessraum erstrecken, die auf das dem oberen Ende gegenüberliegende untere Ende des Prozessraums zulaufen, und in der dem unteren Ende des Prozessraums zugewandten Hälfte des Prozessraums wenigstens ein Filter F, das die einzige direkte Verbindung zwischen dem jeweiligen Filterrohrinneren und dem Prozessraum bildet, aufweisen sowie außerhalb des Prozessraums aus der Waschkolonne herausgeführt werden,

- der Quotient Q= L/D aus dem Abstand L zwischen dem oberen und dem unteren Ende des Prozessraums und dem Durchmesser D des Prozessraums 0,3 bis 4 beträgt,

- sich am unteren Ende des Prozessraums nach unten gerichtet der Kristallschmelzeraum der Waschkolonne anschließt, wobei zwischen den beiden Räumen eine rotationsfähige Abtrageinrichtung integriert und durch den Kristallschmelzeraum ein Kristallschmelzekreis hindurchgeführt ist, der außer dem Kristallschmelzeraum

    - eine außerhalb der Waschkolonne befindliche Förderpumpe P1, die eine Saug- und eine Druckseite aufweist,

    - eine erste Förderverbindung G1, die vom Kristallschmelzeraum der Waschkolonne zur Saugseite der Förderpumpe P1 führt,

    - eine zweite Förderverbindung G2, die von der Druckseite der Förderpumpe P1 in den Kristallschmelzeraum der Waschkolonne zurückführt und einen Auslass A aus dem Kristallschmelzekreis mit regelbarem Durchfluss aufweist, sowie,

    - einen Wärmeübertrager W, über den entweder die Förderverbindung G1 vom Kristallschmelzeraum zur Saugseite der Förderpumpen P1, oder die Förderverbindung G2 von der Druckseite der Förderpumpe P1 zum Kristallschmelzeraum geführt ist, umfasst,

- dem oberen Ende des Prozessraums nach oben gerichtet ein Verteilerraum vorgelagert ist, der vom Prozessraum wenigstens durch einen Boden B getrennt ist, welcher Durchgänge U aufweist, die auf der dem Prozessraum zugewandten Seite des Bodens B in den Prozessraum und auf der vom Prozessraum abgewandten Seite des Bodens B in den Verteilerraum führen,

- sich außerhalb der Waschkolonne eine Förderpumpe P2, die eine Saug- und eine Druckseite aufweist, und eine Quelle QS der Suspension S befinden, wobei

    - eine erste Förderverbindung E1 von der Quelle QS zur Saugseite der Förderpumpe P2, und

    - eine zweite Förderverbindung E2 von der Druckseite der Förderpumpe P2 in den Verteilerraum führt,

- sich außerhalb der Waschkolonne gegebenenfalls eine Förderpumpe P3, die eine Saug- und eine Druckseite aufweist, und eine Quelle QT einer Steuerlauge befinden, wobei

    - eine erste Förderverbindung C1 von der Saugseite der Pumpe P3 zur Quelle QT, und

    - eine zweite Förderverbindung C2 von der Druckseite der Pumpe P3 in den Verteilerraum und/oder in den zwischen seinem oberen Ende und den Filtern F der Filterrohre gelegenen Längsabschnitt des Prozessraums führt,

und wobei man bei der Durchführung des Trennverfahrens in seinem stationären Betrieb

- mit der Pumpe P2 kontinuierlich einen Strom ST der Suspension S von der Quelle QS durch die Förderverbindungen E1, E2 über den Verteilerraum und durch die Durchgänge U hindurch in den Prozessraum der Waschkolonne führt,

- gegebenenfalls mit der Pumpe P3 einen Strom SL der Steuerlauge von der Quelle QT durch die Förderverbindungen C1, C2 über den Verteilerraum und die Durchgänge U hindurch und/oder direkt in den Prozessraum der Waschkolonne führt,

- über die Filter F der Filterrohre insgesamt einen Strom SM umfassend Mutterlauge und gegebenenfalls Steuerlauge als Ablaugestrom in das Filterrohrinnere hinein- und über die Filterrohre aus der Waschkolonne herausführt, und diesen aus der Waschkolonne herausgeführten Ablaugestrom SM als die Quelle QT für die Steuerlauge verwendet,

- durch die Mutter- und gegebenenfalls Steuerlaugeführung im Prozessraum der Waschkolonne die Ausbildung eines Kristallbetts an Acrylsäurekristallen aufrechterhält, das eine dem oberen Ende des Prozessraums zugewandte Aufbaufront (die Aufbaufront bezeichnet den Übergang von der Kristallsuspension zum (verdichteten) Kristallbett und ist durch einen relativ abrupten Anstieg des Kristallgehalts je Volumeneinheit gekennzeichnet) aufweist, an der sich kontinuierlich Kristalle des zugeführten Stroms ST der Suspension S (des Suspensionsstroms) ans Kristallbett anlagern,

- das Kristallbett durch die aus dem hydraulischen Strömungsdruckverlust der Mutter- und gegebenenfalls Steuerlaugeführung im Prozessraum resultierende Kraft von oben nach unten an den Filtern F vorbei auf die rotierende Abtrageinrichtung zuführt,

- mit der rotierenden Abtrageinrichtung vom auf sie stoßenden Kristallbett Acrylsäurekristalle abträgt,

- den Strom der abgetragenen Acrylsäurekristalle durch die rotierende Abtrageinrichtung hindurch und/oder an der rotierenden Abtrageinrichtung vorbei in den sich in Förderrichtung des Kristallbetts an den Prozessraum anschließenden Kristallschmelzeraum führt, und in dem durch den Kristallschmelzeraum geführten Kristallschmelzekreis (vereinfacht häufig auch nur "Schmelzkreis") durch Eintrag von Wärme mit dem Wärmeübertrager W zu einem Kristallschmelzestrom aufschmilzt, und

- den Durchfluss des Auslasses A so regelt, dass, bezogen auf die Stärke des vorgenannten Kristallschmelzestroms, vom Kristallschmelzeraum ausgehend ein Teilstrom an Kristallschmelze als Waschschmelzestrom durch die rotierende Abtrageinrichtung hindurch und/oder an der rotierenden Abtrageinrichtung vorbei gegen die Bewegungsrichtung des Kristallbetts in den Prozessraum zurückströmt, wo er im abwärts geförderten Kristallbett aufsteigt und dabei von den Kristallen im Kristallbett verbliebene und mit diesem unter die Filter F geförderte Mutterlauge abwäscht und zurückdrängt, wobei sich im von den Filtern F bis zum unteren Ende des Prozessraums erstreckenden Längsabschnitt des Prozessraums im Kristallbett eine Waschfront ausbildet, die das Kristallbett von oben nach unten in eine Mutterlaugezone und in eine Waschschmelzezone aufteilt, und der restliche Teilstrom des vorgenannten Kristallschmelzestroms den Kristallschmelzekreis durch den Auslass A verlässt.

Acrylsäure, entweder für sich oder in Form ihrer Salze oder ihrer Ester, ist insbesondere zur Herstellung von Polymerisaten für die verschiedensten Anwendungsgebiete (z.B. Klebstoffe, Superabsorber, Bindemittel) von Bedeutung.

Bei der Synthese von Acrylsäure fällt selbige üblicherweise nicht als Reinprodukt an, sondern als Teil eines Stoffgemisches, das neben der in hoher Reinheit erwünschten Zielverbindung noch unerwünschte Bestandteile wie z.B. Lösungsmittel, Ausgangsverbindungen und Nebenprodukte enthält. Häufig handelt es sich dabei bei dem Stoffgemisch um eine Flüssigkeit.

Beispielsweise ist Acrylsäure durch katalytische Gasphasenoxidation von Glyzerin, Propan, Propen und/oder Acrolein erhältlich. Dabei werden diese Ausgangsverbindungen in der Gasphase, in der Regel mit inerten Gasen wie molekularem Stickstoff, $CO_2$ und/oder Wasserdampf verdünnt, im Gemisch mit molekularem Sauerstoff bei erhöhten Temperaturen sowie gegebenenfalls erhöhtem Druck über übergangsmetallische Mischoxidkatalysatoren geleitet und oxidativ in ein Acrylsäure enthaltendes Produktgasgemisch umgewandelt.

Durch kondensative und/oder absorptive Maßnahmen wird die Acrylsäure nachfolgend üblicherweise in die flüssige (kondensierte) Phase überführt, im Rahmen derer bereits eine Grundabtrennung der Acrylsäure von den sie im Produktgasgemisch begleitenden Verbindungen erzielt wird.

Unter Anwendung unterschiedlichster Kombinationen von thermischen Trennverfahren (als solche kommen z.B. die Rektifikation, die Extraktion, das Strippen, die Destillation, die Desorption etc. in Betracht) wird die Acrylsäure schließlich in hoher Reinheit aus den vorgenannten flüssigen Phasen abgetrennt. Bestandteil solcher Verfahrenskombinationen ist vielfach das Verfahren der Suspensionkristallisation.

Kühlt man ein in seinem flüssigen Aggregatzustand befindliches, Acrylsäure enthaltendes Stoffgemisch ab, und bewirkt dabei die Ausbildung von Kristallen der Acrylsäure, so ist die Suspensionskristallisation ein mögliches Verfahren, um die Acrylsäure aus dem Stoffgemisch abzutrennen.

Dabei macht man sich zunutze, dass beim Wachstum der aus der Acrylsäure entstehenden Kristalle die in dem flüssigen Stoffgemisch neben Acrylsäure enthaltenen Bestandteile häufig aus dem Kristallgitter verdängt werden und in der Mutterlauge zurückbleiben (der Begriff Mutterlauge soll in dieser Schrift so verstanden werden, dass er sowohl Schmelzen (in ihnen entfällt ein Gewichtsanteil von $\geq$ 50 Gew.-% auf die Acrylsäure) aus Acrylsäure und Verunreinigungen als auch Lösungen der Acrylsäure und diese gegebenenfalls begleitende Verunreinigungen in Lösungsmitteln oder in Lösungsmittelgemischen (auf die Acrylsäure entfällt in ihnen ein Gewichtsanteil von < 50 Gew.-%) mit der Maßgabe umfasst, dass bei ihrer Abkühlung (d.h., bei der Abkühlung der Mutterlauge) die Acrylsäure auskristallisiert).

Manchmal erhält man bereits in einem einstufigen Suspensionskristallisationsprozess hochreine Kristalle der Acrylsäure. Bei Bedarf kann die Suspensionskristallisation auch mehrstufig durchgeführt werden.

Das Verfahren der Suspensionskristallisation zur kristallisativen Abtrennung von Acrylsäure ist bekannt (vgl. z.B. DE-A 10 2007 043758, DE-A 10 2007 043748, DE-A 10 2007 004960, DE-A 10 2007 043759 und DE Aktenzeichen Nr. 10 2009 000987.6).

Anwendungstechnisch zweckmäßig wird es mit Hilfe eines einen Sekundärraum und wenigstens einen Primärraum aufweisenden indirekten Wärmeübertragers (Kühlers bzw. Kristallisators) durchgeführt.

Durch die Übertragung von Wärme aus dem dem Sekundärraum zugeführten (und diesen in der Regel durchströmenden), die Acrylsäure enthaltenden flüssigen Stoffgemisch durch die den Sekundärraum und den wenigstens einen Primärraum voneinander trennende materielle Trennwand (die Wärmeübertragungsfläche) hindurch in ein in dem wenigstens einen Primärraum fließendes Kühlmittel hinein, kühlt das flüssige Stoffgemisch ab, bis seine Sättigungsgrenze mit Acrylsäure überschritten ist und das flüssige Stoffgemisch der Übersättigung durch Ausbildung (durch Ausscheidung) von aus Acrylsäure aufgebautem Kristallisat entgegenwirkt.

Ist der gewünschte Kristallisationsgrad (der Begriff Kristallisationsgrad meint dabei den Massenbruch oder auch Massenanteil des in der resultierenden Suspension von Kristallen der Acrylsäure in (flüssig) verbliebener Mutterlauge enthaltenen feinteiligen Kristallisats an der Gesamtmasse der Kristallsuspension) erreicht, wird die Kristallsuspension aus dem Sekundärraum herausgeführt.

Durch Abtrennen der gebildeten Acrylsäurekristalle von der Mutterlauge kann die Acrylsäure in entsprechender Reinheit aus der Kristallsuspension isoliert werden.

Ein entscheidender Schritt, der die Reinheit der abgetrennten Acrylsäure maßgeblich beeinflusst, ist dabei das für die Abtrennung der Acrylsäurekristalle von der Mutterlauge, die die von der Acrylsäure verschiedenen Bestandteile in angereicherter Form sowie die noch nicht kristallisierten Anteile der Acrylsäure enthält, angewandte Trennverfahren. Dieser Trennprozess kann mehrstufig sein, wobei zumindest in der letzten Stufe häufig vorzugsweise eine Abtrennung mit einer hydraulischen Waschkolonne angewendet wird.

Die Abtrennung mit einer hydraulischen Waschkolonne kann aber auch die einzige Trennstufe bilden. Grundsätzlich kommt der Abtrennung mit einer hydraulischen Waschkolonne die Aufgabe zu, die verunreinigte Mutterlauge möglichst quantitativ von den Acrylsäurekristallen zu trennen.

Trennverfahren zur reinigenden Abtrennung von Acrylsäurekristallen aus einer Suspension ihrer Kristalle in Mutterlauge mit Hilfe einer hydraulischen Waschkolonne sind bekannt (vgl. z.B. DE Aktenzeichen Nr. 10 2009 000987.6, WO 2006/111565, DE-A 10 2007 004960, EP-A 1 448 282, US-A 2009/018347, WO 03/041832, WO 01/77056, WO 04/35514, WO 03/41833, WO 02/9839, DE-A 100 36 881, WO 02/55469, WO 03/78378 und der in diesen Schriften zitierte Stand der Technik).

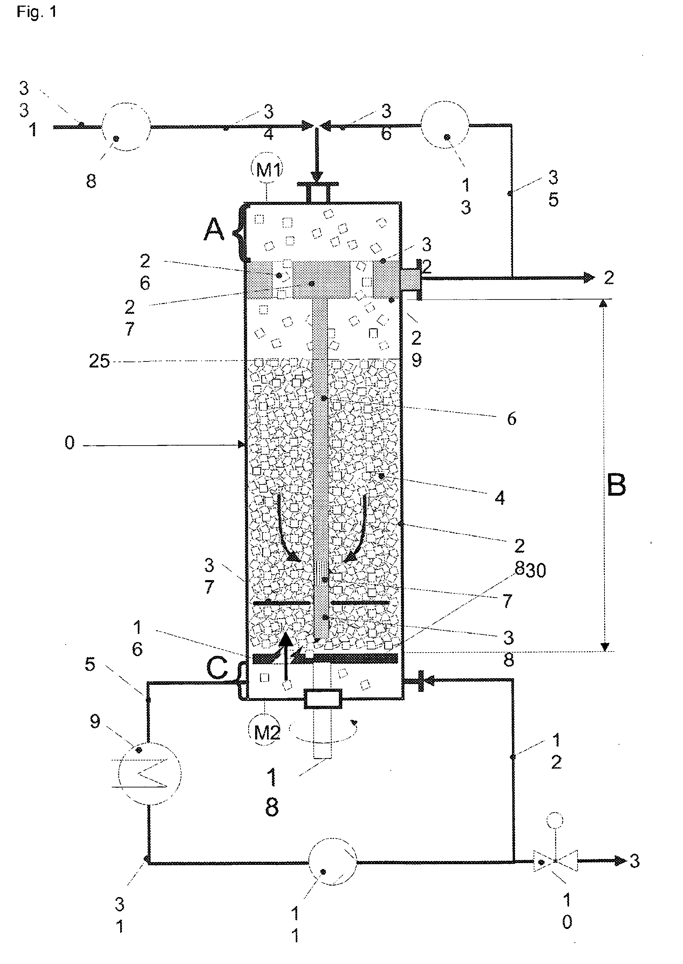

Ein Muster einer hydraulischen Waschkolonne (0) zeigt die Figur 1 dieser Schrift. Sie weist einen zu seiner von oben nach unten verlaufenden Längsachse rotationssymmetrischen Prozessraum (B) auf (alle in dieser Schrift in Klammern gesetzten alphabetischen oder numerischen Adressen beziehen sich auf die dieser Schrift beiliegenden Figuren).

Dieser wird von einer zylindrischen Mantelwand (28) und zwei auf der Symmetrieachse einander gegenüberliegende Enden begrenzt, wobei sich vom oberen Ende (29) des Prozessraums (B) parallel zu dessen Längsachse ein oder mehrere Filterrohre (6) durch den Prozessraum (B) erstrecken, die auf das dem oberen Ende gegenüberliegende unter Ende (30) des Prozessraums (B) zulaufen (ohne es zu durchstoßen), und in der dem unteren Ende des Prozessraum (B) zugewandten Hälfte des Prozessraums (B) wenigstens ein Filter F (7), das die einzige direkte Verbindung zwischen dem jeweiligen Filterrohrinneren und dem Prozessraum (B) bildet, aufweisen sowie außerhalb des Prozessraums (B) aus der Waschkolonne (0) herausgeführt werden.

Am unteren Ende des Prozessraums (B) schließt sich nach unten gerichtet der Kristallschmelzeraum (C) der hydraulischen Waschkolonne (0) an, wobei zwischen den beiden Räumen eine rotationsfähige Abtrageinrichtung (16) integriert und durch den Kristallschmelzraum (C) ein Kristallschmelzekreis (31) hindurchgeführt ist.

Die Abtrageinrichtung (16) ist normalerweise an einer Antriebswelle (18) befestigt, die von einem Antriebsaggregat zur Rotation um ihre Längsachse angetrieben wird, wobei sie das für die Rotation der Abtrageinrichtung (16) erforderliche Drehmoment auf diese überträgt.

Der Kristallschmelzekreis (31) umfasst außer dem Kristallschmelzeraum (C) eine außerhalb der Waschkolonne (0) befindliche Förderpumpe P1 (11), die eine Saug- und eine Druckseite aufweist. Eine erste Förderverbindung G1 (5) führt vom Kristallschmelzeraum (C) der Waschkolonne (0) über einen Wärmeübertrager W (9) zur Saugseite der Förderpumpe P1 (11). Eine zweite Förderverbindung G2 (12) führt von der Druckseite der Förderpumpe P1 (11) in den Kristallschmelzeraum (C) der Waschkolonne (0) zurück. Sie umfasst einen Auslass A (3) mit einem regelbaren (10) Durchfluss.

Dem oberen Ende des Prozessraums (B) ist nach oben gerichtet ein Verteilerraum (A) vorgelagert, der vom Prozessraum (B) wenigstens durch einen Boden B (32) getrennt ist, welcher Durchgänge U (26) aufweist, die auf der dem Prozessraum zugewandten Seite des Bodens B in den Prozessraum (B) und auf der vom Prozessraum (B) abgewandten Seite des Bodens B in den Verteilerraum (A) führen.

Außerhalb der hydraulischen Waschkolonne (0) befindet sich eine Förderpumpe P2 (8), die eine Saug- und eine Druckseite aufweist. Eine erste Förderverbindung E1 (33) führt von einer Quelle QS (1) der Suspension der Acrylsäurekristalle in Mutterlauge zur Saugseite der Förderpumpe P2 (8). Eine zweite Förderverbindung E2 (34) führt von der Druckseite der Förderpumpe P2 (8) in den Verteilerraum (A) der hydraulischen Waschkolonne (0).

Außerhalb der Waschkolonne (0) befindet sich in der Regel (aber nicht in notwendiger Weise) zusätzlich eine Förderpumpe P3 (13), die eine Saug- und eine Druckseite aufweist. Eine erste Förderverbindung C1 (35) führt von der Saugseite der Pumpe P3 (13) zu einer Quelle QT für sogenannte Steuerlauge (als Steuerlauge wird über das wenigstens eine Filterrohr (6) herausgeführte (abgeführte) Ablauge verwendet (vgl. z.B. WO 2006/111565)).

Eine zweite Förderverbindung (36) führt von der Druckseite der Pumpe P3 (13) in den Verteilerraum (A) der hydraulischen Waschkolonne (0) und/oder in den zwischen seinem oberen Ende (29) und dem wenigstens einen Filter (7) des wenigstens einen Filterrohrs (6) gelegenen Längsabschnitt des Prozessraums (B).

Bei der Durchführung des Abtrennverfahrens in seinem stationären Betrieb wird mit der Pumpe P2 (8) kontinuierlich ein Strom der Suspension von Kristallen der Acrylsäure in Mutterlauge durch die Förderverbindungen E1 (33), E2 (34) über den Verteilerraum (A) und durch die Durchgänge U (26) hindurch in den Prozessraum (B) der Waschkolonne (0) geführt (gegebenenfalls wird zusätzlich mit der Pumpe P3 (13) Steuerlauge durch die Förderverbindungen C1 (35), C2 (36) über den Verteilerraum (A) und durch die Durchgänge U (26) hindurch und/oder direkt in den Prozessraum der Waschkolonne (0) geführt). Die Durchgänge U (26) wirken dabei auf eine möglichst gleichmäßige Verteilung der Kristallsuspension über den Querschnitt des Prozessraums (B) hin. Die Druckverhältnisse im Filterrohrinnern sowie im Prozessraum (B) sind so gestaltet, dass über die Filter F (7) der Filterrohre (6) ein Strom umfassend Mutterlauge und gegebenenfalls Steuerlauge als Ablaugestrom in das Filterrohrinnere hinein- und über die Filterrohre (6) (in der Regel über einen Ablaugesammelraum (27), der z.B. in den Boden B integriert sein kann) aus der Waschkolonne (0) herausgeführt (über einen entsprechenden Auslass) wird (2).

Dieser Ablaugestrom bildet die Quelle QT für einen optional mitverwendeten Steuerlaugestrom.

Durch die Mutter- und gegebenenfalls Steuerlaugeführung im Prozessraum der Waschkolonne (0) (zunächst von oben nach unten und anschließend mit überlagertem Querstrom durch die Filter (7) in die Filterrohre (6) hinein) wird die bei der Inbetriebnahme des Trennverfahrens erstmals erfolge Ausbildung eines (verdichteten) "Kristallbett (filterkuchen)s (4)" an Acrylsäurekristallen stetig fortgeschrieben und so die Ausbildung eines Kristallbetts (4) an Acrylsäurekristallen aufrechterhalten, das eine dem oberen Ende des Prozessraums zugewandte Aufbaufront (25) aufweist, an der sich kontinuierlich Kristalle des zugeführten Stroms der Suspension von Acrylsäurekristallen in Mutterlauge an den (verdichteten) Kristallbettfilterkuchen (4) anlagern (in der Literatur wird die Aufbaufront häufig auch als Filtrationsfront bezeichnet).

Durch die aus dem hydraulischen Strömungsdruckverlust der Mutter- und gegebenenfalls Steuerlauge auf deren Strömungsweg im Prozessraum (B) durch das Kristallbett (4) resultierende Kraft wird das Kristallbett (4) verdichtet und von oben nach unten an den Filtern F (7) vorbei (quasi als Filterkuchen der Querfiltration) auf die rotierende Abtrageinrichtung (16) zu gefördert.

Mit der rotierenden Abtrageinrichtung (16) werden vom auf sie stoßenden Kristallbett (4) Acrylsäurekristalle kontinuierlich abgetragen. Der so entstehende Strom an abgetragenen Acrylsäurekristallen wird je nach Ausgestaltung der rotierenden Abtrageinrichtung (16) durch selbige hindurch und/oder an selbiger vorbei in den sich in Förderrichtung des Kristallbetts (4) an den Prozessraum (B) anschließenden Kristallschmelzeraum (C) gefördert, und in dem durch den Kristallschmelzeraum (C) geführten Kristallschmelzekreis (31) (bzw. Schmelzkreis (31)) durch Eintrag von Wärme mit dem Wärmeüber-trager W (9) zu einem Kristallschmelzestrom aufgeschmolzen (selbstverständlich kann der Wärmeübertrager W zu diesem Zweck alternativ auch in die Förderverbindung G2 integriert sein; auch kann zu diesem Zweck mehr als ein Wärmeübertrager in den Kristallschmelzekreis integriert sein).

Der Durchfluss des Auslasses A (3) ist dabei so geregelt (10), dass, bezogen auf die Stärke des vorgenannten Kristallschmelzestroms, vom Kristallschmelzeraum (C) ausgehend ein Teilstrom der spezifisch vergleichsweise leichteren (eine geringere Massendichte aufweisenden), und von den in den Kristallschmelzeraum geförderten Kristallen verdrängten, Kristallschmelze als Waschschmelzestrom, je nach Ausgestaltung der rotierenden Abtrageinrichtung (16) durch selbige hindurch und/oder an selbiger vorbei, gegen die Bewegungsrichtung des Kristallbetts (4) in den Prozessraum (B) zurückströmt (der aufsteigende Waschschmelzemassenstrom wird im Normalfall nicht größer sein, als der über die Kristallsuspension in den Prozessraum (B) geführte Kristallmassenstrom), wo er im abwärts geförderten Kristallbett (4) aufsteigt und dabei von den Kristallen die im Kristallbett (4) verbliebene und mit diesem unter die Filter F (7) geförderte Mutterlauge abwäscht und dabei die Mutterlauge nach oben zurückdrängt, wobei sich im von den Filtern F (7) bis zum unteren Ende des Prozessraums (30) erstreckenden Längsabschnitt des Prozessraums (B) im Kristallbett (4) eine Waschfront (37) ausbildet, die das Kristallbett von oben nach unten in eine Mutterlaugenzone (erstreckt sich von der Waschfront (37) bis zur Aufbaufront) und in eine Waschschmelzezone (erstreckt sich von der Waschfront (37) bis zum unteren Ende des Kristallbetts (4)) aufteilt, und der restliche Teilstrom des vorgenannten Kristallschmelzestroms den Schmelzkreis (31) durch den Auslass A (3) verlässt (die Förderpumpe P1 (11) fungiert als reine Kreislaufpumpe).

D.h., durch die entgegengesetzt zur Förderrichtung des Kristallbetts (4) strömende Waschschmelze wird das unterhalb der Filter F (7) nur noch mit einer Restmenge an Mutterlauge getränkte Kristallbett (4) im Ergebnis praktisch in die im Prozessraum (B) aufwärts strömende Waschschmelze hineingedrückt (und umgekehrt) und als eine Waschwirkung (weitere mögliche Waschwirkungen sind auf Seite 9 der WO 01/77056 aufgeführt) die bei der "Filtration" im Kristallbett (4) verbliebene Mutterlauge durch die Waschschmelze in begrenztem Umfang einfach zurückgedrängt. Bei entsprechender Einstellung des Waschschmelzestroms auf die Rahmenbedingungen des Abtrennprozesses stellt sich ein stationärer Zustand ein, so dass sich auf einer definierten Höhe im Prozessraum (B) eine sogenannte Waschfront (37) einstellt (faktisch eine weitgehend stabile "Phasengrenze" zwischen Waschschmelze (Reinschmelze) und Mutterlauge). Die Waschfront ist als diejenige Höhe im sich vom unteren Ende des Kristallbetts bis zur oberen Filterkante erstreckenden Abschnitt des Prozessraums (B) definiert, auf der über die Prozessraumhöhe betrachtet die höchsten Temperatur- und Konzentrationsgradienten auftreten.

Oberhalb und unterhalb der Waschfront (37) im Wesentlichen erreichen die höhenabhängigen Temperaturen (Konzentrationen) vergleichsweise rasch (in der Regel innerhalb einer Höhenänderung ("Waschfrontbereich" genannt) von weniger als $\pm 5$ cm) einen sich höhenabhängig jeweils nicht mehr ändernden Wert.

Dieser ist im Bereich oberhalb der Waschfront (37) im Wesentlichen die Temperatur (die entsprechende Konzentration) der dem Prozessraum (B) zugeführten Suspension von Acrylsäurekristallen in Mutterlauge und im Bereich unterhalb der Waschfront (37) die Schmelzpunkttemperatur (die entsprechende Konzentration) der Waschschmelze (Reinschmelze). Die Höhenposition der Waschfront (37) kann durch Regelung des Verhältnisses von im Prozessraum gefördertem Kristallmassenstrom zu entgegengeführtem Waschschmelzestrom in begrenztem Umfang variiert werden. Unterhalb einer jeweiligen Mindestlänge der Waschschmelzezone wird die Waschwirkung (die Abtrennwirkung) mit zunehmender Länge der Waschschmelzezone besser. Anwendungstechnisch zweckmäßig liegt die Waschfront (37) 50 bis 200 mm, oft bis 100 mm unterhalb der unteren Filterkante (unterhalb der unteren Kante der Filter F (7)).

Zur Inbetriebnahme des wie vorstehend beschrieben im stationären Betrieb durchzuführenden Trennverfahrens zur reinigenden Abtrennung von Acrylsäurekristallen aus deren Suspension in Mutterlauge empfiehlt die WO 01/77056, die entsprechende Kristallisatsuspension (Kristallsuspension) unmittelbar in die unbefüllte hydraulische Waschkolonne einzuspeisen und durch die Filter der Filterrohre hindurch zunächst nur Mutterlauge abzuführen, bis sich im Prozessraum der Waschkolonne in der gewünschten Betthöhe ein Kristallfestbett ausgebildet hat. Anschließend werden Abtrageinrichtung und Kristallschmelzekreis in Betrieb genommen und nach einem gewissen Vorlauf mit geschlossenem Durchfluss des Auslasses des Kristallschmelzekreises selbiger so eingeregelt, dass die gewünschte Position der Waschfront resultiert.

Nachteilig an dieser Art und Weise der Inbetriebnahme ist jedoch, dass mit ihr in einer wirtschaftlich relevanten Häufigkeit eine Blockade des Kristallschmelzekreises einhergeht. Diese ist im Regelfall darauf zurückzuführen, dass bei einer unmittelbaren Einspeisung der Kristallsuspension in die unbefüllte hydraulische Waschkolonne bis zum Zeitpunkt des Erreichens der gewünschten Betthöhe in erhöhtem Umfang bereits Kristallisat in den Kristallschmelzekreis gelangt. Wird selbiger dann in Betrieb genommen, wirbeln die sich zuvor absetzenden Kristalle (die zwar im Wesentlichen keine Verdichtung erfahren haben) schlagartig auf, woraus die beschriebene Blockade resultieren kann (insbesondere wenn man berücksichtigt, dass die Förderpumpe für den Kristallschmelzekreis (die Schmelzkreispumpe) ihre volle Förderleistung erst nach einer gewissen Anlaufzeit erreicht).

Grundsätzlich kann dem vorstehend beschriebenen Erscheinungsbild dadurch entgegengewirkt werden, dass man bei der Inbetriebnahme der hydraulischen Waschkolonne zunächst den den Kristallschmelzeraum umfassenden Schmelzkreis sowie den Prozessraum der zuvor unbefüllten Waschkolonne mit einer Acrylsäure enthaltenden Anfahrflüssigkeit so befüllt, dass die Füllhöhe der Anfahrflüssigkeit im Prozessraum wenigstens die Abtrageinrichtung überragt, und erst daran anschließend die Befüllung der hydraulischen Waschkolonne mit Kristallsuspension sowie gegebenenfalls Ablauge als Steuerlauge fortsetzt.

Bei hydraulischen Waschkolonnen, deren Quotient Q= UD aus dem Abstand L zwischen dem oberen und dem unteren Ende des Prozessraums und dem Durchmesser D des Prozessraums 0,3 bis 4 beträgt, sind bei einer solchen Vorgehensweise im Verlauf des weiteren Betriebs des Trennverfahrens mit vergleichsweise hoher Regelmäßigkeit und nach bereits relativ kurzen Betriebsdauern im die Förderverbindung E2 und den Verteilerraum umfassenden Raumbereich jedoch Arbeitsdrucke aufgetreten, die zum Bersten der aus Sicherheitsgründen in diesem Raumbereich eingebauten Berstscheibe führten.

Die Aufgabe der vorliegenden Erfindung bestand angesichts der beschriebenen Probleme darin, geeignete Abhilfe zu schaffen.

Demgemäß wird ein Verfahren der Inbetriebnahme eines Trennverfahrens zur reinigenden Abtrennung von Acrylsäurekristallen aus einer Suspension S ihrer Kristalle in Mutterlauge mit einer Vorrichtung, die eine hydraulische Waschkolonne umfasst, die einen zu seiner von oben nach unten verlaufenden Längsachse rotationssymmetrischen Prozessraum aufweist, der von einer zylindrischen Mantelwand und zwei auf der Symmetrieachse einander gegenüberliegenden Enden begrenzt wird, wobei

- sich vom oberen Ende des Prozessraums parallel zu dessen Längsachse ein oder mehrere Filterrohre durch den Prozessraum erstrecken, die auf das dem oberen Ende gegenüberliegende untere Ende des Prozessraums zulaufen (ohne es zu durchstoßen), und in der dem unteren Ende des Prozessraums zugewandten Hälfte des Prozessraums wenigstens ein Filter F, das die einzige direkte Verbindung zwischen dem jeweiligen Filterrohrinneren und dem Prozessraum bildet, aufweisen sowie außerhalb des Prozessraums aus der Waschkolonne herausgeführt werden,

- der Quotient Q = UD aus dem Abstand L zwischen dem oberen und dem unteren Ende des Prozessraums und dem Durchmesser D des Prozessraums 0,3 bis 4 beträgt,

- sich am unteren Ende des Prozessraums nach unten gerichtet der Kristallschmelzeraum der Waschkolonne anschließt, wobei zwischen den beiden Räumen eine rotationsfähige Abtrageinrichtung integriert und durch den Kristallschmelzeraum ein Kristallschmelzekreis hindurchgeführt ist, der außer dem Kristallschmelzeraum

    - eine außerhalb der Waschkolonne befindliche Förderpumpe P1, die eine Saug- und eine Druckseite aufweist,

    - eine erste Förderverbindung G1, die vom Kristallschmelzeraum der Waschkolonne zur Saugseite der Förderpumpe P1 führt,

    - eine zweite Förderverbindung G2, die von der Druckseite der Förderpumpe P1 in den Kristallschmelzeraum der Waschkolonne zurückführt und einen Auslass A aus dem Schmelzkreis mit regelbarem Durchfluss aufweist, sowie

    - einen Wärmeübertrager W, über den entweder die Förderverbindung G1 vom Kristallschmelzeraum zur Saugseite der Förderpumpen P1, oder die Förderverbindung G2 von der Druckseite der Förderpumpe P1 zum Kristallschmelzeraum geführt ist, umfasst,

- dem oberen Ende des Prozessraums nach oben gerichtet ein Verteilerraum vorgelagert ist, der vom Prozessraum wenigstens durch einen Boden B getrennt ist, welcher Durchgänge U aufweist, die auf der dem Prozessraum zugewandten Seite des Bodens B in den Prozessraum und auf der vom Prozessraum abgewandten Seite des Bodens B in den Verteilerraum führen,

- sich außerhalb der Waschkolonne eine Förderpumpe P2, die eine Saug- und eine Druckseite aufweist, und eine Quelle QS der Suspension S befinden, wobei

    - eine erste Förderverbindung E1 von der Quelle QS zur Saugseite der Förderpumpe P2, und

    - eine zweite Förderverbindung E2 von der Druckseite der Förderpumpe P2 in den Verteilerraum führt,

- sich außerhalb der Waschkolonne gegebenenfalls eine Förderpumpe P3, die eine Saug- und eine Druckseite aufweist, und eine Quelle QT einer Steuerlauge befinden, wobei

    - eine erste Förderverbindung C1 von der Saugseite der Pumpe P3 zur Quelle QT, und

    - eine zweite Förderverbindung C2 von der Druckseite der Pumpe P3 in den Verteilerraum und/oder in den zwischen seinem oberen Ende und den Filtern F der Filterrohre gelegenen Längsabschnitt des Prozessraums führt,

und wobei man bei der Durchführung des Trennverfahrens in seinem stationären Betrieb

- mit der Pumpe P2 kontinuierlich einen Strom ST der Suspension S von der Quelle QS durch die Förderverbindungen E1, E2 über den Verteilerraum und durch die Durchgänge U hindurch in den Prozessraum der Waschkolonne führt,

- gegebenenfalls mit der Pumpe P3 einen Strom SL der Steuerlauge von der Quelle QT durch die Förderverbindungen C1, C2 über den Verteilerraum und die Durchgänge U hindurch und/oder direkt in den Prozessraum der Waschkolonne führt,

- über die Filter F der Filterrohre insgesamt einen Strom SM umfassend Mutterlauge und gegebenenfalls Steuerlauge als Ablaugestrom in das Filterrohrinnere hinein und über die Filterrohre aus der Waschkolonne herausführt, und diesen aus der Waschkolonne herausgeführten Ablaugestrom SM als die Quelle QT für die Steuerlauge verwendet,

- durch die Mutter- und gegebenenfalls Steuerlaugeführung im Prozessraum der Waschkolonne die Ausbildung eines Kristallbetts an Acrylsäurekristallen aufrechterhält, das eine dem oberen Ende des Prozessraums zugewandte Aufbaufront aufweist, an der sich kontinuierlich Kristalle des zugeführten Stroms ST der Suspension S ans Kristallbett anlagern,

- das Kristallbett durch die aus dem hydraulischen Strömungsdruckverlust der Mutter- und gegebenenfalls Steuerlaugeführung im Prozessraum resultierende Kraft von oben nach unten an den Filtern F vorbei auf die rotierende Abtrageinrichtung zu fördert,

- mit der rotierenden Abtrageinrichtung vom auf sie stoßenden Kristallbett Acrylsäurekristalle abträgt,

- den Strom der abgetragenen Acrylsäurekristalle durch die rotierende Abtrageinrichtung hindurch und/oder an der rotierenden Abtrageinrichtung vorbei in den sich in Förderrichtung des Kristallbetts an den Prozessraum anschließenden Kristallschmelzeraum fördert, und in dem durch den Kristallschmelzeraum geführten Kristallschmelzekreis (bzw. Schmelzkreis) durch Eintrag von Wärme mit dem Wärmeübertrager W zu einem Kristallschmelzestrom aufschmilzt, und

- den Durchfluss des Auslasses A so regelt, dass, bezogen auf die Stärke des vorgenannten Kristallschmelzestroms, vom Kristallschmelzeraum ausgehend ein Teilstrom an Kristallschmelze als Waschschmelzestrom durch die rotierende Abtrageinrichtung hindurch und/oder an der rotierenden Abtrageinrichtung vorbei gegen die Bewegungsrichtung des Kristallbetts in den Prozessraum zurückströmt, wo er im abwärts geförderten Kristallbett aufsteigt und dabei von den Kristallen die im Kristallbett verbliebene und mit diesem unter die Filter F geförderte Mutterlauge abwäscht und zurückdrängt, wobei sich im von den Filtern F bis zum unteren Ende des Prozessraums erstreckenden Längsabschnitt des Prozessraums im Kristallbett eine Waschfront ausbildet, die das Kristallbett von oben nach unten in eine Mutterlaugezone und in eine Waschschmelzezone aufteilt, und der restliche Teilstrom des vorgenannten Kristallschmelzestroms den Schmelzkreis durch den Auslass A verlässt,

zur Verfügung gestellt, das dadurch gekennzeichnet ist, dass man bei der Inbetriebnahme des Trennverfahrens zur erstmaligen Ausbildung des Kristallbetts im Prozessraum

- den den Kristallschmelzeraum umfassenden Schmelzkreis und den Prozessraum der zuvor unbefüllten Waschkolonne mit einer Acrylsäure enthaltenden Anfahrflüssigkeit AT zunächst so befüllt, dass die Füllhöhe der Anfahrflüssigkeit AT im Prozessraum wenigstens die Abtrageinrichtung überragt,

- anschließend die Befüllung der Waschkolonne fortsetzt, indem man mit der Pumpe P2 einen Strom ST* der Suspension S von der Quelle QS durch die Förderverbindungen E1, E2 über den Verteilerraum und durch die Durchgänge U hindurch in den Prozessraum der Waschkolonne und von dem dabei durch die Filterrohre aus der Waschkolonne herausgeführten Ablaugestrom SM* als Quelle QT* gegebenenfalls mit der Pumpe P3 einen Teilstrom als Steuerlaugestrom SL* durch die Förderverbindungen C1, C2 über den Verteilerraum und die Durchgänge U hindurch und/oder direkt in den Prozessraum der Waschkolonne führt, und dies wenigstens so lange, bis der Zeitpunkt $t_S$ erreicht ist, bei dem der Differenzdruck $P_D = P_K - P_V$, wobei $P_K$ der an einer beliebig ausgewählten Stelle im Kristallschmelzeraum zu einem bestimmten Zeitpunkt der Zufuhr des Stroms ST* jeweils bestehende Druck und $P_V$ der zum jeweils gleichen Zeitpunkt an einer beliebig ausgewählten Stelle im Verteilerraum jeweils bestehende Druck ist, in Abhängigkeit von der Dauer der Zufuhr des Stroms ST* nicht mehr ansteigt oder konstant bleibt, sondern plötzlich abnimmt, und dies mit der Maßgabe, dass

- bis zum Zeitpunkt $t_S$ die mittlere Flächenbelastung der Filter F, berechnet als der arithmetische Mittelwert des während der Zufuhr des Stroms ST* durch die Filter F der Filterrohre zum jeweiligen Zeitpunkt insgesamt strömenden Ablaugestroms SM* (dies ist der Ablaugegesamtstrom; d.h. die Summe aller in den einzelnen Filterrohren abgeführten Ablaugeströme, wobei über alle Filterrohre summiert wird), geteilt durch die Gesamtfläche aller Filter F, nicht mehr als 80 m³/(m²·h) beträgt,

- die Acrylsäure enthaltende Anfahrflüssigkeit AT eine solche ist, aus der beim Abkühlen bis zur einsetzenden Kristallisation die sich bei der Kristallisation bildenden Kristalle Acrylsäurekristalle sind, und

- zwischen der in Grad Celsius angegebenen Kristallbildungstemperatur $T^{KB}$ dieser Acrylsäurekristalle in der Anfahrflüssigkeit AT und der in Grad Celsius angegebenen Temperatur $T^S$ der Suspension S des Stroms ST* die Beziehung

$$T^{KB} \leq T^S + 15°C$$

erfüllt ist.

Erfindungsgemäß bevorzugt beträgt der arithmetische Mittelwert (in dieser Schrift auch als "mittlere Filterflächenbelastung" oder als "mittlere Flächenbelastung der Filter F" bezeichnet) des während der Zufuhr des Stroms ST* durch die Filter F der Filterrohre zum jeweiligen Zeitpunkt insgesamt strömenden Ablaugestroms SM*, geteilt durch die Gesamtfläche aller Filter F, bis zum Zeitpunkt $t_S$ nicht mehr als 75 und besonders bevorzugt nicht mehr als 70 m³/(m²·h).

In der Regel wird der vorgenannte auf die Gesamtfläche aller Filter F normierte arithmetische Mittelwert (die mittlere Filterflächenbelastung) wenigstens 5 oder wenigstens 10, mit Vorteil wenigstens 15 und mit besonderem Vorteil wenigstens 20 m³/(m²·h) betragen.

D.h., erfindungsgemäß günstige Bereiche für den vorgenannten auf die Gesamtfläche aller Filter F normierten arithmetischen Mittelwert (die mittlere Filterflächenbelastung) sind die Bereiche >0 bis 80 m³/(m²·h), bevorzugt 5 bis 75 m³/(m²·h), besonders bevorzugt 10 bis 70 m³/(m²·h), ganz besonders bevorzugt 15 bis 65 m³/(m²·h) und mit besonderem Vorteil 20 bis 50 m³/(m²·h).

Alles Vorgenannte und alles weitere in dieser Schrift Gesagte trifft insbesondere dann zu, wenn der Quotient Q= L/D ≥ 0,5 oder ≥ 0,7 beträgt. Natürlich trifft alles Vorgenannte und alles weitere in dieser Schrift Gesagte auch dann zu, wenn L/D ≤ 3,5, oder ≤ 3, oder ≤ 2,5, oder ≤ 2 beträgt. Nicht zu große Quotienten Q= UD sind erfindungsgemäß vorteilhaft. Dies auch vor dem Hintergrund, dass der beim Transport des Kristallbetts im Prozessraum entlang der Kontaktfläche zwischen Kristallbett und Innenwand des Prozessraums zu überwindende Reibungswiderstand, bezogen auf das Volumen des Kristallbetts, mit einem abnehmenden Q sinkt.

Anwendungstechnisch zweckmäßig wird der Abstand L zwischen dem oberen und dem unteren Ende des Prozessraums ≥ 0,5 m, vorzugsweise ≥ 0,8 m und besonders bevorzugt ≥ 1 m betragen. In der Regel wird L jedoch ≤ 5 m und häufig ≤ 4 m oder ≤ 3 m betragen.

Günstige Innendurchmesser D eines Prozessraums einer erfindungsgemäß geeigneten hydraulischen Waschkolonne liegen im Bereich von 300 bis 3000 mm, vorzugsweise im Bereich von 700 bis 2000 mm.

Günstige für die Kristalle zugängliche Prozessrauminnenvolumina betragen beim erfindungsgemäßen Verfahren 0,05 bis 20 m³, vorteilhaft 0,2 bis 10 m³ sowie besonders vorteilhaft 1 bis 5 m³.

Unter der Fläche eines Filters F wird in dieser Schrift dessen Anströmfläche (nicht dessen "offene" Filterfläche, d.h., nicht die "freien Löcher" des porösen Materials) verstanden. D.h., erstreckt sich ein Filter F bei einem Außenradius r des zugehörigen Filterrohrs und einer Höhe a des Filters F über den gesamten Rohrumfang, beträgt die erfindungsgemäß relevante Fläche des Filters F demnach 2 π·r·a.

Der Durchmesser D des Prozessraums meint in dieser Schrift dessen Innendurchmesser. Der Abstand L zwischen dem oberen und dem unteren Ende des Prozessraums meint in dieser Schrift den lichten Abstand zwischen der Unterseite des die Durchgänge U aufweisenden Bodens B und der Oberfläche des durch die rotierende Abtrageinrichtung beschriebenen Rotationskörpers.

Trägt man bei der erfindungsgemäßen Inbetriebnahme des Trennverfahrens den während der Zufuhr des Stroms ST* bis zum Zeitpunkt $t_S$ durch die Filter F der Filterrohre der hydraulischen Waschkolonne zum jeweiligen Zeitpunkt insgesamt strömenden Ablaugestrom SM* als Ordinate gegen die Zeit t als Abszisse auf, so bildet die im Zeitbereich t= 0 (Beginn der Zufuhr des Stroms ST*) bis zum Zeitpunkt t= $t_S$ unter der dabei resultierenden Kurve befindliche Fläche, geteilt durch $t_S$, den in dieser Schrift verwendeten arithmetischen Mittelwert des während der Zufuhr des Stroms ST* bis zum Zeitpunkt $t_S$ durch die Filter F der Filterrohre zum jeweiligen Zeitpunkt insgesamt strömenden Ablaugestroms SM*. Dividiert man ihn durch die Gesamtfläche aller Filter F erhält man die mittlere Flächenbelastung der Filter F.

Unter der Kristallbildungstemperatur $T^{KB}$ einer Anfahrflüssigkeit AT wird in dieser Schrift diejenige Temperatur verstanden, bei der aus dieser Flüssigkeit heraus bei ihrer Abkühlung die Ausbildung von Acrylsäurekristallisat einsetzt (die Möglichkeit des Auftretens von Übersättigungserscheinungen wird dabei außer acht gelassen). Alternativ ausgedrückt ist die Kristallbildungstemperatur $T^{KB}$ einer Anfahrflüssigkeit AT diejenige Temperatur, die in der Anfahrflüssigkeit AT in dem Moment vorliegt, der erreicht wird, wenn man, ausgehend von einer aus der Anfahrflüssigkeit AT durch Abkühlung derselben erzeugten Kristallsuspension bzw. Kristallisatsuspension (Suspension von Acrylsäurekristallisat) unter ständiger (im Idealfall idealer) Durchmischung Wärme zuführt, um die in der Kristallisatsuspension enthaltenen Acrylsäurekristalle zu schmelzen, und der letzte Acrylsäurekristall gerade geschmolzen ist (sie wird in der Literatur teilweise auch als Lösetemperatur oder als Kristallisationseinsetztemperatur bezeichnet).

Erfindungsgemäß vorteilhaft erfüllen die Temperaturen $T^{KB}$ und $T^S$ bei der erfindungsgemäßen Inbetriebnahme die Relation $T^{KB} \leq T^S + 10°C$ und besonders vorteilhaft die Relation $T^{KB} \leq T^S + 5°C$.

Grundsätzlich kann die Kristallbildungstemperatur $T^{KB}$ der Anfahrflüssigkeit AT auch unterhalb der Temperatur des bei der erfindungsgemäßen Inbetriebnahme der hydraulischen Waschkolonne zugeführten Stroms ST* an Acrylsäurekristallen in Mutterlauge liegen.

In der Regel wird $T^{KB}$ beim erfindungsgemäßen Verfahren jedoch nicht mehr als 20°C, meist nicht mehr als 10°C, und oft nicht mehr als 5°C unterhalb von $T^S$ liegen. Als Anfahrflüssigkeit AT kommen für das erfindungsgemäße Verfahren z.B. von der Suspension S abgetrennte (z.B. durch Filtration) Mutterlauge, oder nach ihrer Erzeugung wieder aufgeschmolzene Suspension S, oder diejenige Flüssigkeit, aus der die Suspension S durch Abkühlen erzeugt worden ist, oder die Schmelze von aus einer Suspension S zuvor in einer (der) hydraulischen Waschkolonne reinigend abgetrennten Acrylsäurekristallen (d.h. Reinschmelze), oder Gemische von zwei oder mehreren der vorgenannten möglichen Anfahrflüssigkeiten in Betracht.

Mit dem Begriff "Pumpe" meint diese Schrift Pumpen zur Förderung von Flüssigkeiten (d.h., im Wesentlichen inkompressiblen Medien). Sie weisen eine Saugseite und eine Druckseite auf. Durch eine mit ihrer Saugseite verbundene Förderverbindung saugt die Förderpumpe die zu fördernde Flüssigkeit (bzw. Suspension) an. In der Pumpe wird die zu fördernde Flüssigkeit auf einen erhöhten Druck gebracht und durch eine mit ihrer Druckseite verbundene Förderverbindung in die gewünschte Förderrichtung weggedrückt. Anwendungstechnisch vorteilhaft handelt es sich bei den relevanten Förderverbindungen in einfachster Weise um Rohrleitungen (Förderleitungen), durch die hindurch die Förderung erfolgen kann. Für die erfindungsgemäße Verfahrensweise eignen sich insbesondere die in der DE-A 10228859 und in der DE Anmeldenummer 102008054587.2 beschriebenen Förderpumpen (insbesondere die in diesen Schriften beschriebenen Radialkreiselpumpen). Zur Förderung der Suspension S eignen sich insbesondere Radialkreiselpumpen mit halboffenem radialem Laufrad (vgl. DE Anmeldenummer 102008054587.2). Der Zugang zur Saugseite bzw. Druckseite der jeweiligen Pumpe kann in der Regel über entsprechende Armaturen geöffnet oder gesperrt werden. Insbesondere bei der Förderpumpe P2 handelt es sich anwendungstechnisch vorteilhaft um eine "Drehzahl"-geregelte Förderpumpe. D.h., die Einstellung der resultierenden Förderstromstärke erfolgt vorzugsweise über eine Anpassung der Drehzahl und nicht über eine Anpassung des freien Querschnitts in der Förderverbindung (nicht über ein Regelventil), da im letzteren Fall das Risiko, dass es zu einem Verschluss der Förderverbindung kommt (z.B. durch Kristallanhäufungen), erhöht ist.

Ferner ist es für die erfindungsgemäße Verfahrensweise von Vorteil, wenn man bei der Inbetriebnahme des Trennverfahrens zur erstmaligen Ausbildung des Kristallbetts im Prozessraum den den Kristallschmelzeraum umfassenden Schmelzkreis (Kristallschmelzekreis) und den Prozessraum der zuvor unbefüllten Waschkolonne mit einer Acrylsäure enthaltenden Anfahrflüssigkeit AT zunächst so befüllt, das die Füllhöhe der Anfahrflüssigkeit AT im Prozessraum wenigstens die Filter F überragt, vorzugsweise wenigstens bis zur Mitte des Abstands L vom unteren bis zum oberen Ende des Prozessraums ragt, besonders bevorzugt wenigstens bis zum letzten Viertel des Abstands L vom unteren bis zum oberen Ende des Prozessraums ragt, ganz besonders bevorzugt wenigstens bis zum oberen Ende des Prozessraums ragt, noch vorteilhafter über den Prozessraum hinaus bis in den Verteilerraum hineinragt und das Volumen desselben bis wenigstens zur Hälfte ausfüllt, und am Besten über den Prozessraum hinaus bis in den Verteilerraum hineinragt und das Volumen desselben vollständig ausfüllt (anwendungstechnisch zweckmäßig wird im letzteren Fall zusätzlich die Förderverbindung E2 (gegebenenfalls auch die Förderverbindung E1) und ein gegebenenfalls vorgehaltener, die Pumpe P3 und die Förderverbindungen C1, C2 umfassender, Steuerlaugekreis mit der Anfahrflüssigkeit AT befüllt).

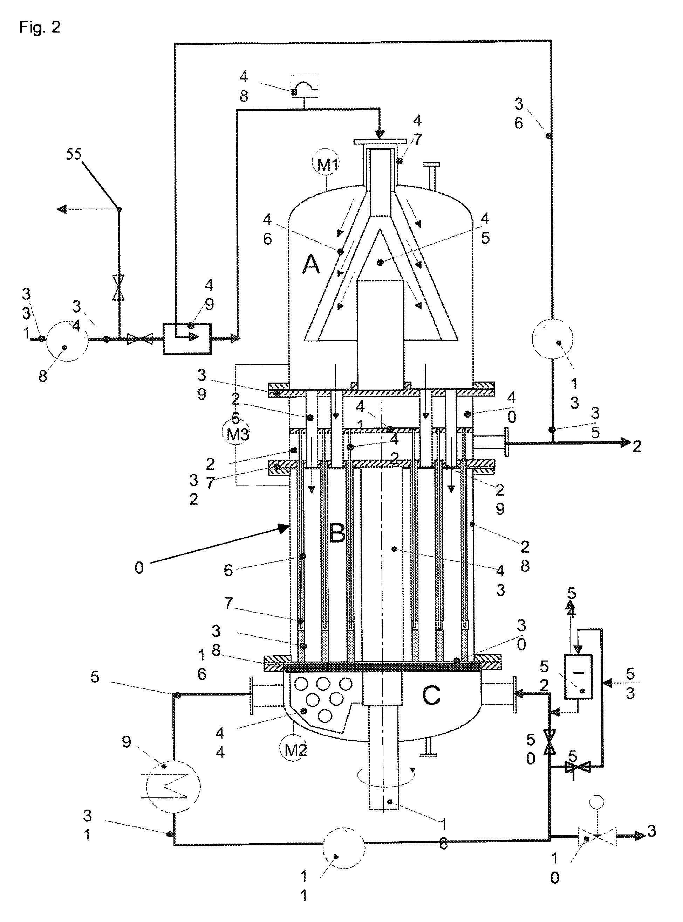

Weist die hydraulische Waschkolonne ein Spülflüssigkeitssystem auf, wie es z.B. die EP-A 1448282 empfiehlt und die Figur 2 dieser Schrift zeigt (42), erfolgt das beschriebene Befüllen erfindungsgemäß bevorzugt über dieses Spülflüssigkeitssystem.

Mit der Befüllung des Prozessraums mit der Anfahrflüssigkeit AT geht beim erfindungsgemäßen Verfahren im Sinne von über die Filter F mit dem Prozessraum kommunizierenden Röhren gleichzeitig ein entsprechendes Befüllen der Filterrohre einher. Wie in der WO 2006/111565 ausgeführt, wird der Druck an den Filtern F im Filterrohrinneren während des stationären Betriebs eines Abtrennverfahrens in einer hydraulischen Waschkolonne auf einem niedrigeren Wert gehalten, als der Druck an den Filtern F auf der Prozessraumseite.

Dies trifft auf die erfindungsgemäße Inbetriebnahme ebenfalls zu, sobald die Räume der hydraulischen Waschkolonne vollständig mit kondensierter Phase befüllt sind und daran anschließend mit der Zufuhr des Stroms ST* der Kristallsuspension S sowie gegebenenfalls des Steuerlaugestroms SL* fortgefahren wird (bis zu diesem Zeitpunkt wird die in der hydraulischen Waschkolonne befindliche Gasphase verdrängt und über ein am Waschkolonnenkopf befindliches Ventil ausgelassen (das Ventil ist so lange geöffnet, so lange die Schwingungsfrequenz eines diesbezüglich angebrachten Schwingers eine ihn umgebende Gasphase reflektiert (der Differenzdruck $P_D = P_K - P_V$ nimmt dabei zunächst stetig zu, bis die kondensierte Phase diejenige Stelle im Verteilerraum erreicht, an der die Detektion des Drucks $P_V$ erfolgt; daran anschließend bleibt der Differenzdruck $P_D$ im Wesentlichen zunächst konstant)).

Gemäß eingehender Analyse der Vorgänge bei der Inbetriebnahme einer hydraulischen Waschkolonne durch die Anmelderin kommt es anschließend in der hydraulischen Waschkolonne vermutlich zu folgenden Abläufen.

Die durch den vorstehend beschriebenen Druckunterschied aufgeprägte Flüssigkeitsströmung im Prozessraum der hydraulischen Waschkolonne von zunächst oben nach unten und anschließend selbiger überlagert quer durch die Filter F hindurch in die Filterrohre hinein, führt, bei anhaltender Zufuhr des Stroms ST* sowie gegebenenfalls des Steuerlaugestroms SL*, zunächst zu einer Anschwemmung von Acrylsäurekristallen in der lokalen Umgebung des jeweiligen Filters F. Aus dem beim Durchströmen derselben resultierenden erhöhten lokalen Druckverlust, erwächst eine Verdichtung dieser Anschwemmungen zu schneeballartigen Filterkuchen um die Filter F herum. Diese Filterkuchen beginnen im weiteren Verlauf sowohl in die Breite als auch in die Länge (insbesondere in die Höhe) zu wachsen.

Erstreckt sich der Filterkuchen (das verdichtete Bett aus Kristallen) erstmals in geschlossener Form über den gesamten Querschnitt des Prozessraums, beginnt der Differenzdruck $P_D = P_K - P_V$ plötzlich abzunehmen (zuvor noch bestehende, für Flüssigkeit im Wesentlichen ohne nennenswerten Druckverlust überwindbare Kanäle (mit vergleichsweise großem Strömungsquerschnitt), über die der Kristallschmelzeraum und der Verteilerraum quasi ungestört miteinander zu kommunizieren vermochten, sind jetzt wegverdichtet). Der Zeitpunkt zu dem dies erreicht wird, ist der Zeitpunkt $t_S$.

Offensichtlich ist es nun im erfindungsgemäß relevanten Bereich des Quotienten Q= L/D nicht möglich, den vorgenannten Schluss des Kristallbetts zu erzielen, ohne dass das Kristallbett nicht bereits wenigstens in eine Teilmenge der Durchgänge U hinein- bzw. durch selbige hindurchgewachsen ist. Wird nun im weiteren Verlauf der Inbetriebnahme des Trennverfahrens beim Übergang in den stationären Betriebszustand der Transport des "geschlossenen" Kristallbetts aufgenommen, kann in einzelnen Durchgängen U Filterkuchen verbleiben ("stecken bleiben"). Die Wahrscheinlichkeit für dieses Ereignis wächst offensichtlich mit der Stärke des bei der Inbetriebnahme pro Gesamtfilterfläche insgesamt abströmenden Ablaugestromes SM*, der den Grad der Verdichtung im Filterkuchen entscheidend mitbestimmt. Verbleibt aber Filterkuchen in einzelnen Durchgängen U, so werden aus diesen im weiteren Verlauf der Ausübung des Trennverfahrens sich ihrerseits in den Verteilerraum hinein wachsend ausdehnende Schneebälle aus Filterkuchen.

Letzteres bedingt eine zur Aufrechterhaltung des Trennverfahrens überdurchschnittliche Zunahme des erforderlichen Förderdrucks der Suspension S sowie schlussendlich das Platzen der schützenden Berstscheibe.

Dem entsprechend ist es für das erfindungsgemäße Verfahren vorteilhaft, wenn bei der erfindungsgemäßen Inbetriebnahme wenigstens während 50%, vorzugsweise während 75% oder während 90% und besonders bevorzugt während des gesamten Zeitraums, gerechnet vom Beginn der Zufuhr des Stroms ST* der Suspension S sowie gegebenenfalls des Steuerlaugestroms SL* bis zum Erreichen des Zeitpunkts $t_S$, der zum jeweiligen Zeitpunkt durch die Filter F der Filterrohre insgesamt strömende Ablaugestrom SM*, geteilt durch die Gesamtfläche aller Filter F (diese Gesamtfläche ist in dieser Schrift die Summe der Flächen aller Filter F der im Prozessraum der hydraulischen Waschkolonne befindlichen Filterrohre) nicht mehr als 80, vorzugsweise nicht mehr als 75 und besonders bevorzugt nicht mehr als 70 m$^3$/(m$^2$·h) bzw. nicht mehr als 60 m$^3$/(m$^2$·h) beträgt. Mit Vorteil liegen die Werte des vorgenannten zum jeweiligen Zeitpunkt durch die Filter F der Filterrohre insgesamt strömenden Ablaugestromes SM* (dies ist die Summe aller in den jeweiligen Filterrohren abgeführten Ablaugeströme, wobei über alle im Prozessraum der hydraulischen Waschkolonne befindlichen Filterrohre summiert wird), geteilt durch die Gesamtfläche aller Filter F, wenigstens während 50%, vorzugsweise während 75% und ganz besonders bevorzugt während des gesamten Zeitraums der Zufuhr des Stroms ST* der Suspension S sowie gegebenenfalls des Steuerlaugestroms SL* bis zum Erreichen des Zeitpunkts $t_S$ im Bereich >0 bis 80 m$^3$/(m$^2$·h), bevorzugt im Bereich 5 bis 75 m$^3$/(m$^2$·h), besonders bevorzugt im Bereich 15 bis 65 m$^3$/(m$^2$·h) und ganz besonders bevorzugt im Bereich 20 bis 50 m$^3$/(m$^2$·h). Anwendungstechnisch bevorzugt ist es, bei der erfindungsgemäßen Inbetriebnahme (ab Zufuhr der Suspension S) bis zum Zeitpunkt ts eine im Wesentlichen konstante Filterflächenbelastung anzuwenden.

Anwendungstechnisch zweckmäßig wird sich bei der erfindungsgemäßen Inbetriebnahme bis zum Zeitpunkt $t_S$ (gerechnet vom Beginn der Zufuhr des Stroms ST* der Suspension S) der arithmetische Mittelwert M des dem Prozessraum der Waschkolonne insgesamt zugeführten Stroms an Flüssigkeit (Mutterlauge als Bestandteil des Stroms ST* der Suspension S und gegebenenfalls Steuerlauge in Form des Steuerlaugestroms SL*), geteilt durch die freie Querschnittsfläche des Prozessraums (= $\pi \cdot (D/2)^2$, abzüglich z.B. der Querschnittsfläche der Filterrohre sowie der Querschnittsfläche eines gegebenenfalls im Prozessraum eingesetzten zentralen Verdrängerkörpers) im Bereich > 0 bis 30 m$^3$/(m$^2$·h), vorzugsweise im Bereich 1 bis 25 m$^3$/(m$^2$·h), besonders bevorzugt im Bereich 5 bis 25 m$^3$/(m$^2$·h) bzw. 10 bis 20 m$^3$/(m$^2$·h) bewegen.

Trägt man bei der erfindungsgemäßen Inbetriebnahme des Trennverfahrens den während der Zufuhr des Stroms ST* der Suspension S bis zum Zeitpunkt $t_S$ dem Prozessraum der Waschkolonne zum jeweiligen Zeitpunkt insgesamt zugeführten Strom an Flüssigkeit als Ordinate gegen die Zeit t als Abszisse auf, so bildet die im Zeitbereich t= 0 (Beginn der Zufuhr des Stroms ST*) bis zum Zeitpunkt t= $t_S$ unter der dabei resultierenden Kurve befindliche Fläche, geteilt durch $t_S$, den oben genannten arithmetischen Mittelwert M.

Es ist erfindungsgemäß vorteilhaft, wenn bei der erfindungsgemäßen Inbetriebnahme wenigstens während 50%, vorzugsweise während 75% und besonders bevorzugt während des gesamten Zeitraums, gerechnet vom Beginn der Zufuhr des Stroms ST* der Suspension S sowie gegebenenfalls des Steuerlaugestroms SL* bis zum Erreichen des Zeitpunkts $t_S$, der zum jeweiligen Zeitpunkt dem Prozessraum der Waschkolonne insgesamt zugeführte Strom an Flüssigkeit, geteilt durch die Querschnittsfläche des Prozessraums > 0 bis 30 m$^3$/(m$^2$·h), vorzugsweise 1 bis 25 m$^3$/(m$^2$·h), besonders bevorzugt 5 bis 25 m$^3$/(m$^2$·h) bzw. 10 bis 20 m$^3$/(m$^2$·h) beträgt.

Der Acrylsäuregehalt der bei der erfindungsgemäßen Inbetriebnahme über den Verteilerraum der hydraulischen Waschkolonne deren Prozessraum zugeführten Suspension S von Acrylsäurekristallen in Mutterlauge wird häufig ≥ 60 Gew.-%, oder ≥ 70 Gew.-% oder ≥ 80 Gew.-%, oder ≥ 90 Gew.-%, oder ≥ 95 Gew.-% betragen (in natürlicher Weise beträgt er < 100 Gew.-%,

meist ≤ 98 Gew.-%).

Besonders relevant ist das erfindungsgemäße Verfahren der Inbetriebnahme dann, wenn der Kristallisationsgrad der bei der erfindungsgemäßen Inbetriebnahme dem Verteilerraum der hydraulischen Waschkolonne zugeführten Kristallsuspension S ≥ 0,10, oder ≥ 0,20, oder ≥ 0,25 beträgt. In der Regel wird der vorgenannte Kristallisationsgrad bei der erfindungsgemäßen Inbetriebnahme ≤ 0,60, häufig ≤ 0,50 und teilweise ≤ 0,40 betragen. Erfindungsgemäß relevante Kristallisationsgrade der Suspension S sind somit z.B. auch diejenigen im Bereich 0,2 bis 0,3.

Alles in dieser Schrift Gesagte gilt ferner insbesondere dann, wenn die Längstausdehnung der Kristalle (die längste geradlinige Direktverbindung zweier auf der Kristalloberfläche befindlicher Punkte) mehrheitlich (mehr als die numerische Hälfte aller Kristalle) 50 bis 1600 μm bzw. 200 bis 900 μm beträgt.

Es gilt insbesondere aber dann, wenn die Acrylsäurekristalle eine würfel- bis quaderförmige Erscheinungsform aufweisen, und dabei ein Länge zu Dicke Verhältnis im Bereich 1:1 bis 6:1, vorzugsweise im Bereich 1:1 bis 4:1, und besonders bevorzugt im Bereich 1,5:1 bis 3,5:1 zeigen. Die Dicke der Kristalle liegt dabei üblicherweise im Bereich von 20 bis 600 μm, oft im Bereich von 50 bis 300 μm. Die Länge der Kristalle liegt gleichzeitig üblicherweise im Bereich von 50 bis 1500 μm, oft bei 200 bis 800 μm.

Hinsichtlich der materiellen Beschaffenheit der für das erfindungsgemäße Verfahren einzusetzenden hydraulischen Waschkolonne ist erfindungsgemäß vorteilhaft der Lehre der WO 03/041832 zu folgen. D.h., als Wandmaterial wird vorzugsweise Edelstahl der DIN-Werkstoff-Nr. 1.4571, oder 1.4539, oder 1.4462, oder 1.4541 verwendet.

Darüber hinaus ist die hydraulische Waschkolonne einschließlich des zugehörigen Kristallschmelzekreises vorzugsweise wie in der US-A 2009/018347 beschrieben thermisch isoliert.

Die Lagerung der mit der Abtrageinrichtung fest verbundenen Antriebswelle folgt erfindungsgemäß vorteilhaft gemäß der Lehre der DE Anmelde Nr. 102009000987.6.

Als rotierende Abtrageinrichtung kommt für das erfindungsgemäße Verfahren z.B. eine Durchtrittsöffnungen (für die abgetragenen Kristalle) aufweisende rotierende Messerscheibe in Betracht, wie sie z.B. in der EP-A 1 448 282 und in der DE Anmelde Nr. 102009000987.6 beschrieben ist. Die Rotation der Abtrageinrichtung kann sowohl kontinuierlich als auch zeitlich getaktet erfolgen.

Anstelle einer Durchtrittsöffnungen aufweisenden Messerscheibe kann es sich bei der rotierenden Abtrageinrichtung auch um ein einzelnes (gegebenenfalls in einem Schaft eingearbeitetes (von einem Schaft gehaltenes)) rotierendes Abtragmesser handeln. In diesem Fall strömt der durch das rotierende Abtragmesser abgetragene Kristallstrom an diesem vorbei in den Kristallschmelzeraum. In beiden Fällen, sowohl im Fall der rotierenden Messerscheibe als auch im Fall des rotierenden Einzelmessers, trennt der durch die rotierende Abtrageinrichtung beschriebene Rotationskörper (wie bereits erwähnt) Prozessraum und Kristallschmelzeraum voneinander. Selbstverständlich kommt als rotierende Abtrageinrichtung auch jedwede zwischen einer kreisrunden Durchtrittsöffnungen aufweisenden Messerscheibe und einem rotierenden Einzelmesser liegende Übergangsform in Betracht. Grundsätzlich kann die Geometrie der Messerscheibe jedoch beliebig sein.

Da dem vom Kristallschmelzeraum aufsteigenden Waschschmelzestrom und dem in den Kristallschmelzeraum geförderten Strom an von der Abtrageinrichtung abgetragenen Acrylsäurekristallen die selben, den Prozessraum und den Kristallschmelzraum durch die Abtrageinrichtung hindurch und/oder an der Abtrageinrichtung vorbei miteinander verbindenden Passagen zur Verfügung stehen (die Kristalle müssen gegen den aufsteigenden Waschschmelzestrom nach unten strömen können), weist die Abtrageinrichtung anwendungstechnisch zweckmäßig ein nicht zu geringes Öffnungsverhältnis OV auf. Unter dem Öffnungsverhältnis OV wird in dieser Schrift, bezogen auf die Abtrageinrichtung im nicht rotierenden Zustand, das Verhältnis der Summe der Querschnittsflächen der durch die Abtrageinrichtung hindurch und/oder der an der Abtrageinrichtung vorbei führenden Passagen zur Querschnittsfläche des Kristallbetts an seinem der Abtrageinrichtung zugewandten Ende verstanden. Ist die Querschnittsfläche einer individuellen Passage durch die Passage hindurch nicht konstant, ist für die Summenbildung jeweils die kleinste Querschnittsfläche der Passage zu verwenden. Üblicherweise beträgt OV wenigstens 0,01, oder wenigstens 0,03 bzw. wenigstens 0,05, häufig wenigstens 0,1 und vielfach wenigstens 0,5 oder mehr (teilweise sogar wenigstens 0,9). In natürlicher Weise beträgt OV< 1, meist ≤ 0,95, teilweise ≤0,8 oder ≤0,5, oder sogar ≤0,2.

Wie bereits gesagt, ist die rotationsfähige Abtrageinrichtung der hydraulischen Waschkolonne beim erfindungsgemäßen Verfahren vorteilhaft als Messerscheibe ausgebildet. Vorzugsweise ist diese (kreis)rund. Als den Prozessraum mit dem Kristallschmelzeraum verbindende Passagen für die vom Kristallbett abgetragenen Kristalle weist sie zweckmäßig Schlitze (Durchgangsöffnungen) auf, an deren Rand (die von der Rotationsrichtung abgewandte Umrisslinienseite des Schlitzes (z.B. eines Langlochs) vorteilhaft die Messer angeordnet sind. Die Schlitze mit den Messern sind über die Messerscheibe vorzugsweise so verteilt, dass über das gesamte der Messerscheibe zugewandte Ende des Kristallbetts Kristalle abgetragen werden, wenn die Messerscheibe rotiert. Mit Vorteil sind die Schlitze radial ausgerichtet und jeder Schlitz mit einer schräg sitzenden Klinge (einem schräg sitzenden Messer) bestückt, mit der die Kristalle vom Kristallbett abgetragen werden. Die Verteilung der Schlitze über die Messerscheibe ist bevorzugt außerdem so gestaltet, dass bei einer Umdrehung der Messerscheibe durch jeden Schlitz im Wesentlichen der gleiche Massenstrom an Kristallen strömt. Das jeweilige Messer (die jeweilige Klinge) ragt zweckmäßig über die dem Kristallbett zugewandte Oberfläche (eine gegebenenfalls bestehende Profilierung derselben bleibt dabei unberücksichtigt, d.h., Bezugspunkt ist der höchste Punkt der Profilierung) hinaus (in typischer Weise 1 bis 15 mm, oft 2 bis 10 mm, oder 3 bis 5 mm), so dass von der Klinge Kristalle abgetragen und der Schlitzöffnung zugeführt werden.

Der Radius von erfindungsgemäß geeigneten Messerscheiben kann für großtechnische Verfahren z. B. 300 bis 3000 mm betragen. Vorgenannte Schlitze weisen häufig eine Langlochgeometrie auf (die Definition eines Langlochs findet sich z. B. in der DE-A 102007028333 und in der DE-A 102007028332). Die Schlitzgeometrie kann aber auch rechteckig sein, oder zwischen derjenigen eines Langlochs und derjenigen eines Rechtecks liegen.

Der Lochdurchmesser (Abstand der beiden Längskanten des Langlochs) kann z. B. 20 bis 100 mm (typisch 50 bis 70 mm) und der Abstand der beiden Lochzentren 100 bis 500 mm betragen. Die dem Kristallbett zugewandte Oberfläche der Messerscheibe ist anwendungstechnisch zweckmäßig außerdem mit einem Profil aus konzentrischen Rillen versehen (der Rillenquerschnitt ist vorteilhaft dreieckig; die Rillentiefe kann z. B. 2 bis 10 mm, oder 3 bis 7 mm betragen, die Rillenweite 10 bis 15 mm und der Abstand von in radialer Richtung aufeinanderfolgenden Rillen so bemessen sein, dass die zugehörigen Dreiecksquerschnitte gemeinsame Eckpunkte haben). Die Profilierung gewährleistet eine möglichst gleichmäßige Verteilung der aus dem Waschschmelzeraum in den Prozessraum rückströmenden Waschschmelze über den Querschnitt des Prozessraums. Die Figuren 5 und 8 der EP-A 1448282 zeigen beispielhafte Ausgestaltungen einer erfindungsgemäß als Abtrageinrichtung geeigneten Messerscheibe. Der Winkel $\gamma$, den die Fläche der Abtragelemente der Abtrageinrichtung (z. B. die Abtragklingen bzw. die Abtragmesser) und die Rotationsachse der Antriebswelle einschließen, beträgt beim erfindungsgemäßen Verfahren häufig 20° bis 70°, und vielfach 30° bis 60°. Die Antriebswelle ragt beim erfindungsgemäßen Verfahren von unten kommend anwendungstechnisch vorteilhaft bis zur Messerscheibe (bzw. generell bis zur Abtrageinrichtung). Von der Antriebswelle radial weglaufende, mit Lochöffnungen ausgerüstete Lamellen (Stege) tragen (stützen) die Messerscheibe anwendungstechnisch zweckmäßig (44).

Typische Kristallmassenzufuhrströme betragen, bezogen auf die Querschnittsfläche des Prozessraums an dessen Zufuhr-Ende, beim erfindungsgemäßen Verfahren 1 bis 20 t/m$^2 \cdot$ h (t = metrische Tonne). Die Drehzahl der Antriebswelle der Abtrageinrichtung liegt typisch im Bereich von 2 bis 40, häufig 4 bis 20 und oft 6 bis 15 oder 4 bis 10 pro Minute. Die Länge der Antriebswelle der Abtrageinrichtung beträgt insbesondere für großtechnische Verfahren 0,5 bis 4 m.

Der Kristallschmelzekreis ist bei einer hydraulischen Waschkolonne für ein erfindungsgemäßes Verfahren anwendungstechnisch zweckmäßig so beschaffen, dass er ein wesentlich größeres Reservoir an Kristallschmelze als der Kristallschmelzeraum für sich alleine vorzuhalten vermag (bezogen auf das Gesamtvolumen des Kristallschmelzekreises entfallen in der Regel lediglich 30 bis 60 Vol.-% oder 40 bis 50 Vol.-% auf das Volumen des Kristallschmelzeraums; unabhängig davon beträgt im stationären Betriebszustand das Verhältnis von im Schmelzkreis im Kreis geführtem Massestrom zu durch die rotierende Abtrageinrichtung vom unteren Ende des Kristallbetts abgetragenem und in den Kristallschmelzeraum gefördertem Kristallstrom anwendungstechnisch zweckmäßig in der Regel 2 bis 30 zu 1, und vorzugsweise 5 bis 20 zu 1.

D.h., der Kristallschmelzekreis weist normalerweise einen niederen Gehalt an noch nicht geschmolzenen abgetragenen Acrylsäurekristallen auf, was zum einen seine und deren Förderung begünstigt. Zum anderen ist die absolute Wärmekapazität des flüssigen Anteils des Inhalts des Schmelzkreises wesentlich höher, als die absolute Wärmekapazität des festen Anteils des Schmelzkreises (Phasenübergangswärmen bleiben dabei unberücksichtigt).

Der dem Kristallschmelzeraum zugeführte Kristallstrom wird in der in selbigem im Kreis geführten Kristallschmelze suspendiert und diese Suspension nachfolgend im Schmelzkreis über einen Wärmeübertrager (Aufschmelzer) W geführt, der auf indirektem (bevorzugt) oder direktem Weg die zum Schmelzen der Kristalle erforderliche Wärme in den Schmelzkreis einträgt. Aufgrund der vorgenannten Verhältnisse der absoluten Wärmekapazitäten resultiert aus dem zum Erreichen der angestrebten Schmelze der Kristalle erforderlichen Wärmeeintrag in den Schmelzkreis lediglich eine vergleichsweise geringe Temperaturerhöhung, was für die ausgeprägt zu unerwünschter radikalischer Polymerisation neigende Acrylsäure infolge der damit einhergehenden nur vergleichsweise geringen thermischen Belastung vorteilhaft ist.

Im Idealfall weist die im Kristallschmelzeraum befindliche Kristallschmelze im stationären Betrieb des Trennverfahrens Schmelzpunkttemperatur (bzw. Kristallbildungstemperatur) der abgetragenen Kristalle, bezogen auf deren Schmelze, auf (im Idealfall= 14°C). Üblicherweise wird diese im Kristallschmelzekreis jenseits des Wärmeübertragers W um nicht mehr als 10°C, besser um nicht mehr als 5°C, vorzugsweise um nicht mehr als 3 bzw. 2°C und besonders bevorzugt um nicht mehr als 1°C überschritten.

Mit Vorteil wird beim erfindungsgemäßen Verfahren die Förderverbindung G1 über den Wärmeübertrager W geführt.

Anwendungstechnisch zweckmäßig wird als Wärmeübertrager W ein Rohrbündelwärme-übertrager eingesetzt. Es handelt sich dabei um einen indirekten Wärmeübertrager. D.h., die Wärmeübertragung erfolgt nicht im durch Mischen erzwungenen direkten Kontakt zwischen fluidem Wärmeträger und dem den Wärmeeintrag benötigenden fluiden Gemisch. Vielmehr erfolgt die Wärmeübertragung indirekt zwischen den durch eine Trennwand getrennten Fluiden.

Solche Rohrbündelwärmeübertrager bestehen normalerweise aus einem abgeschlossenen weiten Mantelrohr, das die in einander gegenüberliegenden Rohrböden befestigten zahlreichen, in der Regel glatten oder gerippten, Übertragerrohre kleinen Innendurchmessers umschließt.

Der Abstand von Rohrmitte zur Rohrmitte der Bündelrohre beträgt anwendungstechnisch zweckmäßig das 1,3- bis 2,5-fache des Rohraußendurchmessers. Die entstehende große spezifische Wärmeaustauscherfläche - als Austauschfläche je Einheit des Raumbedarfs - ist ein bekannter Vorzug von Rohrbündelwärmeübertragem als Wärmeübertrager W für das erfindungsgemäße Verfahren. Grundsätzlich kann der Rohrbündelwärmeübertrager beim erfindungsgemäßen Verfahren senkrecht oder waagrecht angeordnet sein. Erfindungsgemäß bevorzugt ist er waagrecht angeordnet.

Erfindungsgemäß bevorzugt strömt der Inhalt des Schmelzkreises innerhalb der Übertragerrohre. Der fluide Wärmeträger (erfindungsgemäß vorteilhaft ein Gemisch aus Wasser und Glykol (z.B. mit 10 bis 60 Gew.-% Glykol; bevorzugt ist ein Gemisch aus 70 Gew.-% Wasser und 30 Gew.-% Glykol bzw. 65 Gew.-% Wasser und 35 Gew.-% Glykol; seine Temperatur beträgt zweckmäßig 25 bis 40°C)) strömt erfindungsgemäß bevorzugt außerhalb der Übertragerrohre. Leitbleche zur besseren Führung des fluiden Wärmeträgers im Mantelraum sind erfindungsgemäß günstig und dienen in der Regel dem zusätzlichen Zweck, die Übertragerrohre zu stützen. Die Leitbleche erhöhen in der Regel die Strömungsgeschwindigkeiten im Mantelraum und damit u.a. die Wärmeübergangszahlen. Nach der Strömungsrichtung des Mantelraumfluids in Bezug auf die Übertragerrohre sind z.B. Längsstrom- und Kreuzstrom - sowie Querstromrohrbündelwärmeübertrager unterscheidbar. Grundsätzlich kann der fluide Wärmeträger auch mäanderförmig um die Übertragerrohre bewegt und lediglich über den Rohrbündelwärmeübertrager betrachtet im Gleichoder Gegenstrom zum die Wärme aufnehmenden fluiden Gemisch geführt werden.

Im Einstromrohrbündelwärmeübertrager bewegt sich der Stoffstrom des Schmelzkreises durch alle Übertragerrohre in der gleichen (einen) Richtung.

Mehrstromrohrbündelwärmeübertrager enthalten in einzelne Sektionen unterteilte Rohrbündel (in der Regel enthalten die einzelnen Sektionen eine identische Anzahl von Rohren). Trennwände teilen an die Rohrböden (durch die die Übertragerrohre abgedichtet geführt und an denen sie befestigt sind) sich anschließende Kammern in Abschnitte auf und lenken den aus einer Sektion in den Kammerteil eintretenden (die übertragene Wärme aufnehmenden) Stoffstrom in eine zweite Sektion um und damit zurück. Der Wärme aufnehmende Stoffstrom durchfließt je nach Anzahl der Sektionen die Länge des Rohrbündelwärmeübertragers mehrmals (zweimal, dreimal, viermal etc.) mit vergleichsweise hoher Geschwindigkeit in alternierender Richtung (Zweistrom-, Dreistrom-, Vierstrom- etc. -rohrbündelwärmeübertrager). Wärmeübergangszahl und Austauschweg nehmen entsprechend zu.

Alternativ zum Rohrbündelwärmeübertrager kann für das erfindungsgemäße Verfahren als Wärmeübertrager W auch ein Plattenwärmeübertrager (Plattenwärmeaustauscher) eingesetzt werden. Plattenwärmeübertrager sind normalerweise nach Art der Filterpressen in der Regel aus gewellten oder anderweitig profilierten, mit Kanälen für den fluiden Wärmeträger und das die übertragene Wärme aufnehmende fluide Gemisch versehenen Platten (in der Regel aus Graphit oder Metall, z.B. Edelstahl) in kompakter Bauweise zusammengesetzt. Die beiden Wärme austauschenden Fluide fließen dann im Gleich-, Gegen- und/oder Querstrom als dünne Schichten wechselnd (z.B. auf- und abwärts) durch ihre Kammerreihen und stehen an beiden Kammerwänden miteinander in Wärmeübertragung. Die gewellten Plattenprofile erhöhen die Turbulenz und verbessern die Wärmeübergangszahlen. Für den erfindungsgemäßen Zweck verwendbare Plattenwärmeaustauscher sind z.B. beschrieben in der EP-A 1079194, der US 6,382,313, der EP-A 1232004 und der WO 01/32301. Selbstverständlich können als Wärmeübertrager W auch Spiralrohrwärmeübertrager oder sonstige Wärmeübertrager verwendet werden.

Mit Vorteil wird für das erfindungsgemäße Verfahren als Wärmeübertrager W ein Dreistromrohrbündelwärmeübertrager verwendet, durch dessen Rohre das Stoffgemisch des Schmelzkreises zwangsgefördert wird.

Die Rohraußendurchmesser können dabei bei einer Wanddicke von 2 mm der Rohre 25 mm betragen. Bei einer Länge der Rohre von 3000 mm beträgt deren Gesamtzahl anwendungstechnisch zweckmäßig 121 oder 225 (jeweils ca. ein Drittel der Rohrgesamtzahl für eine Strömungsrichtung). Die Rohrteilung beträgt gleichzeitig vorteilhaft 32 mm (60°-Teilung). Durch 9 oder 20 zwischen den Rohrböden (in denen die Austauscherrohre befestigt sind) angebrachte Umlenkscheiben (Scheibendicke: jeweils 5 mm) wird der die Wärmeübertragerrohre umgebende zylindrische Raum (der Primärraum) in 10 oder 21 Längsabschnitte (Segmente) unterteilt. Alle Umlenkscheiben sind im Prinzip kreisförmig. Der Kreisdurchmesser beträgt 584 oder 492 mm. An jeder der kreisförmigen Umlenkscheiben ist jedoch ein Kreisabschnitt weggeschnitten, dessen radiale tiefe von der Umfangslinie nach innen 82 oder 94 mm beträgt, so dass ein entsprechender Durchtritt für das Glykol-Wasser-Gemisch als Wärmeträger entsteht, wobei diese Durchtritte aufeinanderfolgend alternierend einander gegenüberliegend angebracht sind (im übrigen sind die Umlenkbleche an der Behälterwand abdichtend befestigt; dort wo die Wärmeübertragerrohre auf die Umlenkbleche stoßen, sind entsprechende Bohrungen in den Umlenkblechen. Der Eintritt des Wärmeträgers und des die Wärme aufnehmenden Stoffgemischs kann sich anwendungstechnisch günstig auf derselben Seite des Wärmeübertragers befinden. Der zugeführte Massenstrom des Wärmeübertragers beträgt typisch 20 000 bis 80 000 kg/h bei gleichzeitig zugeführten Schmelzkreisstoffströmen von 50 000 bis 200 000 kg/h. Bei einer Konfiguration wie in Figur 2 liegt der Arbeitsdruck (ohne Berücksichtigung von hydrostatischen Effekten) auf der Saugseite der Pumpe P1 (unmittelbar nach Austritt des Schmelzkreises aus dem Wärmeübertrager W) beim erfindungsgemäßen Verfahren unterhalb des Drucks im Kristallschmelzeraum (C) und beträgt häufig 0,1 bis 4 bar. Der Arbeitsdruck auf der Druckseite der Pumpe P1 (mittelbar nach Austritt des Schmelzkreises aus der Pumpe P1) beträgt bei einer Konfiguration gemäß Figur 2 beim erfindungsgemäßen Verfahren häufig 1 bis 10 bar.

Fertigungsmaterial des Rohrbündelwärmeübertragers ist vorzugsweise Edelstahl vom DIN-Typ 1.4571, oder 1.4541, oder 1.4306 rohrseitig und Kohlenstoffstähle wie 1.0425 oder Edelstähle wie 1.4541, oder 1.4571, oder 1.4306 mantelseitig.

Die Herstellung der Suspension S von Acrylsäurekristallen für das erfindungsgemäße Verfahren erfolgt erfindungsgemäß vorteilhaft wie in der DE-A 10 2007 043748, und in der DE-A 10 2007 043758 beschrieben durch Kühlungssuspensionskristallisation in einem indirekten Wärmeübertrager.

Aus dem Wärmeübertrager heraus wird die in selbigem erzeugte Suspension von Acrylsäurekristallen in Mutterlauge anwendungstechnisch vorteilhaft zunächst in einen durchmischten Puffertank PT geführt, wie es die DE-A 10 2007 043759 beschreibt. Aus diesem Pufferbehälter (als Quelle QS) heraus kann die Kristallsuspension dann von der Förderpumpe P2 als Suspension S angesaugt werden (über die Förderverbindung E1, die den Puffertank PT mit der Saugseite der Förderpumpe P2 verbindet).

In der Regel liegt die Temperatur der beim erfindungsgemäßen Verfahren dem Verteilerraum der hydraulischen Waschkolonne zugeführten Suspension S von Acrylsäurekristallen im Temperaturbereich von -25°C bis +14°C, häufig im Bereich von -5°C bis +12°C und bevorzugt im Bereich von +4 bzw. von +6 bis +9°C.

Der Gehalt der in der Suspension S enthaltenen Mutterlauge an Acrylsäure wird in der Regel noch ≥ 70 Gew.-% betragen. Er kann aber auch ≥ 80 Gew.-%, oder ≥ 85 Gew.-%, oder ≥ 87 Gew.-%, oder ≥ 90 Gew.-%, oder ≥ 92 Gew.-%, oder ≥ 94 Gew.-%, oder ≥ 95 Gew.-%, oder ≥ 96 Gew.-%, oder ≥ 97 Gew.-%, oder ≥ 98 Gew.-%, oder ≥ 99 Gew.-% betragen.

Bereits bevor beim erfindungsgemäßen Verfahren damit begonnen wird, dem Verteilerraum der hydraulischen Waschkolonne einen Strom ST* der Suspension S aus dem Puffertank PT als Quelle QS zuzuführen, ist es erfindungsgemäß von Vorteil, die Förderpumpe P2 in Betrieb zu nehmen, und über die Förderverbindung E1, die die Saugseite der Förderpumpe P2 mit dem Puffertank PT verbindet, von der Suspension S anzusaugen. Anschließend wird die angesaugte Suspension S von der Förderpumpe P2 zwar in die Förderverbindung E2 gedrückt, die von der Druckseite der Förderpumpe P2 in den Verteilerraum der hydraulischen Waschkolonne führt. In Strömungsrichtung auf dem Weg von der Druckseite der Förderpumpe P2 zum Eingang (Eingangsstutzen) des Verteilerraums der hydraulischen Waschkolonne ist vor dem Eingang in den Verteilerraum in der Förderverbindung E2 jedoch erfindungsgemäß vorteilhaft eine erste Armatur eingebaut, die die Förderverbindung E2 zunächst sperrt.

Zusätzlich zweigt zwischen der Druckseite der Förderpumpe P2 und der ersten Armatur von der zweiten Förderverbindung E2 erfindungsgemäß vorteilhaft eine in den Puffertank PT zurückführende Förderverbindung E3 ((55) in Fig. 2) ab, deren Verbindung mit dem Puffertank PT durch eine in die Förderverbindung E3 erfindungsgemäß vorteilhaft eingebaute zweite Armatur zwar gesperrt werden kann, zunächst jedoch offen gehalten wird. Dadurch kreist die Suspension S durch die bereits in Betrieb befindliche Förderpumpe P2 zunächst in einfacher Weise durch die Förderverbindungen E1, E2 und E3 hindurch über den Puffertank PT. Ab dem Zeitpunkt, ab dem dem Verteilerraum der hydraulischen Waschkolonne im Rahmen der erfindungsgemäßen Inbetriebnahme der Strom ST* der Suspension S zugeführt werden soll, sperrt die zweite Armatur die Förderverbindung E3 zum Puffertank PT und gleichzeitig öffnet die erste Armatur die Förderverbindung E2 zum Verteilerraum der hydraulischen Waschkolonne (als solche Armaturen können z.B. Ventile, Klappen oder Kugelhähne zum Sperren und Öffnen verwendet werden). Sowohl das Durchmischen im Puffertank PT als auch das Fördern der Kristallsuspension S erfolgt beim erfindungsgemäßen Verfahren vorteilhaft so, dass dabei möglichst kein Bruch und/oder keine sonstige Gestaltsänderung der suspendierten Kristalle resultiert. Dies gilt insbesondere für das oben beschriebene Kreisen der Kristallsuspension S.

Für den Fall, dass bei der erfindungsgemäßen Inbetriebnahme vorab der Zufuhr des Stroms ST* der Suspension S über den Verteilerraum und durch die Durchgänge U hindurch in den Prozessraum der hydraulischen Waschkolonne hinein sowohl der den Kristallschmelzeraum umfassende Schmelzkreis, als auch der Prozessraum und der Verteilerraum sowie die Förderleitungen E2 (und gegebenenfalls E1), C1, C2 und die Förderpumpe P3 vollständig mit der Acrylsäure enthaltenden Anfahrflüssigkeit AT befüllt werden, und mit der Zufuhr des Stroms ST* der Suspension S in den Verteilerraum diese Zufuhr begleitend gleichzeitig auch Steuerlaugestrom in den Verteilerraum und/oder direkt in den Prozessraum geführt wird, ist es anwendungstechnisch zweckmäßig, bereits vorab des Beginns der Zufuhr des Stroms ST* der Suspension S in den Verteilerraum, die Förderpumpe P3 in Betrieb zu nehmen und einen Strom der Anfahrflüssigkeit AT über den aus Verteilerraum, Prozessraum, Filterrohrinnerem, Förderverbindung C1, Förderpumpe P3 und Förderverbindung C2 gebildeten Kreis kreisen zu lassen.

Wird beim erfindungsgemäßen Verfahren der Inbetriebnahme ein Steuerlaugestrom SL* mitverwendet, so handelt es sich dabei um einen rückgeführten Teilstrom des über die Filterrohre aus der Waschkolonne insgesamt herausgeführten Ablaugestroms SM*. Im Normalfall erfolgt die vorgenannte Rückführung des Ablaugeteilstroms mit der im Wesentlichen selben Temperatur, mit der der Ablaugestrom SM* aus der Waschkolonne herausgeführt wird. Üblicherweise entspricht diese Temperatur derjenigen Temperatur, mit der die Suspension S der Acrylsäurekristalle in Mutterlauge dem Verteilerraum der hydraulischen Waschkolonne zugeführt wird. Selbstverständlich kann die vorgenannte Rückführung des Ablaugeteilstroms als Steuerlaugestrom SL* z.B. auch über einen direkten und/oder indirekten Wärmeaustauscher erfolgen, der die Temperatur des als Steuerlaugestrom SL* rückgeführten Ablaugeteilstroms erhöht. Anwendungstechnisch zweckmäßig sollte die Temperatur des Steuerlaugestroms SL* jedoch um nicht mehr als 15°C, vorzugsweise um nicht mehr als 10°C, und besonders bevorzugt um nicht mehr als 5°C oberhalb der Temperatur des in den Verteilerraum der hydraulischen Waschkolonne geführten Stroms ST* der Suspension S liegen. Das Vorgenannte trifft in entsprechender Weise auch auf den stationären Betrieb des Abtrennverfahrens in der hydraulischen Waschkolonne zu.

Ein mitverwendeter Steuerlaugestrom verfolgt sowohl bei der erfindungsgemäßen Inbetriebnahme als auch im stationären Betrieb den Zweck der Beeinflussung des hydraulischen Druckverlustes und damit der auf die Kristalle, das Kristallbett resultierenden Wirkkraft (z.B. der auf das geschlossene Kristallbett wirkenden Vorschubkraft). Ist der mit der Zufuhr der Suspension S der Acrylsäurekristalle in Mutterlauge einhergehende zugeführte Mutterlaugestrom diesbezüglich nicht befriedigend oder fluktuiert selbiger während der Zufuhrdauer in einem gewissen Umfang mit der Zeit, so kann dies mittels des Steuerlaugestroms ausgeglichen werden (eine ausführliche Erläuterung der Wirkweise des Steuerlaugestroms findet sich in der WO 2006/111565). Die Zufuhr des Steuerlaugestroms in den Prozessraum der hydraulischen Waschkolonne kann sowohl über den Verteilerraum und durch die Durchgänge U hindurch, als auch auf direktem Weg in den Prozessraum hinein erfolgen. Grundsätzlich kann dabei auf unterschiedlichen Höhen des Kristallbetts Steuerlauge in den Prozessraum geführt werden. Im Normalfall erfolgt die Zufuhr von Steuerlauge jedoch stets oberhalb der Filter F.

Da die Lage der Aufbau- bzw. Filtrationsfront im stationären Betrieb der hydraulischen Waschkolonne unter anderem durch die Vorschubgeschwindigkeit des Kristallbettes in der Waschkolonne beeinflusst wird, kann die Position der Aufbaufront bei auftretenden Störungen durch Anpassung des Steuerlaugestroms stabil gehalten ("gesteuert") werden (vgl. WO 2006/111565). Eine zunehmende (abnehmende) Stromstärke des Steuerlaugestroms bewirkt in der Regel eine Verschiebung der Aufbaufront nach unten (oben). Alternativ müßte die Stromstärke des zugeführten Suspensionstroms ST* variiert werden. Aus in der WO 2006/111565 ausgeführten Gründen sollte die Aufbaufront in der hydraulischen Waschkolonne weder zu hoch noch zu tief positioniert werden. Der Teil des Kristallbetts, der sich im stationären Betrieb beginnend am Kristallabtrag bis zum Beginn des Filters F erstreckt, wird auch als Waschzone bezeichnet. Der darüber befindliche Teil des Kristallbetts, der sich bis zur Aufbaufront erstreckt, wird auch Konzentrierungszone genannt. An diese schließt sich bis zum oberen Ende des Prozessraums die sogenannte Suspensionszone an. Üblicherweise ragen die Filterrohre der hydraulischen Waschkolonne in die Waschzone hinein, sind in diesem Bereich der Waschkolonne jedoch nicht mehr hohl (vgl. z.B. WO 01/77056, WO 03/41833 und WO 03/41832). Man bezeichnet diesen Teil der Filterrohre auch als Filterrohrverdränger.

Die Regelung der Stärke des Steuerlaugestroms kann z.B. durch Anpassung der Drehzahl der Förderpumpe P3 und/oder durch ein zusätzliches Regelventil erfolgen.

Grundsätzlich kann die Zufuhr der Suspension S und die Zufuhr von Steuerlaugestrom in den Verteilerraum der hydraulischen Waschkolonne räumlich getrennt erfolgen. Selbstredend können der Zuführstrom der Suspension S und ein dem Verteilerraum zugeführter Steuerlaugestrom aber auch bereits außerhalb des Verteilerraums zusammengeführt, miteinander vermischt und der dabei resultierende Gemischstrom (sozusagen über eine zusammengelegte Förderverbindung E2/C2) in den Verteilerraum der hydraulischen Waschkolonne geführt werden.

Beispielsweise kann das Vermischen der beiden Ströme dadurch erfolgen, dass die beiden Förderverbindungen E2 und C2 zunächst in einen statischen Mischer geführt werden und hinter selbigem als nur noch eine gemeinsame Förderverbindung zum Verteilerraum der hydraulischen Waschkolonne laufen.

Alternativ können die Förderverbindungen E2 und C2 vor dem Zuführraum der hydraulischen Waschkolonne als koaxiale Förderleitungen (Rohrleitungen) ausgeführt sein. Die Suspension S wird dabei anwendungstechnisch zweckmäßig in der inneren Förderverbindung (in der inneren Rohrleitung) und die Steuerlauge in der äußeren Förderverbindung (in der äußeren Rohrleitung) geführt. Eine Mischstrecke von z.B. 0,5 bis 20 m vor Eintritt in den Verteilerraum der hydraulischen Waschkolonne läuft das innen geführte Rohr aus und nur noch das äußere der beiden Rohre ist als dann gemeinsame Förderverbindung E2, C2 zum Verteilerraum weitergeführt. Erfindungsgemäß vorteilhaft verjüngt sich die innen geführte Rohrleitung zu ihrem Auslauf hin (in der Regel konisch; z.B. von ca. 80 mm Innendurchmesser auf ca. 50 mm Innendurchmesser). Auf diese Weise fungiert die innen auslaufende Rohrleitung als Treibdüse (vgl. Seiten 3/4 der DE-A 102006045089) mit der Suspension S als Treibstrahl. Als solcher saugt er in Strömungsrichtung unmittelbar hinter dem Ende der inneren Rohrleitung von der außen fließenden Steuerlauge an und vermischt sich mit selbiger zum Gemischstrom, welcher als solcher in den Verteilerraum strömt. Der Innendurchmesser der äußeren Rohrleitung kann bei vorgenannter Dimensionierung der inneren Rohrleitung z.B. 150 mm betragen. Der Abstand von der Innenseite der Wand der äußeren Rohrleitung zur Außenwand der inneren Rohrleitung kann dabei z.B. 40 bis 50 mm (z.B. 46 mm) betragen. Die Stärke des außen geführten Steuerlaugestroms kann dabei 5 bis 80 m³/h und die Stärke des innen geführten Suspensionsstroms 10 bis 50 m³/h betragen.

Die Ermittlung der Drucke $P_K$ und Pv kann z.B. so wie die Druckmessungen der WO 2006/111565 erfolgen. Anwendungstechnisch vorteilhaft werden für diesen Zweck Membranmanometer verwendet, die außerhalb der hydraulischen Waschkolonne angebracht sind. Über kleine offene Bohrungen (typischer Bohrlochdurchmesser = 0,1 bis 3 mm), die in einen zum Membranmanometer führenden Stutzen auslaufen, stehen die Messumformer mit dem Kolonneninneren in Verbindung (prinzipiell wird dabei analog den Ausführungen in der DE-A 10211290 und in der WO 2006/111565 vorgegangen). Um ein Zukristallisieren der vorgenannten Bohrungen und Stutzen zu verhindern, werden sie und die Manometer erfindungsgemäß vorteilhaft mit einem geringfügigen Wärmestrom begleitbeheizt (in der Regel ist diesbezüglich eine Einhausung der hydraulischen Waschkolonne in einem beheizten Gebäude ausreichend, wie es z.B. die WO 03/041832 und die US-A 2009/018347 beschreiben). Selbstverständlich kann der Differenzdruck $P_D$ auch unmittelbar über eine Differenzdruckmessung detektiert werden, wie sie die WO 2006/111565 ebenfalls beschreibt. Erfindungsgemäß bevorzugt werden als Manometer M1 und M2 (vgl. Figur 1 und Figur 2) solche vom Typ 2088 GS Membransensor, Meßbereich: 0-10 bar, der Lieferfirma Rosemount eingesetzt. Als Differenzdruckmanometer M3 (vgl. Figur 2) eignen sich für das erfindungsgemäße Verfahren vorzugsweise solche vom Typ 3051 CD Differenzmembransensor, Meßbereich: 0 - 500 mbar, der Lieferfirma Rosemount.

Mit Vorteil schließt die jeweilige Bohrung auf der Innenseite des jeweiligen Raums der hydraulischen Waschkolonne glatt ab. Der Durchmesser solcher offenen Bohrungen beträgt vom Kolonneninneren her gesehen anwendungstechnisch zweckmäßig ≤ 5 mm, oft ≤ 3mm und in der Regel ≥ 0,1 mm. Durch die Wandung hindurch kann anwendungstechnisch zweckmäßig ein sich zum Kolonneninneren hin kontinuierlich oder stufenförmig verjüngender Bohrungsdurchmesser angewendet werden.

Bis im Rahmen der erfindungsgemäßen Inbetriebnahme der Zeitpunkt $t_S$ erreicht ist, sind der Wärmeaustauscher W, die Förderpumpe P1 (wenigstens ab Beginn der Zufuhr des Suspensionsstroms ST*) und die rotationsfähige Abtrageinrichtung erfindungsgemäß bevorzugt außer Betrieb und der Durchfluss des Auslasses A vorzugsweise gesperrt.

Grundsätzlich können die vorgenannten Elemente aber auch bereits in beschränktem Umfang in Betrieb genommen sein. Ist der Durchfluss des Auslasses A vorab des Zeitpunktes ts in beschränktem Umfang bereits geöffnet, so ist dies beim Vorgang des erfindungsgemäßen Befüllens bei der Stärke des Stroms ST* sowie gegebenenfalls SL* entsprechend zu berücksichtigen. Ferner sollte die Rotationsbewegung der Abtrageinrichtung nicht zu stark sein, da sich selbiges auf den Prozess des Schließens des Kristallbetts sowie seinen weiteren Aufbau negativ auswirkt. Die Heizleistung des Wärmeaustauschers W kann vorab des Zeitpunktes $t_S$ bereits von Null verschieden sein, um beim Befüllen mit Suspension S bereits in den Schmelzkreis gelangende Acrylsäurekristalle aufzuschmelzen. Sie sollte vorab des Zeitpunktes $t_S$ anwendungstechnisch zweckmäßig jedoch nicht höher als bei 50% der im stationären Betrieb des Trennverfahrens angewandten Heizleistung liegen (beispielsweise könnte sie bei 10% oder bei 20% dieser Heizleistung liegen). Unter Heizleistung wird dabei der an den Schmelzkreis abgegebene Wärmestrom verstanden. Sie kann durch entsprechende Variation der Temperatur des Wärmeträgers (diese bewegt sich im Normalfall, wie bereits erwähnt, im Bereich 25 bis 40°C) und/oder Variation von dessen Stromstärke bedarfsgerecht angepasst werden.

Ist bei der erfindungsgemäßen Inbetriebnahme der Zeitpunkt ts erreicht, wird (wie bereits gesagt vorteilhaft bei zunächst nicht rotierender Abtrageinrichtung und außer Betrieb befindlichem Schmelzkreis) die Zufuhr des Stroms ST* der Suspension S in den Verteilerraum sowie die Zufuhr eines gegebenenfalls mitverwendeten Steuerlaugestroms SL* unterbrochen. Daran anschließend werden der Schmelzkreis und die Rotation der Abtrageinrichtung in Betrieb genommen, wobei die Abfolge der Inbetriebnahme ihrer einzelnen Elemente im Wesentlichen beliebig ist. Vorzugsweise ist die Abfolge jedoch wie nachfolgend beschrieben.

Erfindungsgemäß vorteilhaft wird zunächst die Heizleistung des Wärmeaustauschers W (bezogen auf eine in Betrieb befindliche Förderpumpe P1) auf etwa 50 bis 80% seiner Heizleistung im stationären Betrieb eingestellt. Danach wird die Förderpumpe P1 und anschließend die Rotation der Abtrageinrichtung in Betrieb genommen (beide werden auf ihren Betriebswert im stationären Zustand eingestellt).

Ab dem Zeitpunkt, ab dem die Rotation der Abtrageinrichtung in Betrieb genommen ist, wird die Zufuhr der Suspension S von der Quelle QS durch die Förderverbindungen E1, E2 über den Verteilerraum und durch die Durchgänge U hindurch in den Prozessraum der Waschkolonne wieder aufgenommen. Das gleiche gilt für einen gegebenenfalls mitverwendeten Steuerlaugestrom. Die Abfolge der Inbetriebnahme ist dabei belanglos.

Die Stromstärken werden dabei so gewählt, dass das Kristallbett und dessen Stand (Aufbaufront) sich nach unten zu bewegen beginnen. Die diesbezüglich erforderlichen Stromstärken von Suspensions- und gegebenenfalls Steuerlaugestrom müssen dabei nicht in notwendiger Wiese größer sein als diejenigen, die bei der erfindungsgemäßen Inbetriebnahme bis zum Zeitpunkt ts angewendet worden sind (erfindungsgemäß bevorzugt werden bis zum Zeitpunkt ts über die Zeit im Wesentlichen konstante Ströme und Stromstärken angewendet). Grundsätzlich können gegebenenfalls sogar bei ansonsten gleichbleibenden Strömen geringere Stromstärken als vor dem Zeitpunkt ts ausreichen, um zu erreichen, dass sich nicht nur das Kristallbett sondern auch dessen Stand (dessen Aufbaufront) nach unten zu bewegen beginnt. Dies rührt daher, dass bei der erfindungsgemäßen Inbetriebnahme bis zum Zeitpunkt ts eine zunehmende Verdichtung des sich ausbildenden Kristallbetts resultiert.

Als Folgewirkung resultiert auch bei einem Beibehalt der Stärke des Ablaugestroms ein über die Betriebsdauer zunehmender hydraulischer Druckverlust, der bei der Wiederinbetriebnahme nach dem Durchschreiten des Zeitpunktes ts ausreichen kann, um das Kristallbett und dessen Stand in Bewegung zu setzen.

In weniger günstigen Fällen kann jedoch ein auf die Einheit der Gesamtfläche aller Filter F bezogener Ablaugestrom von bis zu 250 m³/(m²·h), oder von bis zu 320 m³/(m²·h) erforderlich sein, um das Kristallbett und dessen Stand (Aufbaufront) in Bewegung zu setzen. Höhere Werte sind im Regelfall nicht erforderlich.

In besonders günstigen Fällen kann auch ein in entsprechender Weise bezogener Ablaugestrom von lediglich 40 m³/(m²·h) bereits ausreichend sein, um das Kristallbett und dessen Stand in Bewegung zu setzen. Der Stand des Kristallbetts im Prozessraum (die "Aufbaufront") beginnt zu fallen.

Da jetzt bereits am unteren Ende des Kristallbetts durch die Abtrageinrichtung Acrylsäurekristalle abgetragen, sowie in den Kristallschmelzeraum gefördert und im Schmelzkreis aufgeschmolzen werden, wird nun anwendungstechnisch zweckmäßig die Polymerisationsinhibierung (Zudosierung von Polymerisationsinhibitor und gegebenenfalls Luft oder ein sonstiges Sauerstoff enthaltendes Gas) des Schmelzkreises in Betrieb genommen.

Selbstverständlich muss das erfindungsgemäße Verfahren aus Gründen der Sicherheit im Beisein von Polymerisationsinhibitoren durchgeführt werden, um eine unerwünschte radikalische Polymerisation der Acrylsäure auszuschließen.

Während die Mutterlauge der Suspension S normalerweise Polymerisationsinhibitoren wie Phenothiazin (PTZ) und/oder den Monomethylether des Hydrochinons (MEHQ) in, durch die zu ihrer Erzeugung angewandte Suspensionskristallisation bedingt, angereicherten Mengen aufweist, sind die in der Suspension S suspendierten Acrylsäurekristalle normalerweise an Polymerisationsinhibitoren entreichert, da selbige bei der Kristallbildung üblicherweise nicht im sich ausbildenden Kristall eingebaut werden.

Werden im Schmelzkreis derartige vom Kristallbett abgetragene Kristalle aufgeschmolzen, führt dies dort, wo dieses Schmelzen punktuell von Statten geht, lokal zu einer Unterinhibierung der entstehenden Schmelze. Eine solche Unterinhibierung birgt ein erhöhtes Risiko für eine unerwünschte, durch die frei werdende Polymerisationswärme sich selbst beschleunigende radikalische Polymerisation der Acrylsäure, weshalb diesem Risiko entgegengewirkt werden muss.

In vergleichsweise einfacher Weise kann ein solches Entgegenwirken dadurch erfolgen, dass dem Schmelzkreis in Strömungsrichtung hinter dem Wärmeübertrager W (falls dieser in die Förderverbindung G1 integriert ist) aber vor der Saugseite der Förderpumpe P1 eine Lösung des entsprechenden Inhibitors (mit vergleichsweise erhöhter Inhibitorkonzentration) in Reinprodukt (in einer Schmelze von zuvor reinigend abgetrennten Acrylsäurekristallen) zudosiert (eine Zudosierung hinter der Druckseite der Förderpumpe P1 würde einen erhöhten Zufuhrdruck bedingen, ist grundsätzlich jedoch möglich) wird.

Erfindungsgemäß vorteilhaft kann diese Inhibitorlösung mit derjenigen Temperatur (z. B. über ein T-Stück) zudosiert werden, die der Schmelzkreis an der Zuführstelle aufweist. Häufig wird die Inhibitorlösung jedoch mit einer Temperatur im Bereich von 15 bis 35°C zudosiert. Typische Inhibitorgehalte einer solchen zugeführten Inhibitorlösung liegen z. B. bei 0,1 bis 1,5 Gew.-% PTZ und/oder bei 0,1 bis 5 Gew.-% MEHQ. Bezogen auf die Massenstromstärke im Auslass A liegt die Massenstromstärke der in den Schmelzkreis dosierten Inhibitorlösung in der Regel bei 0,1 bis 10 %, vorzugsweise bei 0,5 bis 3 %. Wird mit PTZ inhibiert, beträgt dessen Gewichtsanteil im Kristallschmelzeraum und im Auslass A typisch 50 bis 500 Gew. ppm. Wird mit MEHQ inhibiert, beträgt dessen Gewichtsanteil im Kristallschmelzeraum typisch 10 bis 500 Gew. ppm. Insbesondere dann, wenn der Schmelzkreis mittels MEHQ polymerisationsinhibiert wird (die Art der Polymerisationsinhibierung orientiert sich primär an der ins Auge gefassten Verwendung des dem Auslass A entnommenen Reinproduktes; ist beabsichtigt, das ausgelassene Reinprodukt in erster Linie in Polymerisationsreaktionen einzusetzen, erfolgt die Inhibierung vorzugsweise mit dem "Lagerinhibitor MEHQ"; ist beabsichtigt, das ausgelassene Reinprodukt in erster Linie für von Polymerisationsreaktionen verschiedene chemische Prozesse einzusetzen, wird vorzugsweise mit dem Prozessinhibitor PTZ inhibiert (vor allem dann, wenn der chemische Prozess thermischen Belastungen ausgesetzt ist)) erfolgt zusätzlich eine Coinhibierung durch Einbringen (z.B. Eindüsen) eines molekularen Sauerstoff enthaltenden Gases in den Schmelzkreis. Als solche molekularen Sauerstoff enthaltenden Gase kommen vor allem Mischungen aus molekularem Sauerstoff und Inertgas (z. B. $N_2$, $CO_2$, He, Ar) in Betracht (vorzugsweise ist das den molekularen Sauerstoff enthaltende Gas frei von Wasserdampf (vorab getrocknet) und frei von Feststoffpartikeln (vorab gefiltert)). Selbstverständlich kann auch reiner molekularer Sauerstoff zudosiert werden. Erfindungsgemäß bevorzugt wird als molekularen Sauerstoff enthaltendes Gas Luft zudosiert.

Erfindungsgemäß vorteilhaft erfolgt die Zudosierung des molekularen Sauerstoff enthaltenden Gases in den Schmelzkreis in Strömungsrichtung desselben auf der Druckseite der Förderpumpe P1 nach dem Auslass A.

Zum Zweck der Zudosierung des molekularen Sauerstoff enthaltenden Gases in den Schmelzkreis, wird der von der Förderpumpe P1 weggedrückte Kristallschmelzestrom mit Hilfe eines T-Stücks in zwei Teilströme gleicher Zusammensetzung aufgeteilt. Das Verhältnis ihrer Stromstärken wird zweckmäßig mittels zweier Armaturen (50) und (51) eingestellt. Die zuvor bestehende Sperrung dieser Aufteilung ist jetzt aufgehoben. Der kleinere der beiden Teilströme (er beträgt in der Regel wenigstens 5, normalerweise jedoch nicht mehr als 20 % des vorab der Aufteilung vorliegenden Gesamtstroms; der andere Teilstrom wird als Hauptteilstrom bezeichnet) durchströmt dann eine Sauerstoffeinbringstrecke (z.B. als Treibstrahl eine Strahldüse (vgl. DE-A 102006045089) in der das den molekularen Sauerstoff enthaltende Gas (vorzugsweise Luft) angesaugt wird). Im einfachsten Fall wird über ein T-Stück am Beginn der Einbringstrecke das molekularen Sauerstoff enthaltende Gas aus einer Druckleitung zugeführt (53). Vorzugsweise weist das den molekularen Sauerstoff enthaltende Gas dabei diejenige Temperatur auf, die auch der Schmelzkreis an der Zufuhrstelle aufweist. Vielfach weist es jedoch Umgebungstemperatur (≥15°C und ≤35°C) auf.

Nach einer ausreichend langen Mischstrecke wird der den molekularen Sauerstoff enthaltenden Gasstrom zudosiert aufweisende Teilstrom durch einen Gasabscheider (52) geführt, um in ihm nicht gelöstes Gas wieder abzutrennen. Diese Maßnahme verfolgt den Zweck, zu verhindern, dass sich solchermaßen nicht gelöstes Gas im weiteren Verlauf des Verfahrens in Form einer Gasblase unterhalb des unteren Endes des Kristallbetts ansammelt, und dadurch das Aufsteigen der Waschschmelze aus dem Kristallschmelzeraum ins Kristallbett und damit letztlich die Waschwirkung der hydraulischen Waschkolonne mindert.

Grundsätzlich können als ein solcher Gasabscheider alle bekannten Typen von Gasabscheidern eingesetzt werden, wie sie auch z. B. in der EP-A 492400 aufgeführt sind. Dazu gehören Zentrifugalabscheider (z. B. Zyklonabscheider) ebenso wie Schwerkraftabscheider. Letztere sind aufgrund ihrer einfachen Bauweise bei gleichzeitig befriedigender Trennwirkung erfindungsgemäß bevorzugt. Letztlich ist ein mit Prallblechen ausgestatteter Behälter diesbezüglich ausreichend. Der vorgenannte Teilstrom wird dabei in der einfachsten Ausführungsform in die Behältermitte auf dort angebrachte Prallplatten geführt. Am oberen Ende des Behälters befindet sich der Auslass für das im Prallbereich abgeschiedene spezifisch leichtere Gas (54) und im unteren Bereich des Behälters befindet sich der Auslass für die spezifisch schwerere Flüssigphase.

Hinter dem Gasabscheider werden der mit molekularem Sauerstoff angereicherte Teilstrom und der Hauptteilstrom wieder zu einem Gesamtstrom zusammengeführt.

Im Anschluss an die Inbetriebnahme der Polymerisationsinhibierung wird mit Vorteil zunächst die Stärke des erfindungsgemäß vorzugsweise mitverwendeten Steuerlaugestroms vermindert, damit der Stand des Kristallbetts nicht weiter abfällt, sondern sich auf einer gewünschten Höhe einstellt. Dann wird anwendungstechnisch zweckmäßig der Durchfluss des Auslass A geöffnet. Zu diesem Zweck ist am Auslass normalerweise ein Ventil oder eine andere Armatur montiert.

Der Durchlass wird lediglich so weit geöffnet, dass vom Kristallschmelzeraum noch ein ausreichender Flüssigkeitsstrom in das auf die Abtrageinrichtung zu bewegte Kristallbett aufsteigt.

Vorzugsweise wird man den Durchfluss des Auslass A zunächst noch soweit beschränken, dass die aus dem Kristallschmelzeraum aufsteigende Flüssigkeit bis an die Filter F aufsteigt und als Bestandteil des Ablaugestroms durch die Filter F und die Filterrohre hindurch aus dem Prozessraum herausgeführt wird (weniger vorteilhaft könnte die Steighöhe durch entsprechende Regelung des Durchfluss des Auslass A auch von Anfang an unterhalb der Unterkante der Filter F liegend eingeregelt werden).

Nun wird vorzugsweise die Temperaturregelung des Kristallschmelzekreises in Betrieb genommen und die Heizleistung des Wärmeübertrages W so geregelt, dass die Temperatur im Kristallschmelzekreis jenseits des Wärmeübertrages W um nicht mehr als 10 bzw. 5°C, vorzugsweise um nicht mehr als 3 oder 2°C und besonders bevorzugt um nicht mehr als 1°C (üblicherweise jedoch ≥ 0,01°C) oberhalb der Kristallbildungstemperatur abgetragener Reinkristalle aus deren Kristallschmelze heraus (14°C) liegt (am Anfang kann die Regelung noch von Hand erfolgen, bevor an die automatische Regelung übergeben wird; letztere wird mit Hilfe entsprechender Thermoelemente oder Widerstandsthermometer realisiert).

Schließlich wird anwendungstechnisch zweckmäßig kontinuierlich oder in bestimmten Zeitintervallen der Durchfluss des Auslasses A zunehmend geöffnet, bis auf der für die gewünschte Lage der Waschfront im Kristallbett unterhalb der Filter F ins Auge gefassten Kristallbetthöhe die als Regelgröße gewählte, zwischen 14°C und der Temperatur der dem Verteilerraum zugeführten Kristallsuspension liegende Kristallbetttemperatur (die Solltemperatur) vorliegt. Beispielsweise kann als Regelgröße (Solltemperatur) der arithmetische Mittelwert der beiden Temperaturen gewählt werden (vgl. DE-A 10036881 und WO 02/09839). Ab diesem Zeitpunkt kann der Durchlass des Auslasses A automatisch mit Hilfe der Abweichung der auf der für die Waschfrontlage vorgesehenen Kristallbetthöhe gemessenen Temperatur von der vorgenannten Solltemperatur geregelt werden. Ist die auf der für die Waschfrontlage vorgesehenen Kristallbetthöhe gemessene Temperatur niedriger als die ausgewählte Solltemperatur, wird der Durchfluss des Auslass A vermindert. Ist die auf der für die Waschfrontlage vorgesehenen Kristallbetthöhe gemessene Temperatur höher als die ausgewählte Solltemperatur, wird der Durchfluss des Auslass A erhöht (z. B. durch Öffnung des entsprechenden Regelventils).

Beispielsweise durch Regelung der Stärke des erfindungsgemäß bevorzugt mitverwendeten Steuerlaugestroms durch messtechnisches Erfassen des Stands des Kristallbetts (der Aufbaufront) gemäß der Lehre der WO 2006/111565 kann die Filtrationsfront vergleichsweise einfach auf der gewünschten Höhe gehalten werden.

Im weiteren Verlauf der Ausführung des Trennverfahrens wird anwendungstechnisch geschickt der Dosiermengenstrom der Inhibitorlösung in Reinprodukt im Verhältnis zum aus dem Auslass A herausgeführten Reinproduktmengenstrom automatisch geregelt (im Fall des molekularen Sauerstoff enthaltenden Gases wird die Überschusszudosierung mit nachfolgender Überschussabtrennung beibehalten).

Durch Probenahme vom aus dem Auslass A herausgeführten Stoffstrom und Analyse der Proben wird in zweckmäßiger Weise bestimmt, ab welchem Zeitpunkt der ausgelassene Stoffstrom die gewünschte Reinheit aufweist. Von diesem Zeitpunkt an kann der Auslassstrom z. B. in ein Reinprodukt-Tanklager geführt werden. Zuvor ausgelassene Stoffströme mit noch unzureichender Reinheit können z. B. zum Zweck ihrer Rekristallisation in den Kristallisationsprozess zur Erzeugung der Suspension S rückgeführt werden. Alternativ können sie auch in die Erzeugung des Stoffstroms rückgeführt werden, aus welchem durch Suspensionskristallisation die Suspension S erwächst (z. B. in die fraktionierende Kondensation des Produktgasgemischs einer zur Herstellung der Acrylsäure durchgeführten heterogen katalysierten partiellen Gasphasenoxidation von Propan und/oder Propylen, wie es z. B. in der WO 01/77056 und in der WO 08/090190 für die in der hydraulischen Waschkolonne abgetrennt Mutterlauge beschrieben ist.

Das Trennverfahren ist jetzt in seinen stationären Betriebszustand übergegangen.

Die Anzahl der Filterrohre in einer hydraulischen Waschkolonne kann im Fall einer großtechnischen Anwendung des erfindungsgemäßen Verfahrens 3 bis 200 oder mehr betragen. Bezogen auf die Einheit der Querschnittsfläche des Prozessraums der hydraulischen Waschkolonne beträgt ihre Anzahl in typischer Weise 10 bis 100 je $m^2$. Die Länge der Waschzone liegt typisch beim 0,5- bis 20-fachen, bevorzugt beim 1- bis 8-fachen und ganz besonders bevorzugt beim 2- bis 5-fachen des Abstandes des der Mantelwand des Prozessraums nächstliegenden Filterrohres zur Einhüllenden (in der Regel beträgt dieser Abstand 25 bis 500 mm, häufig 40 bis 250 mm, oft 80 bis 200 mm).

Typische Innendurchmesser der Filterrohre betragen beim erfindungsgemäßen Verfahren 5 bis 200 mm, häufig 10 bis 100 mm, vielfach 20 bis 80 mm. Die Wanddicke der Filterrohre liegt dabei regelmäßig bei 1 bis 10 mm. Wie bereits erwähnt sind die Filterrohre anwendungstechnisch zweckmäßig auf einer definierten Höhe mit einem Filter F versehen, das sich in der Regel um den gesamten Umfang eines Filterrohres erstreckt. Die Höhe der Filterelemente F liegt häufig bei 20 bis 200 mm. Die die filtrierende Wirkung des Filterelements F bedingende Perforation desselben kann sowohl gelocht, als auch längs geschlitzt ausgeführt sein. Die Schlitzbreite bzw. der Lochdurchmesser betragen bevorzugt 50 bis 400, z.B. 100 bis 300 µm beim erfindungsgemäßen Verfahren. Außen- und Innendurchmesser eines Filterrohres sind erfindungsgemäß bevorzugt über seine Länge konstant. Erfindungsgemäß vorteilhaft weisen die Filterrohre einer erfindungsgemäß geeigneten hydraulischen Waschkolonne eine einheitliche Gestalt auf. An das Filterelement F eines Filterrohres schließt sich nach unten normalerweise, wie bereits gesagt, ein Filterrohrverdränger (38) an. In ihn vermag keine Flüssigkeit einzudringen. Er kann sowohl zylindrisch, konisch oder als Kombination dieser Formen ausgeführt sein. Der Anschlussaußendurchmesser ist in der Regel identisch mit dem Außendurchmesser des Filterelements. Erfindungsgemäß bevorzugt besteht der Filterrohrverdränger aus einem Material mit einer geringen Wärmeleitfähigkeit (z. B. Teflon oder Polyethylen). Die Länge der Filterrohrverdränger beträgt regelmäßig 50 oder 100 bis 500 mm).

Die Länge der Waschzone beträgt beim erfindungsgemäßen Verfahren in der Regel 50 bis 500 mm. Die Gesamthöhe des verdichteten Kristallbetts (des verdichteten Filterkuchens) in einer hydraulischen Waschkolonne beträgt beim erfindungsgemäßen Verfahren typisch 300 bis 4000 mm, häufig 400 bis 3000 mm, vielfach 500 bis 2000 mm, oder 600 bis 1500 mm bzw. bis 1000 mm.

Der Treibdruck (angegeben als Überdruck gegen Atmosphäre) in einer hydraulischen Waschkolonne beträgt häufig bis zu 10 bar, vielfach bis zu 8 bar und oft 1 bis 5 bar bzw. 0,5 bis 4 bar. Der hydraulische Strömungsdruckverlust der Mutterlauge der zugeführten Suspension S beträgt in der Regel ≥ 100 mbar bis ≤ 5 bar bzw. ≤ 10 bar. Hinsichtlich der Verteilung der Filterrohre einer hydraulischen Waschkolonne über den Querschnitt derselben wird erfindungsgemäß vorteilhaft wie in der EP-A 1 448 282 empfohlen verfahren. Die Länge der Filterrohre (ohne Einbezug der Filterrohrverdränger) entspricht der Länge L des Prozessraums abzüglich der oben angegebenen Länge für die Waschzone.

Bedingt durch die von den Verunreinigungen bewirkte Gefrierpunkterniedrigung liegt die Temperatur der dem Verteilerraum der hydraulischen Waschkolonne zugeführten Suspension S in notwendiger Weise tiefer, als die Kristallbildungstemperatur des aus dem Auslass A ausgelassenen Reinproduktes (der Waschschmelze). Im Bereich der Waschfront kommt es daher zu einem Temperaturausgleich der aus der kalten Suspension S stammenden kalten Kristalle mit der Waschschmelze, bei dem die Waschschmelze teilweise oder vollständig rekristallisiert (diese Rekristallisation bildet einen weiteren Reinigungsmechanismus). Dadurch wird wenigstens ein Teil der Waschschmelze zurückgewonnen. Die beschriebene Rekristallisation der Waschschmelze trägt zur Stabilisierung und Ausbildung der Waschfront bei und ist umso ausgeprägter, je weiter die Temperatur der Suspension S unterhalb der Kristallbildungstemperatur der Waschschmelze liegt. Grundsätzlich kann die vorgenannte Differenztemperatur, die sich unter anderem durch den gewählten Kristallisationsgrad der Suspension S beeinflussen lässt, 15°C und mehr betragen. Vielfach wird eine Differenztemperatur von 4 bis 10°C und bei geringem Verunreinigungsgehalt der Mutterlauge oft auch nur von 2 bis 4°C eingestellt. Gelingt quantitative Rekristallisation (die Waschfront liegt unterhalb der Unterkante der Filter F), können aus dem Kristallschmelzekreis letztlich 100 % des dem Prozessraum zugeführten Kristallstroms als Reinproduktstrom abgeführt werden (beide Ströme weisen im Wesentlichen die selbe Massenstromstärke auf;(es geht keine Waschschmelze verloren). Wird der Unterschied der Temperatur der Suspension S und der Waschschmelze zu groß, so kann daraus ein Verschluss der Poren im verdichteten Kristallbett resultieren, was einer ordnungsgemäßen Durchführung des Abtrennverfahrens abträglich ist.

Mit einer Lage der Waschfront an den Filtern F, geht normalerweise eine nicht quantitative Rekristallisation des aufwärts steigenden Waschschmelzestroms einher. Ein Teilstrom desselben wird demzufolge als Bestandteil des Ablaugestroms abgeführt.

Liegt der bei der erfindungsgemäßen Inbetriebnahme bis zum Zeitpunkt $t_S$ während der Zufuhr des Stroms ST* durch die Filter F der Filterrohre zum jeweiligen Zeitpunkt insgesamt strömende Ablaugestrom SM*, geteilt durch die Gesamtfläche aller Filter F, überwiegend bei besonders niederen Werten (z. B. < 20m$^3$/(m$^2 \cdot$ h)), so steigt damit die Möglichkeit, dass bei der Inbetriebnahme des Kristallschmelzekreises hinter dem Zeitpunkt ts der Waschschmelzstrom ungewollt bis zu den Filtern F steigt. Eine an selbigen einsetzende Rekristallisation vermag die Filteröffnungen gegebenenfalls zu verstopfen und die Anwendung einer in der EP-A 1 448 282 empfohlenen Spülflüssigkeit (übliche Spülsäureströme (z.B. leicht erwärmte Mutterlaugeströme) je Filter F liegen bei 10 bis 1000 I/h, bevorzugt bei 50 bis 200 I/h) erforderlich machen. Auf die Gesamtfläche der Filter F normierte Ablaugeströme ≥ 20 m$^3$/(m$^2 \cdot$h) sind daher bei der erfindungsgemäßen Inbetriebnahme bevorzugt.

Für die Durchführung des erfindungsgemäßen Verfahrens ist es ferner vorteilhaft, den Querschnitt der hydraulischen Waschkolonne kurz vor (von oben betrachtet) der rotierenden Abtrageinrichtung geringfügig zu vergrößern (um 5 bis 100 mm bezogen auf seinen Durchmesser). Dies ermöglicht es, die radiale Ausdehnung der Abtrageinrichtung etwas größer als die radiale Ausdehnung des Kristallbetts zu wählen (sie kann grundsätzlich aber auch kleiner als diese sein), was den gleichmäßigen Abtrag an Kristallen über den gesamten Kristallbettquerschnitt begünstigt. Zur Verbesserung der Suspendierung der durch die rotierende Abtrageinrichtung abgetragenen Kristalle in der im Kristallschmelzeraum befindlichen Kristallschmelze ist es hilfreich, an der Antriebswelle für die Abtrageinrichtung unterhalb der letzteren Paddel zu befestigen, die den Kristallschmelzeraum durchmischen. Diesem Zweck können auch Verstärkungselemente (z.B. Verstärkungsrippen oder Lamellen, die in der Regel Durchtrittsöffnungen aufweisen) dienen, die zwischen der zur Befestigung der Abtrageinrichtung an der Welle verwendeten Nabe und der Abtrageinrichtung großflächig ausgeführt werden sowie an der Innenwand des Kristallschmelzraums befestigte Stromstörer (vgl. zu beiden Elementen die Figur 2 der EP-A 1 448 282).

Die Durchgänge U das wenigstens einen Bodens B verfolgen beim erfindungsgemäßen Verfahren den Zweck, einer über den Querschnitt des Prozessraums möglichst gleichmäßigen Zufuhr von Suspension S aus dem Verteilertraum heraus in den Prozessraum hinein. Erfindungsgemäß vorteilhaft sind die Durchgänge U gleichmäßig über den Boden B verteilt. Vorzugsweise weisen die Durchgänge U kreisrunde Öffnungen und einen längs des Durchgangs U bevorzugt konstanten Querschnitt auf.

Die Öffnungen der Durchgänge U weisen mit Vorteil Querschnittsflächen auf, die, bezogen auf eine kreisrunde Form, einem Durchmesser von 15 bis 300, bevorzugt von 50 bis 150 mm entsprechen.

Die Höhe (Länge) der Durchgänge U kann beim erfindungsgemäßen Verfahren bis zu 1000 mm betragen (jeweils gemessen vom Verteilerraum bis zum Prozessraum). In der Regel beträgt sie wenigstens 50 bis 200 mm. Häufig liegt sie bei 400 bis 800 mm. Das Verhältnis der Gesamtfläche aller dem Prozessraum zugewandten Öffnungen der Durchgänge U zur Gesamtfläche des Prozessraumquerschnitts beträgt beim erfindungsgemäßen Verfahren häufig 0,10 bis 0,60 und vielfach 0,20 bis 0,40.

Zusätzlich zum Durchgänge U aufweisenden Boden B kann der Verteilerraum gemäß der Empfehlung der EP-A 1 448 282 Verteilerhilfen enthalten, die für eine möglichst gleichmäßige Zufuhr der Suspension S (über den Querschnitt des Prozessraums betrachtet) aus dem Verteilerraum in den Prozessraum förderlich sind. Als solche Verteilerhilfen kommen z.B. im Verteilerraum untergebrachte Packungen in Betracht. Eine solche Verteilerhilfe kann aber auch ein Rührer sein, der den Inhalt des Verteilerraums umrührt und dadurch möglichst homogen hält. Weitere mögliche Verteilerhilfen sind z.B. ein Verteilerkonus gemäß Figur 2 der EP-A 1 448 282 oder Leitbleche gemäß Figur 7 der EP-A 1 448 282. Als Verteilerhilfe kommen aber auch ineinander geschachtelte "Trichter" in Betracht, wie es die Figur 2 dieser Schrift zeigt (46). Die Trichterhälse ragen dabei jeweils in den Zulaufstützen des Verteilerraums und die Trichterköpfe ragen in den Verteilerraum. Zwischen den beabstandeten Oberflächen der Trichterköpfe läuft die Suspension S dem Querschnitt des Verteilerraums über selbigen gleichmäßig verteilt zu. Die Platzierung eines Verdrängerkörpers (43) im Zentrum des Prozessraums der hydraulischen Waschkolonne, wie es die EP-A 1 448 282 empfiehlt, ist zur Vergleichsmäßigung der Kristallbettausbildung ebenfalls vorteilhaft. Der zentrale Verdrängerkörper weist normalerweise eine kreiszylindrische Gestalt auf, deren Außendurchmesser üblicherweise größer ist als derjenige eines Filterrohres. Grundsätzlich kann aber auch der zentral platzierte Verdrängerkörper auf der Höhe der Filter F der Filterrohre ein Filter entsprechender Höhe wie die Filter F aufweisen und in seinem Inneren hohl ausgeführt sein, wodurch er die Wirkung eines größeren Filterrohres übernimmt. In diesem Fall ist der Verdrängerkörper für die Belange der vorliegenden Erfindung wie ein Filterrohr zu berücksichtigen und einzubeziehen. Für das erfindungsgemäße Verfahren besonders gut geeignete hydraulische Waschkolonnen sind somit insbesondere jene, die den Ausführungen der EP-A 1 448 282 sowie der DE Anmeldenummer 102009000987.6 folgen. Eine Ausführungsform einer derartigen hydraulischen Waschkolonne zeigt die Figur 2 dieser Schrift.

Erfindungsgemäß vorteilhaft wird bei der hydraulischen Waschkolonne (0) gemäß Figur 2 der Verteilerraum (A) vom Prozessraum (B) durch einen Boden B (32) und durch einen weiteren Boden B* (39) getrennt, die ihrerseits einen zylindrischen Raum definieren. Beide Böden weisen Öffnungen auf (vorzugsweise kreisrunde Bohrungen (Löcher)), von denen die Öffnungen des Bodens B* (39) mit einem Teil (dem zweiten Teil) der Öffnungen des Bodens B (32) über durchgängige Verbindungsstücke, die Durchgänge U (26), verbunden sind (beide Öffnungen münden in einen Durchgang U (26)). Über die Durchgänge U (26) gelangt die aufzutrennende Suspension S in den Prozessraum (B) der Waschkolonne (0).

Der Boden B (32) weist zusätzlich einen ersten Teil an Öffnungen auf, die kein Gegenstück im Boden B* (39) aufweisen und die in die Filterrohre (6) münden. Vorzugsweise handelt es sich auch bei diesem zweiten Teil von Öffnungen um kreisrunde Bohrungen (Löcher).

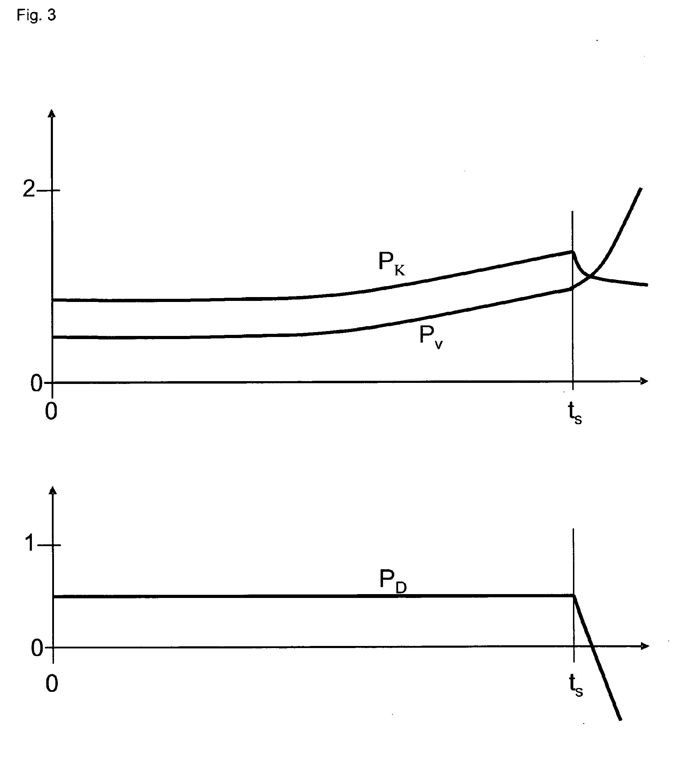

Dieser erste Teil von Öffnungen ist ebenso wie die Filterrohre (6), die in diese Öffnungen münden, bevorzugt gleichmäßig über den Querschnitt des Bodens B (32) verteilt, wie es z.B. die Figur 3 der EP-A 1 448 282 zeigt.

Durch diese Gleichverteilung werden überwiegend gleichseitige Dreiecke definiert. Erfindungsgemäß vorteilhaft sitzt der zweite Teil der Öffnungen des Bodens B (32) im Zentrum solcher Dreiecke. Vorzugsweise ist dieser zweite Teil der Öffnungen ebenfalls gleichmäßig über den Querschnitt des Bodens B (32) verteilt. Erfindungsgemäß von Vorteil ist es, wenn im Wesentlichen alle Dreieckszentren durch zum zweiten Teil gehörige Öffnungen besetzt werden.

Der Raum um die Durchgänge U (26) bildet den Sammelraum (27) für die Ablauge, die aus der Waschkolonne herausgeführt wird (2).

Von Vorzug ist es für das erfindungsgemäße Verfahren, wenn zwischen dem Ablaugesammelraum (27) und dem Suspensionsverteilerraum (A) als ein weiterer Raum ein Spülflüssigkeitszuführraum (40) vorhanden ist, der durch Einziehen eines Zwischenbodens B** (41) geschaffen werden kann. Die Durchgänge U (26) werden dabei im Wesentlichen abdichtend durch den Zwischenboden B** (41) geführt. Zusätzlich weist der Zwischenboden B** (41) Öffnungen (bevorzugt kreisrunde Bohrungen (Löcher)) auf, in die Spülrohre (42) münden, die in gleicher Weise wie die Filterrohre (6) über den Querschnitt verteilt sind und bis in das untere Drittel des jeweiligen Filterelements (7) in ein zughöriges Filterrohr (6) hineinragen. Der Außendurchmesser der Spülrohre (42) wird erfindungsgemäß zweckmäßig so gewählt, dass er dem 0,3- bis 0,6-fachen des Innendurchmessers der Filterrohre (6) entspricht. Im unteren Drittel sind an der Außenwand jedes Spülrohrs (42) mit Vorteil Zentriernocken angebracht, die eine zentrierte Position des jeweiligen Spühlrohrs (42) im zugehörigen Filterrohr (6) sicherstellen.

Die Öffnungen im Boden B** (41) sind vorteilhaft so beschaffen, dass sie wahlweise geschlossen oder geöffnet werden können. Sind sie geschlossen, kann in den Raum (40) ein beliebiges Heizmedium eingespeist werden, um kristalline Ablagerungen und Inkrustationen auf dem Boden B* (39) und am Eintritt in die Durchgänge U (26) aufzuschmelzen. Sind Öffnungen im Boden B** (41) geöffnet, können z.B. durch Verwendung von erwärmter, zuvor aus der Waschkolonne herausgeführter Ablauge, kristalline Ablagerungen an den Filterelementen F (7) aufgeschmolzen werden.

Erfindungsgemäß vorteilhaft wird bei der reinigenden Abtrennung von Acrylsäurekristallen aus einer Suspension S ihrer Kristalle in Mutterlauge mittels einer hydraulischen Waschkolonne so verfahren, dass sowohl bei der Inbetriebnahme des Trennverfahrens als auch in seinem stationären Betrieb stetig ein kleiner zuvor erwärmter (auf Temperaturen im Bereich von 14 bis 20 oder bis 25°C) Teilstrom (bezogen auf den aus der hydraulischen Waschkolonne insgesamt herausgeführten Ablaugestrom bis zu 40 bzw. bis zu 25%, in der Regel wenigstens 5%) des insgesamt aus der Waschkolonne herausgeführten Ablaugestroms über alle Spülrohre (42) verteilt als Präventivmaßnahme in die zugehörigen Filterrohre (6) geleitet wird, um die Ausbildung von Inkrustationen auf deren Filterelementen F (7) zu verhindern. Die vorgenannte Erwärmung wird dabei anwendungstechnisch zweckmäßig durch Zwangsförderung des Teilstroms mittels einer entsprechenden Förderpumpe durch einen von einem Wärmeträger durchströmten indirekten Wärmeaustauscher vorgenommen (vgl. Figur 7 der EP-A 1 448 282). An die Filterelemente F (7) schließen sich normalerweise die Filterrohrverdränger (38) an. Im Zentrum des Prozessraums (B) der Waschkolonne (0) befindet sich vorzugsweise ein kreiszylindrisch ausgeführter zentraler Verdrängerkörper (43). Hinsichtlich dessen Ausmaße und materieller Beschaffenheit wird auf die Ausführungen der EP-A 1 448 282 verwiesen. Bevorzugt ist der zentrale Verdrängerkörper (43) am Boden B (32) statisch befestigt (der im Befestigungsbereich in der Regel keinen Durchlass, keine Öffnung aufweist) und ragt bis etwa 1 bis 20 mm über die Abtrageinrichtung (16) (z.B. eine Messerscheibe). Er kann jedoch auch fest mit der Messerscheibe verbunden und dadurch mit selbiger rotierend ausgeführt sein. Von der Antriebswelle (18) im Kristallschmelzeraum (C) radial weglaufende, mit Lochöffnungen ausgerüstete Lamellen (44) bzw. Stege tragen (stützen) die Abtragerichtung (Messerscheibe) (16) anwendungstechnisch zweckmäßig.

Eine Kombination aus einem Verteilerkonus (45) und einer über diesem angeordneten Verschachtelung von zu einander beabstandeten Trichtern (46) fungiert im Verteilerraum A als zusätzliche Verteilerhilfe. Dabei laufen die Hälse der Trichter im Zulaufstutzen (47) des Verteilerraumes aus und die Köpfe der Trichter ragen über den Verteilerkonus. Eine Berstscheibe (48) in der Förderverbindung E2 (34) sichert gegen sicherheitstechnisch unzulässige Überdrucke ab. Im Mischer (49) werden die Förderverbindung E2 (34) und die Förderverbindung C2 (36) zusammengeführt. Die Zusammenführung der beiden Förderverbindungen E2, C2 erfolgt anwendungstechnisch zweckmäßig in Gestalt von koaxial geführten Rohrleitungen. Die Suspension S wird dabei vorteilhaft in der inneren der beiden Rohrleitungen und die Steuerlauge in der äußeren Rohrleitung geführt. Eine Mischstrecke vor dem Eintritt in den Zulaufstutzen läuft die innen geführte Rohrleitung aus und nur die äußere Rohrleitung wird als dann gemeinsame Förderverbindung E2, C2 zum Verteilerraum weitergeführt. Die innen geführte Rohrleitung verjüngt sich zu ihrem Auslauf hin konisch und fungiert an ihrem Auslauf als Treibdüse mit der Suspension S als Treibstrahl, der auf der sich anschließenden Mischstrecke von der außen geführten Steuerlauge ansaugt und sich dadurch mit selbiger vermischt.

Das Manometer M3 ist vorteilhaft ein Differenzdruckmanometer vom Typ 3051 CD Differenzmembransensor, Meßbereich: 0 bis 500 mbar, der Lieferfirma Rosemount, das den unmittelbaren Zugriff auf den Druckunterschied zwischen Meßstellen unmittelbar oberhalb eines Durchgangs U (26) im Verteilerraum und unmittelbar unterhalb eines Durchgangs U (26) im Prozessraum ermöglicht. Im Übrigen kommt identischen Adressen in der Figur 2 die gleiche Bedeutung wie in der Figur 1 zu.

Die Figur 3 dieser Schrift zeigt qualitativ den Verlauf der Drücke $P_K$ und $P_V$ sowie des Differenzdrucks $P_D$ bis zum Durchschreiten des Zeitpunkts ts. Die Abszisse zeigt dabei die Zeit (ts liegt in der Regel im Bereich von 10 min bis 2 h) und die Ordinate zeigt die Drucke in bar. Bei der der Figur 3 zugrunde liegenden Inbetriebnahme wurde die hydraulische Waschkolonne, einschließlich ihres Schmelzkreises, zunächst vollständig mit Mutterlauge der zu behandelnden Suspension S als Anfahrflüssigkeit AT befüllt.

Das erfindungsgemäße Verfahren der Inbetriebnahme eines Trennverfahrens zur reinigenden Abtrennung von Acrylsäurekristallen aus einer Suspension S ihrer Kristalle in Mutterlauge ist insbesondere dann vorteilhaft, wenn die Suspension S, bezogen auf ihren molaren Gehalt an Acrylsäure, einen vergleichsweise hohen molaren Gesamtgehalt an von Acrylsäure verschiedenen Bestandteilen aufweist.

Somit eignet sich das erfindungsgemäße Verfahren insbesondere zur Abtrennung von Kristallen der Acrylsäure, die auf das Produktgasgemisch einer heterogen katalysierten partiellen Gasphasenoxidation einer $C_3$-Vorläuferverbindung von Acrylsäure (z.B. Propan, Propylen, Acrolein, Propionsäure, Propanol, Glyzerin und/oder Propionaldehyd) zurückgeht (vgl. z.B. WO 2004/035514, DE-A 10 2007 004960, DE-A 102 43625 und DE-A 103 23758).

Demgemäß kommen als erfindungsgemäß geeignete Suspensionen S z.B. all jene in Betracht, die in den Schriften DE-A 10 2007 043759, WO 01/77056, DE-A 10 2007 043758, DE-A 10 2007 043748 und DE-A 10 2007 004960 offenbart werden.

Solche Suspensionen S können z.B. einen der nachfolgenden Sätze an Gehalten aufweisen:

$\geq$ 70 Gew.-% Acrylsäure,
bis zu 15 Gew.-% Essigsäure,
bis zu 5 Gew.-% Propionsäure,
bis zu 5 Gew.-% niedermolekulare Aldehyde,
bis zu 3 Gew.-% Polymerisationinhibitoren,
0 zu 5 Gew.-% Diacrylsäure (Michael-Addukt), und
bis zu 25 Gew.-% Wasser;

oder

$\geq$ 80 Gew.-% Acrylsäure,
$\geq$ 100 Gew.ppm bis $\leq$ 10 Gew.-% Essigsäure,
$\geq$ 10 Gew.ppm bis $\leq$ 5 Gew.-% Propionsäure,
bis zu 5 Gew.-% niedermolekulare Aldehyde,
bis zu 3 Gew.-% Polymerisationsinhibitoren,
0 bis 5 Gew.-% Diacrylsäure (Michael-Addukt), und
bis zu 10 Gew.-% Wasser;

oder

$\geq$ 90 Gew.-% Acrylsäure,
$\geq$ 100 Gew.ppm bis $\leq$ 5 Gew.-% Essigsäure,
$\geq$ 10 Gew.ppm bis $\leq$ 2 Gew.-% Propionsäure,
bis zu 2 Gew.-% niedermolekulare Aldehyde,
bis zu 2 Gew.-% Polymerisationsinhibitoren,
0 bis 3 Gew.-% Diacrylsäure (Michael-Addukt), und
bis zu 9 Gew.-% Wasser;

oder

≥ 95 Gew.-% Acrylsäure,
≥ 100 Gew.ppm bis ≤ 3 Gew.-% Essigsäure,
≥ 10 Gew.ppm bis ≤ 2 Gew.-% Propionsäure,
bis zu 2 Gew.-% niedermolekulare Aldehyde,
bis zu 2 Gew.-% Polymerisationsinhibitoren,
0 bis 2 Gew.-% Diacrylsäure (Michael-Addukt), und
bis zu 4,9 Gew.-% Wasser;

oder

93 bis 98 Gew.-% Acrylsäure,
1 bis 5 Gew.-% Wasser,
0,001 bis 3 Gew.-% Acrolein,
≥ 0 bis 3 Gew.-% Methacrolein,
≥ 0 bis 3 Gew.-% Methacrylsäure,
0,1 bis 3 Gew.-% Essigsäure,
0,01 bis 3 Gew.-% Propionsäure,
0,001 bis 3 Gew.-% Formaldehyd,
0,001 bis 3 Gew.-% von Formaldehyd verschiedene Aldehyde,
0,01 bis 3 Gew.-% Maleinsäure, und
≥ 0 bis 3 Gew.-% Protoanemonin.

Alles Vorgenannte gilt insbesondere dann, wenn die Suspension S wenigstens 0,1 Gew.-% Wasser enthält.

Ferner gilt es insbesondere dann, wenn die Suspension S nicht mehr als 99 bzw. nicht mehr als 98 Gew.-% Acrylsäure enthält.

Natürlich gilt es auch dann, wenn beide vorgenannten Bedingungen gleichzeitig erfüllt sind.

D.h., bei Suspensionen S, die ≥ 70 bis ≤ 99 Gew.-% Acrylsäure und ≥ 0,1 Gew.-% Wasser (häufig ≤ 20 Gew.-%, oder ≤ 10 Gew.-% Wasser) enthalten, ist das erfindungsgemäße Verfahren besonders vorteilhaft anwendbar.

Befindet sich ein Trennverfahren zur reinigenden Abtrennung von Acrylsäurekristallen aus einer Suspension S ihrer Kristalle in Mutterlauge mit einer hydraulischen Waschkolonne in einem stationären Betriebszustand (ein solcher Zustand ist dadurch charakterisiert, dass die Betriebsbedingungen in Abhängigkeit von der Betriebsdauer im Wesentlichen unverändert beibehalten werden), kann es aufgrund einer plötzlich veränderten Marktnachfrage erforderlich sein, den Durchsatz durch die hydraulische Waschkolonne zu erhöhen oder zu erniedrigen, um die Stärke des durch den Auslass A auszulassenden Reinproduktstroms zu erhöhen oder zu mindern.

Normalerweise geht eine solche Durchsatzsteigerung in notwendiger Weise mit einer Steigerung der Stromstärke des dem Verteilerraum zugeführten Stroms der Kristallsuspension S auf einen neuen stationären Wert einher, während die sonstige Beschaffenheit der Suspension S normalerweise im Wesentlichen unverändert bleibt. Anwendungstechnisch vorteilhaft wird eine solche Erhöhung der Belastung der hydraulischen Waschkolonne mit Suspension S wie folgt umgesetzt. Die Erhöhung erfolgt in einer Schrittgröße von 2 bis 10% des jeweiligen Ausgangswertes. Nach jedem Schritt wird eine Haltezeit von 5 bis 30 min. eingehalten. Wenn sich dabei der Bettstand (die Aufbaufront) in seiner Lage unerwünscht verändert, wird eine entsprechende Anpassung des Steuerlaugestroms vorgenommen.

Bei einer Absenkung der Belastung einer, zuvor in einem stationären Betriebszustand betriebenen, hydraulischen Waschkolonne mit Suspension S (z.B. als Reaktion auf eine sinkende Reinproduktnachfrage) kann dagegen vorteilhaft wie folgt vorgegangen werden. Beim Absenken der Belastung (der Last) kann in einem Schritt eine Absenkung von bis zu 50% des jeweiligen Ausgangswertes vorgenommen werden. Ein Absenken mit entsprechend bezogenen Schrittgrößen von 5 bis 20% ist jedoch ebenfalls möglich. Eine sich jeweils anschließende Wartezeit von 1 bis 20 min. ist auch hier zweckmäßig, jedoch nicht unabdingbar.

Es empfiehlt sich in entsprechender Weise vorzugehen, wenn sich zusätzlich oder für sich der Kristallisationsgrad der Suspension S und/oder die Kristallgröße der in der Suspension S suspendiert enthaltenen Kristalle aus einem stationären Betriebszustand heraus plötzlich ändert.

Um ein, wie in dieser Schrift beschrieben, im stationären Betriebszustand befindliches (d.h., alle Antriebe der Trennvorrichtung samt zugehöriger Stoffströme des Trennverfahrens (einschließlich einer steten Zufuhr von erwärmter Spüllauge durch alle Spühlrohre (42)) sind in Betrieb) Trennverfahren zur reinigenden Abtrennung von Acrylsäurekristallen aus einer Suspension S ihrer Kristalle in Mutterlauge mit einer eine hydraulische Waschkolonne umfassenden Trennvorrichtung (z.B. der in Figur 2 gezeigten; alle nachfolgend verwendeten numerischen und alphabetischen Adressen beziehen sich auf diese Figur 2) regulär zu beenden (außer Betrieb zu nehmen), kann die Abfolge der diesbezüglich zu ergreifenden Maßnahmen grundsätzlich beliebig gewählt werden (d.h., es ist nicht unabdingbar, eine spezielle Reihenfolge der zu ergreifenden Einzelschritte einzuhalten).

Anwendungstechnisch vorteilhaft wird man zu einer solchen Außerinbetriebnahme jedoch die nachfolgend ausgeführte Abfolge einhalten:

1. Als erste Maßnahme wird die Förderpumpe P2 (8) abgeschaltet, die im stationären Betrieb die Suspension S von Acrylsäurekristallen in Mutterlauge in den Verteilerraum (A) der hydraulischen Waschkolonne pumpt.

2. Durch Beibehaltung des Betriebs der Abtrageinrichtung (Messerscheibe) (16) und des von der Förderpumpe P3 (13) geförderten Steuerlaugestroms (dessen Stromstärke im Regelfall beibehalten oder bedarfsgerecht erhöht wird) wird das im Prozessraum (B) befindliche Kristallbett so weit wie möglich aus selbigem herausgefördert (ein solches Ausfördern (Ausfahren) des Kristallbetts ist im Wesentlichen soweit möglich, bis Teilflächen der Filter F (7) aus dem noch vorhandenen Restkristallbett herauszuragen beginnen).

3. Durch Erhöhen der Temperatur im Kristallschmelzekreis (31) auf Werte oberhalb von 15°C und ≤ 35°C (z.B. durch Erhöhen der Stromstärke des dem Wärmeübertrager W (9) zugeführten Wärmeträgermediums) und durch weiter zugeführte und Temperaturen oberhalb von 15°C und ≤ 35°C aufweisende Spüllauge durch den Spülflüssigkeitszuführraum (40) und die Spülrohre (42) wird der beim Ausfahren des Kristallbetts noch in der Waschkolonne verbliebene Kristallbettrest aufgeschmolzen. Während des Aufschmelzens laufen die Abtrageinrichtung (16) und die Förderpumpe P3 (13) für den Steuerlaugestrom vorzugsweise weiter, um die warme Spüllauge intensiver mit den verbliebenen Kristallen in Kontakt zu bringen und so deren Aufschmelzen zu fördern. Während der weiteren Zufuhr von warmer Spüllauge wird anwendungstechnisch vorteilhaft durch den regelbaren Durchfluss (10) hindurch und aus dem Auslass A (3) heraus fortwährend ein Acrylsäurestrom aus dem Kristallschmelzekreis herausgeführt (z.B. in einen Auffangbehälter hinein; aus diesem wird diese Acrylsäure anwendungstechnisch zweckmäßig z.B. in die Kondensationskolonne rückgeführt, in der die kristallisativ zu reinigende Acrylsäure eventuell aus dem Produktgasgemisch einer heterogen katalysierten partiellen Gasphasenoxidation zur Herstellung von Acrylsäure durch z.B. fraktionierende Kondensation abgetrennt wurde; vgl. z.B. WO 2001/077056). Auf diese Weise wird eine zunehmende Vermischung der im Kristallschmelzekreis (31) zu Beginn des Vorgangs der Außerinbetriebnahme ausschließlich vorliegenden bereits gereinigten Acrylsäure (Reinacrylsäureschmelze) mit der als Spüllauge verwendeten, Polymerisationsinhibitor angereichert enthaltenden, Ablauge (Mutterlauge + Steuerlauge) bewirkt, was den Kristallschmelzekreis zusätzlich stabilisiert und einer unerwünschten radikalischen Polymerisation der Acrylsäure in selbigem entgegenwirkt).

4. Nun wird die während des stationären Betriebs zum Zweck der Stabilisierung des Kristallschmelzekreises (31) in diesen hinein erfolgte Zufuhr von molekularen Sauerstoff enthaltendem Gas und von polymerisationsüberinhibierter Reinacrylsäure abgestellt.

5. Während der Einzelschritte 2 bis 4 wird aus dem Auslass A (3) kein Volumenstrom herausgeführt, der größer als der zugeführte Volumenstrom an warmer Spüllauge ist, so dass der Prozessraum der Waschkolonne stets vollständig mit kondensierter Materie befüllt ist. Durch Temperaturfühler (z.B. Thermoelemente), die im stationären Betrieb auch zur Regelung der Position der Waschfront verwendet werden können, wird die Temperatur der den Prozessraum der Waschkolonne einnehmenden kondensierten Phase stetig ermittelt. Zeigen diese Temperaturmessfühler während einer Zeitdauer von wenigstens 5 Minuten, vorzugsweise von wenigstens 10 Minuten, eine Temperatur im Bereich von > 16°C bis 20°C an, kann davon ausgegangen werden, dass das gesamte beim Ausfahren noch in der Waschkolonne verbliebene Kristallisat aufgeschmolzen ist. Jetzt wird die Zufuhr des Wärmeträgermediums in den Wärmeübertrager W (9) abgestellt. Daran anschließend werden die Abtrageinrichtung (16) und die Förderpumpe P(3) (13) für den Steuerlaugestrom sowie die Förderpumpe P(1) (11) für den Kristallschmelzekreis (31) abgestellt.

6. Nun wird die Zufuhr von warmer Spüllauge beendet.

7. Die in der Waschkolonne befindliche Flüssigkeit (flüssige Acrylsäure) wird nun bis zur vollständigen Entleerung abgelassen (z.B. kann an dem in Figur 2 am unteren Ende (rechts) angebrachten Stutzen ein Entleerventil angebracht sein, über welches die Entleerung erfolgt).

Die so außer Betrieb genommene und vollständig entleerte Waschkolonne kann später gemäß der erfindungsgemäßen Verfahrensweise wieder in Betrieb genommen werden.

An dieser Stelle sei noch festgehalten, dass das Aufschmelzen des nicht aus der Waschkolonne ausgetragenen Restkristallbetts beim Außerbetriebnahmevorgang auch durch einen entsprechenden Eintrag von warmem Wasser (z.B. 20 bis 25°C) vorgenommen werden kann. Der Warmwassereintrag kann dabei unmittelbar in den Kristallschmelzekreis hinein oder als "Steuerlauge" und/oder als "Spüllauge" vorgenommen werden.

Muss ein, wie in dieser Schrift beschrieben, im stationären Betriebszustand befindliches Trennverfahren zur reinigenden Abtrennung von Acrylsäurekristallen aus einer Suspension S ihrer Kristalle in Mutterlauge mit einer eine hydraulische Waschkolonne umfassenden Trennvorrichtung (z.B. der in Figur 2 gezeigten) wegen einer plötzlich auftretenden Betriebsstörung außer Betrieb genommen (beendet) werden, wird vorzugsweise ebenfalls die vorstehend für eine reguläre (normale) Beendigung ausgeführte Schrittfolge 1 bis 7 angewendet. Ist der eine oder andere Schritt dabei störungsbedingt nicht ausführbar, so entfällt er regelmäßig und die Außerbetriebnahme wird üblicherweise mit dem numerisch nächsten aufgeführten Schritt fortgesetzt. Kann beispielsweise das Kristallbett wegen einer Störung der Abtrageinrichtung (16) oder der Förderpumpe P3 (13) für die Förderung des Steuerlaugestroms nicht weitestgehend aus dem Prozessraum der hydraulischen Waschkolonne ausgefahren werden, entfällt der Schritt 2 und das gesamte dann noch in der Waschkolonne befindliche Kristallbett wird gemäß der Schrittfolge 3 bis 5 aufgeschmolzen. In jedem Fall ist es jedoch anwendungstechnisch zweckmäßig, als erste Maßnahme (als ersten Schritt) der Außerbetriebnahme die Förderpumpe P2 (8) abzuschalten, und damit die Förderung von Suspension S von Acrylsäurekristallen in Mutterlauge in den Verteilerraum (A) der hydraulischen Waschkolonne zu unterbinden.

Besteht aufgrund der aufgetretenen Betriebsstörung keine Möglichkeit, zu einem Wärmeeintrag mittels des Wärmeübertragers W (9) und durch die Spülrohre (42) zugeführter erwärmter Spüllauge, kann noch in der Waschkolonne befindliches, im Schritt 2 nicht ausgefahrenes Kristallisat infolge des nicht mehr vorhandenen Kälteeintrags durch zugeführte Suspension S auch durch den natürlichen Wärmeeintrag der laufenden Förderpumpen P3 (13) für den Steuerlaugestrom und/oder P1 (11) für den Kristallschmelzekreis (31) langsam aufgeschmolzen werden.

Generell ist bei einer Außerbetriebnahme zu vermeiden, dass im Verlauf der Außerbetriebnahme (des Abfahrens) eine eine erhöhte Temperatur aufweisende ($\geq 20°C$ bzw. $\geq 25°C$) Reinacrylsäure im Kristallschmelzekreis vorliegt. Ferner ist zu vermeiden, dass nach der Außerbetriebnahme flüssige Acrylsäure über längere Zeitdauern (mehrere Tage (bereits $\geq 2$ Tage können kritisch sein) bis mehrere Wochen) in der Waschkolonne verbleibt, ohne ausgetauscht zu werden.

Wird der stationäre Betriebszustand der Waschkolonne nur kurzzeitig gestört, so dass das Kristallbett im Prozessraum der Waschkolonne nach beendeter Störung noch intakt ist (in geschlossenem Zustand vorliegt), wird vorzugsweise wie nachfolgend beschrieben weiterverfahren.

Sofern der folgende Betriebszustand nicht bereits vorliegt, wird er in der nachfolgend angegebenen Abfolge eingestellt:

1. Förderpumpe P2 (8), die Abtrageinrichtung (16) und die Förderpumpe P3 (13) sind abgestellt. Die Zufuhr von warmer Spüllauge durch die Spülrohre (42) hindurch wird aufrechterhalten. Der regelbare Durchfluss (10) zum Auslass A (3) ist geschlossen. Der Wärmeübertrager W (9) und die Pumpe P1 (11) des Kristallschmelzekreises (31) sowie die Zudosierung von molekularem Sauerstoff und Inhibitorlösung zum Kristallschmelzekreis (31) sind in Betrieb.

2. Einschalten der Abtrageinrichtung (16).

3. Einschalten der Förderpumpe P2 (8) für die Kristallsuspension S.

4. Einschalten der Förderpumpe P3 (13) für den Steuerlaugestrom.

5. Langsames Öffnen des regelbaren Durchflusses (10) zum Auslass A (3) um eine angemessen hohe Lage der Waschfront (37) einzustellen (sicher zu stellen).

Damit umfasst vorliegende Anmeldung insbesondere die folgenden erfindungsgemäßen Ausführungsformen:

1. Ein Verfahren der Inbetriebnahme eines Trennverfahrens zur reinigenden Abtrennung von Acrylsäurekristallen aus einer Suspension S ihrer Kristalle in Mutterlauge mit einer Vorrichtung, die eine hydraulische Waschkolonne umfasst, die einen zu seiner von oben nach unten verlaufenden Längsachse rotationssymmetrischen Prozessraum aufweist, der von einer zylindrischen Mantelwand und zwei auf der Symmetrieachse einander gegenüberliegenden Enden begrenzt wird, wobei

- sich vom oberen Ende des Prozessraums parallel zu dessen Längsachse ein oder mehrere Filterrohre durch den Prozessraum erstrecken, die auf das dem oberen Ende gegenüberliegende untere Ende des Prozessraums zulaufen, und in der dem unteren Ende des Prozessraums zugewandten Hälfte des Prozessraums wenigstens ein Filter F, das die einzige direkte Verbindung zwischen dem jeweiligen Filterrohrinneren und dem Prozessraum bildet, aufweisen sowie außerhalb des Prozessraums aus der Waschkolonne herausgeführt werden,

- der Quotient Q = L/D aus dem Abstand L zwischen dem oberen und dem unteren Ende des Prozessraums und dem Durchmesser D des Prozessraums 0,3 bis 4 beträgt,

- sich am unteren Ende des Prozessraums nach unten gerichtet der Kristallschmelzeraum der Waschkolonne anschließt, wobei zwischen den beiden Räumen eine rotationsfähige Abtrageinrichtung integriert und durch den Kristallschmelzeraum ein Kristallschmelzekreis hindurchgeführt ist, der außer dem Kristallschmelzeraum

  - eine außerhalb der Waschkolonne befindliche Förderpumpe P1, die eine Saug- und eine Druckseite aufweist,

  - eine erste Förderverbindung G1, die vom Kristallschmelzeraum der Waschkolonne zur Saugseite der Förderpumpe P1 führt,

  - eine zweite Förderverbindung G2, die von der Druckseite der Förderpumpe P1 in den Kristallschmelzeraum der Waschkolonne zurückführt und einen Auslass A aus dem Kristallschmelzekreis mit regelbarem Durchfluss aufweist, sowie

- einen Wärmeübertrager W, über den entweder die Förderverbindung G1 vom Kristallschmelzeraum zur Saugseite der Förderpumpe P1, oder die Förderverbindung G2 von der Druckseite der Förderpumpe P1 zum Kristallschmelzeraum geführt ist, umfasst,

- dem oberen Ende des Prozessraums nach oben gerichtet ein Verteilerraum vorgelagert ist, der vom Prozessraum wenigstens durch einen Boden B getrennt ist, welcher Durchgänge U aufweist, die auf der dem Prozessraum zugewandten Seite des Bodens B in den Prozessraum und auf der vom Prozessraum abgewandten Seite des Bodens B in den Verteilerraum führen,

- sich außerhalb der Waschkolonne eine Förderpumpe P2, die eine Saug- und eine Druckseite aufweist, und eine Quelle QS der Suspension S befinden, wobei

    - eine erste Förderverbindung E1 von der Quelle QS zur Saugseite der Förderpumpe P2, und

    - eine zweite Förderverbindung E2 von der Druckseite der Förderpumpe P2 in den Verteilerraum führt,

- sich außerhalb der Waschkolonne gegebenenfalls eine Förderpumpe P3, die eine Saug- und eine Druckseite aufweist, und eine Quelle QT einer Steuerlauge befinden, wobei

    - eine erste Förderverbindung C1 von der Saugseite der Pumpe P3 zur Quelle QT, und

    - eine zweite Förderverbindung C2 von der Druckseite der Pumpe P3 in den Verteilerraum und/oder in den zwischen seinem oberen Ende und den Filtern F der Filterrohre gelegenen Längsabschnitt des Prozessraums führt,

und wobei man bei der Durchführung des Trennverfahrens in seinem stationären Betrieb

- mit der Pumpe P2 kontinuierlich einen Strom ST der Suspension S von der Quelle QS durch die Förderverbindungen E1, E2 über den Verteilerraum und durch die Durchgänge U hindurch in den Prozessraum der Waschkolonne führt,

- gegebenenfalls mit der Pumpe P3 einen Strom SL der Steuerlauge von der Quelle QT durch die Förderverbindungen C1, C2 über den Verteilerraum und die Durchgänge U hindurch und/oder direkt in den Prozessraum der Waschkolonne führt,

- über die Filter F der Filterrohre insgesamt einen Strom SM umfassend Mutterlauge und gegebenenfalls Steuerlauge als Ablaugestrom in das Filterrohrinnere hinein- und über die Filterrohre aus der Waschkolonne herausführt, und diesen aus der Waschkolonne herausgeführten Ablaugestrom SM als die Quelle QT für die Steuerlauge verwendet,

- durch die Mutter- und gegebenenfalls Steuerlaugeführung im Prozessraum der Waschkolonne die Ausbildung eines Kristallbetts an Acrylsäurekristallen aufrechterhält, das eine dem oberen Ende des Prozessraums zugewandte Aufbaufront aufweist, an der sich kontinuierlich Kristalle des zugeführten Stroms ST der Suspension S ans Kristallbett anlagern,

- das Kristallbett durch die aus dem hydraulischen Strömungsdruckverlust der Mutter- und gegebenenfalls Steuerlaugeführung im Prozessraum resultierende Kraft von oben nach unten an den Filtern F vorbei auf die rotierende Abtrageinrichtung zu fördert,

- mit der rotierenden Abtrageinrichtung vom auf sie stoßenden Kristallbett Acrylsäurekristalle abträgt,

- den Strom der abgetragenen Acrylsäurekristalle durch die rotierende Abtrageinrichtung hindurch und/oder an der rotierenden Abtrageinrichtung vorbei in den sich in Förderrichtung des Kristallbetts an den Prozessraum anschließenden Kristallschmelzeraum fördert, und in dem durch den Kristallschmelzeraum geführten Kristallschmelzekreis durch Eintrag von Wärme mit dem Wärmeübertrager W zu einem Kristallschmelzestrom aufschmilzt, und

- den Durchfluss des Auslasses A so regelt, dass, bezogen auf die Stärke des vorgenannten Kristallschmelzestroms, vom Kristallschmelzeraum ausgehend ein Teilstrom an Kristallschmelze als Waschschmelzestrom durch die rotierende Abtrageinrichtung hindurch und/oder an der rotierenden Abtrageinrichtung vorbei gegen die Bewegungsrichtung des Kristallbetts in den Prozessraum zurückströmt, wo er im abwärts geförderten Kristallbett aufsteigt und dabei von den Kristallen die im Kristallbett verbliebene und mit diesem unter die Filter F geförderte Mutterlauge abwäscht und zurückdrängt, wobei sich im von den Filtern F bis zum unteren Ende des Prozessraums erstreckenden Längsabschnitt des Prozessraums im Kristallbett eine Waschfront ausbildet, die das Kristallbett von oben nach unten in eine Mutterlaugezone und in eine Waschschmelzezone aufteilt, und der restliche Teilstrom des vorgenannten Kristallschmelzestroms den Kristallschmelzekreis durch den Auslass A verlässt,

dadurch gekennzeichnet, dass man bei der Inbetriebnahme des Trennverfahrens zur erstmaligen Ausbildung des Kristallbetts im Prozessraum

- den den Kristallschmelzeraum umfassenden Kristallschmelzekreis und den Prozessraum der zuvor unbefüllten Waschkolonne mit einer Acrylsäure enthaltenden Anfahrflüssigkeit AT zunächst so befüllt, dass die Füllhöhe der Anfahrflüssigkeit AT im Prozessraum wenigstens die Abtrageinrichtung überragt,

- anschließend die Befüllung der Waschkolonne fortsetzt, indem man mit der Pumpe P2 einen Strom ST* der Suspension S von der Quelle QS durch die Förderverbindungen E1, E2 über den Verteilerraum und durch die Durchgänge U hindurch in den Prozessraum der Waschkolonne und von dem dabei durch die Filterrohre aus der Waschkolonne herausgeführten Ablaugestrom SM* als Quelle QT* gegebenenfalls mit der Pumpe P3 einen Teilstrom als Steuerlaugestrom SL* durch die Förderverbindungen C1, C2 über den Verteilerraum und die Durchgänge U hindurch und/oder direkt in den Prozessraum der Waschkolonne führt, und dies wenigstens so lange, bis der Zeitpunkt $t_s$ erreicht ist, bei dem der Differenzdruck $P_D = P_k - P_v$, wobei $P_k$ der an einer beliebig ausgewählten Stelle im Kristallschmelzeraum zu einem bestimmten Zeitpunkt der Zufuhr des Stroms ST* jeweils bestehende Druck und $P_v$ der zum jeweils gleichen Zeitpunkt an einer beliebig ausgewählten Stelle im Verteilerraum jeweils bestehende Druck ist, in Abhängigkeit von der Dauer der Zufuhr des Stroms ST* nicht mehr ansteigt oder konstant bleibt, sondern plötzlich abnimmt, und dies mit der Maßgabe, dass

- bis zum Zeitpunkt $t_s$ die mittlere Flächenbelastung der Filter F, berechnet als der arithmetische Mittelwert des während der Zufuhr des Stroms ST* durch die Filter F der Filterrohre zum jeweiligen Zeitpunkt insgesamt strömenden Ablaugestroms SM*, geteilt durch die Gesamtfläche aller Filter F, nicht mehr als 80m³/(m² ·h) beträgt,

- die Acrylsäure enthaltende Anfahrflüssigkeit AT eine solche ist, aus der beim Abkühlen bis zur einsetzenden Kristallisation die sich bei der Kristallisation bildenden Kristalle Acrylsäurekristalle sind, und

- zwischen der in Grad Celsius angegebenen Kristallbildungstemperatur $T^{KB}$ dieser Acrylsäurekristalle in der Anfahrflüssigkeit AT und der in Grad Celsius angegebenen Temperatur $T^S$ der Suspension S des Stroms ST* die Beziehung

$$T^{KB} \leq T^S + 15°C$$

erfüllt ist.

2. Ein Verfahren gemäß Ausführungsform 1, dadurch gekennzeichnet, dass bis zum Zeitpunkt $t_S$ die mittlere Flächenbelastung der Filter F nicht mehr als 75 m³/(m² · h) beträgt.

3. Ein Verfahren gemäß Ausführungsform 1, dadurch gekennzeichnet, dass bis zum Zeitpunkt ts die mittlere Flächenbelastung der Filter F nicht mehr als 70 m³/(m² . h) beträgt.

4. Ein Verfahren gemäß einer der Ausführungsformen 1 bis 3, dadurch gekennzeichnet, dass bis zum Zeitpunkt ts die mittlere Flächenbelastung der Filter F wenigstens 5 m³/(m² · h) beträgt.

5. Ein Verfahren gemäß einer der Ausführungsformen 1 bis 3, dadurch gekennzeichnet, dass bis zum Zeitpunkt $t_s$ die mittlere Flächenbelastung der Filter F wenigstens 10 m³/(m² · h) beträgt.

6. Ein Verfahren gemäß einer der Ausführungsformen 1 bis 3, dadurch gekennzeichnet, dass bis zum Zeitpunkt $t_s$ die mittlere Flächenbelastung der Filter F wenigstens 15 m³/(m² · h) beträgt.

7. Ein Verfahren gemäß einer der Ausführungsformen 1 bis 3, dadurch gekennzeichnet, dass bis zum Zeitpunkt $t_s$ die mittlere Flächenbelastung der Filter F wenigstens 20 m³/(m² · h) beträgt.

8. Ein Verfahren gemäß einer der Ausführungsformen 1 bis 7, dadurch gekennzeichnet, dass bis zum Zeitpunkt ts die mittlere Flächenbelastung der Filter F nicht mehr als 60 m³/(m² · h) beträgt.

9. Ein Verfahren gemäß einer der Ausführungsformen 1 bis 7, dadurch gekennzeichnet, dass bis zum Zeitpunkt ts die mittlere Flächenbelastung der Filter F nicht mehr als 50 m³/(m² · h) beträgt.

10. Ein Verfahren gemäß einer der Ausführungsformen 1 bis 9, dadurch gekennzeichnet, dass der Quotient Q ≥ 0,5 beträgt.

11. Ein Verfahren gemäß einer der Ausführungsformen 1 bis 9, dadurch gekennzeichnet, dass der Quotient Q ≥ 0,7 beträgt.

12. Ein Verfahren gemäß einer der Ausführungsformen 1 bis 11, dadurch gekennzeichnet, dass der Quotient Q ≤ 3,5 beträgt.

13. Ein Verfahren gemäß einer der Ausführungsformen 1 bis 11, dadurch gekennzeichnet, dass der Quotient Q ≤ 3 beträgt.

14. Ein Verfahren gemäß einer der Ausführungsformen 1 bis 11, dadurch gekennzeichnet, dass der Quotient Q ≤ 2,5 beträgt.

15. Ein Verfahren gemäß einer der Ausführungsformen 1 bis 11, dadurch gekennzeichnet, dass der Quotient Q ≤ 2 beträgt.

16. Ein Verfahren gemäß einer der Ausführungsformen 1 bis 15, dadurch gekennzeichnet, dass der Abstand L ≥ 0,5 m beträgt.

17. Ein Verfahren gemäß einer der Ausführungsformen 1 bis 15, dadurch gekennzeichnet, dass der Abstand L ≥ 0,8 m beträgt.

18. Ein Verfahren gemäß einer der Ausführungsformen 1 bis 15, dadurch gekennzeichnet, dass der Abstand L ≥ 1 m beträgt.

19. Ein Verfahren gemäß einer der Ausführungsformen 1 bis 8, dadurch gekennzeichnet, dass der Abstand L ≤ 5 m beträgt.

20. Ein Verfahren gemäß einer der Ausführungsformen 1 bis 8, dadurch gekennzeichnet, dass der Abstand L ≤ 4 m beträgt.

21. Ein Verfahren gemäß einer der Ausführungsformen 1 bis 8, dadurch gekennzeichnet, dass der Abstand L ≤ 3 m beträgt.

22. Ein Verfahren gemäß einer der Ausführungsformen 1 bis 21, dadurch gekennzeichnet, dass die Beziehung $T^{KB} \leq T^S + 10°C$ erfüllt ist.

23. Ein Verfahren gemäß einer der Ausführungsformen 1 bis 21, dadurch gekennzeichnet, dass die Beziehung $T^{KB} \leq T^S + 5°C$ erfüllt ist.

24. Ein Verfahren gemäß einer der Ausführungsformen 1 bis 23, dadurch gekennzeichnet, dass $T^{KB}$ nicht mehr als 20°C unterhalb von $T^S$ liegt.

25. Ein Verfahren gemäß einer der Ausführungsformen 1 bis 23, dadurch gekennzeichnet, dass $T^{KB}$ nicht mehr als 10°C unterhalb von $T^S$ liegt.

26. Ein Verfahren gemäß einer der Ausführungsformen 1 bis 23, dadurch gekennzeichnet, dass $T^{KB}$ nicht mehr als 5°C unterhalb von $T^S$ liegt.

27. Ein Verfahren gemäß einer der Ausführungsformen 1 bis 26, dadurch gekennzeichnet, dass die Anfahrflüssigkeit AT von der Suspension S abgetrennte Mutterlauge ist.

28. Ein Verfahren gemäß einer der Ausführungsformen 1 bis 26, dadurch gekennzeichnet, dass die Anfahrflüssigkeit AT die Schmelze von aus der Suspension S abgetrennten Kristallen ist.

29. Ein Verfahren gemäß einer der Ausführungsformen 1 bis 26, dadurch gekennzeichnet, dass die Anfahrflüssigkeit AT aufgeschmolzene Suspension S ist.

30. Ein Verfahren gemäß einer der Ausführungsformen 1 bis 26, dadurch gekennzeichnet, dass die Anfahrflüssigkeit AT diejenige Flüssigkeit ist, aus der durch Abkühlen die Suspension S erzeugt wird.

31. Ein Verfahren gemäß einer der Ausführungsformen 1 bis 26, dadurch gekennzeichnet, dass die Anfahrflüssigkeit AT eine Mischung aus wenigstens zwei der in den Ausführungsformen 27 bis 30 genannten Anfahrflüssigkeiten AT ist.

32. Ein Verfahren gemäß einer der Ausführungsformen 1 bis 31, dadurch gekennzeichnet, dass wenigstens während 50% des Zeitraums, gerechnet vom Beginn der Zufuhr des Stroms ST* der Suspension S bis zum Erreichen des Zeitpunkts ts, die zum jeweiligen Zeitpunkt bestehende Flächenbelastung der Filter F nicht mehr als 80 m³/(m²·h) beträgt.

33. Ein Verfahren gemäß einer der Ausführungsformen 1 bis 31, dadurch gekennzeichnet, dass wenigstens während 50% des Zeitraums, gerechnet vom Beginn der Zufuhr des Stroms ST* der Suspension S bis zum Erreichen des Zeitpunkts ts, die zum jeweiligen Zeitpunkt bestehende Flächenbelastung der Filter F nicht mehr als 70 m³/(m²·h) beträgt.

34. Ein Verfahren gemäß einer der Ausführungsformen 1 bis 31, dadurch gekennzeichnet, dass wenigstens während 50% des Zeitraums, gerechnet vom Beginn der Zufuhr des Stroms ST* der Suspension S bis zum Erreichen des Zeitpunkts ts, die zum jeweiligen Zeitpunkt bestehende Flächenbelastung der Filter F nicht mehr als 60 m³/(m²·h) beträgt.

35. Ein Verfahren gemäß einer der Ausführungsformen 1 bis 31, dadurch gekennzeichnet, dass wenigstens während 75% des Zeitraums, gerechnet vom Beginn der Zufuhr des Stroms ST* der Suspension S bis zum Erreichen des Zeitpunkts $t_S$, die zum jeweiligen Zeitpunkt bestehende Flächenbelastung der Filter F nicht mehr als 80 m³/(m²·h) beträgt.

36. Ein Verfahren gemäß einer der Ausführungsformen 1 bis 31, dadurch gekennzeichnet, dass wenigstens während 75% des Zeitraums, gerechnet vom Beginn der Zufuhr des Stroms ST* der Suspension S bis zum Erreichen des Zeitpunkts ts, die zum jeweiligen Zeitpunkt bestehende Flächenbelastung der Filter F nicht mehr als 70 m³/(m²·h) beträgt.

37. Ein Verfahren gemäß einer der Ausführungsformen 1 bis 31, dadurch gekennzeichnet, dass wenigstens während 75% des Zeitraums, gerechnet vom Beginn der Zufuhr des Stroms ST* der Suspension S bis zum Erreichen des Zeitpunkts ts, die zum jeweiligen Zeitpunkt bestehende Flächenbelastung der Filter F nicht mehr als 60 m³/(m²·h) beträgt.

38. Ein Verfahren gemäß einer der Ausführungsformen 1 bis 31, dadurch gekennzeichnet, dass während des gesamten Zeitraums, gerechnet vom Beginn der Zufuhr des Stroms ST* der Suspension S bis zum Erreichen des Zeitpunkts ts, die zum jeweiligen Zeitpunkt bestehende Flächenbelastung der Filter F nicht mehr als 80 m³/(m²·h) beträgt.

39. Ein Verfahren gemäß einer der Ausführungsformen 1 bis 31, dadurch gekennzeichnet, dass während des gesamten Zeitraums, gerechnet vom Beginn der Zufuhr des Stroms ST* der Suspension S bis zum Erreichen des Zeitpunkts ts die zum jeweiligen Zeitpunkt bestehende Flächenbelastung der Filter F nicht mehr als 70 m$^3$/(m$^2$·h) beträgt.

40. Ein Verfahren gemäß einer der Ausführungsformen 1 bis 31, dadurch gekennzeichnet, dass während des gesamten Zeitraums, gerechnet vom Beginn der Zufuhr des Stroms ST* der Suspension S bis zum Erreichen des Zeitpunkts ts, die zum jeweiligen Zeitpunkt bestehende Flächenbelastung der Filter F nicht mehr als 60 m$^3$/(m$^2$·h) beträgt.

41. Ein Verfahren gemäß einer der Ausführungsformen 1 bis 40, dadurch gekennzeichnet, dass wenigstens während 50% des Zeitraums, gerechnet vom Beginn der Zufuhr des Stroms ST* der Suspension S bis zum Erreichen des Zeitpunkts $t_S$, die zum jeweiligen Zeitpunkt bestehende Flächenbelastung der Filter F wenigstens 5 m$^3$/(m$^2$·h) oder wenigstens 10 m$^3$/(m$^2$·h) beträgt.

42. Ein Verfahren gemäß einer der Ausführungsformen 1 bis 40, dadurch gekennzeichnet, dass wenigstens während 75% des Zeitraums, gerechnet vom Beginn der Zufuhr des Stroms ST* der Suspension S bis zum Erreichen des Zeitpunkts $t_S$, die zum jeweiligen Zeitpunkt bestehende Flächenbelastung der Filter F wenigstens 5 m$^3$/(m$^2$·h) oder wenigstens 10 m$^3$/(m$^2$·h) beträgt.

43. Ein Verfahren gemäß einer der Ausführungsformen 1 bis 40, dadurch gekennzeichnet, dass während des gesamten Zeitraums, gerechnet vom Beginn der Zufuhr des Stroms ST* der Suspension S bis zum Erreichen des Zeitpunkts $t_S$, die zum jeweiligen Zeitpunkt bestehende Flächenbelastung der Filter F wenigstens 5 m$^3$/(m$^2$·h) oder wenigstens 10 m$^3$/(m$^2$·h) beträgt.

44. Ein Verfahren gemäß einer der Ausführungsformen 1 bis 43, dadurch gekennzeichnet, dass während des gesamten Zeitraums, gerechnet vom Beginn der Zufuhr des Stroms ST* der Suspension S bis zum Erreichen des Zeitpunkts ts, der arithmetische Mittelwert M des dem Prozessraum der Waschkolonne insgesamt zugeführten Stroms an Flüssigkeit, geteilt durch die freie Querschnittsfläche des Prozessraums 1 bis 30 m$^3$/(m$^2$·h) beträgt.

45. Ein Verfahren gemäß Ausführungsform 44, dadurch gekennzeichnet, dass der arithmetische Mittelwert M geteilt durch die freie Querschnittsfläche des Prozessraums 5 bis 25 m$^3$/(m$^2$·h) beträgt.

46. Ein Verfahren gemäß einer der Ausführungsformen 1 bis 45, dadurch gekennzeichnet, dass wenigstens während 50% des Zeitraums, gerechnet vom Beginn der Zufuhr des Stroms ST* der Suspension S bis zum Erreichen des Zeitpunkts ts, der dem Prozessraum der Waschkolonne zum jeweiligen Zeitpunkt insgesamt zugeführte Strom an Flüssigkeit, geteilt durch die freie Querschnittsfläche des Prozessraums 1 bis 30 m$^3$/(m$^2$·h) oder 5 bis 25 m$^3$/(m$^2$·h) beträgt.

47. Ein Verfahren gemäß einer der Ausführungsformen 1 bis 45, dadurch gekennzeichnet, dass wenigstens während 75% des Zeitraums, gerechnet vom Beginn der Zufuhr des Stroms ST* der Suspension S bis zum Erreichen des Zeitpunkts $t_S$, der dem Prozessraum der Waschkolonne zum jeweiligen Zeitpunkt insgesamt zugeführte Strom an Flüssigkeit, geteilt durch die freie Querschnittsfläche des Prozessraums 1 bis 30 m$^3$/(m$^2$·h) oder 5 bis 25 m$^3$/(m$^2$·h) beträgt.

48. Ein Verfahren gemäß einer der Ausführungsformen 1 bis 45, dadurch gekennzeichnet, dass während des gesamten Zeitraums, gerechnet vom Beginn der Zufuhr des Stroms ST* der Suspension S bis zum Erreichen des Zeitpunkts ts, der dem Prozessraum der Waschkolonne zum jeweiligen Zeitpunkt insgesamt zugeführte Strom an Flüssigkeit, geteilt durch die freie Querschnittsfläche des Prozessraums 1 bis 30 m$^3$/(m$^2$·h) oder 5 bis 25 m$^3$/(m$^2$·h) beträgt.

49. Ein Verfahren gemäß einer der Ausführungsformen 1 bis 48, dadurch gekennzeichnet, dass der Gehalt der Suspension S an Acrylsäure ≥ 70 Gew.-% beträgt.

50. Ein Verfahren gemäß einer der Ausführungsformen 1 bis 48, dadurch gekennzeichnet, dass der Gehalt der Suspension S an Acrylsäure ≥ 80 Gew.-% beträgt.

51. Ein Verfahren gemäß einer der Ausführungsformen 1 bis 48, dadurch gekennzeichnet, dass der Gehalt der Suspension S an Acrylsäure ≥ 90 Gew.-% beträgt.

52. Ein Verfahren gemäß einer der Ausführungsformen 1 bis 51, dadurch gekennzeichnet, dass der Gehalt der Suspension S an Acrylsäure ≤ 99 Gew.-% beträgt.

53. Ein Verfahren gemäß einer der Ausführungsformen 1 bis 52, dadurch gekennzeichnet, dass der Kristallisationsgrad der Suspension S ≥ 0,10 beträgt.

54. Ein Verfahren gemäß einer der Ausführungsformen 1 bis 52, dadurch gekennzeichnet, dass der Kristallisationsgrad der Suspension S ≥ 0,20 beträgt.

55. Ein Verfahren gemäß einer der Ausführungsformen 1 bis 52, dadurch gekennzeichnet, dass der Kristallisationsgrad der Suspension S ≥ 0,25 beträgt.

56. Ein Verfahren gemäß einer der Ausführungsformen 1 bis 55, dadurch gekennzeichnet, dass der Kristallisationsgrad der Suspension S ≤ 0,60 beträgt.

57. Ein Verfahren gemäß einer der Ausführungsformen 1 bis 55, dadurch gekennzeichnet, dass der Kristallisationsgrad der Suspension S ≤ 0,50 beträgt.

58. Ein Verfahren gemäß einer der Ausführungsformen 1 bis 57, dadurch gekennzeichnet, dass die Längstausdehnung der in der Suspension S enthaltenen Acrylsäurekristalle mehrheitlich 50 bis 1600

μm beträgt.

59. Ein Verfahren gemäß einer der Ausführungsformen 1 bis 57, dadurch gekennzeichnet, dass die Längstausdehnung der in der Suspension S enthaltenen Acrylsäurekristalle mehrheitlich 200 bis 900 μm beträgt.

60. Ein Verfahren gemäß einer der Ausführungsformen 1 bis 59, dadurch gekennzeichnet, dass das Öffnungsverhältnis OV der Abtrageinrichtung ≥ 0,01 beträgt.

61. Ein Verfahren gemäß einer der Ausführungsformen 1 bis 59, dadurch gekennzeichnet, dass das Öffnungsverhältnis OV der Abtrageinrichtung ≥ 0,03 beträgt.

62. Ein Verfahren gemäß einer der Ausführungsformen 1 bis 59, dadurch gekennzeichnet, dass das Öffnungsverhältnis OV der Abtrageinrichtung ≤ 0,9 beträgt.

63. Ein Verfahren gemäß einer der Ausführungsformen 1 bis 62, dadurch gekennzeichnet, dass die Abtrageinrichtung eine Durchgangsöffnungen aufweisende Messerscheibe ist.

64. Ein Verfahren gemäß einer der Ausführungsformen 1 bis 63, dadurch gekennzeichnet, dass, bezogen auf das Gesamtvolumen des Kristallschmelzkreises, 30 bis 60 Vol.-% auf das Volumen des Kristallschmelzeraums entfallen.

65. Ein Verfahren gemäß einer der Ausführungsformen 1 bis 64, dadurch gekennzeichnet, dass man den Kristallschmelzekreis und den Prozessraum der zuvor unbefüllten Waschkolonne mit einer Acrylsäure enthaltenden Anfahrflüssigkeit AT zunächst so befüllt, dass die Füllhöhe der Anfahrflüssigkeit AT im Prozessraum wenigstens die Filter F überragt.

66. Ein Verfahren gemäß Ausführungsform 65, dadurch gekennzeichnet, dass die Füllhöhe der Anfahrflüssigkeit AT wenigstens bis zur Mitte des Abstands L vom unteren bis zum oberen Ende des Prozessraums ragt.

67. Ein Verfahren gemäß Ausführungsform 65, dadurch gekennzeichnet, dass die Füllhöhe der Anfahrflüssigkeit AT wenigstens bis zum letzten Viertel des Abstands L vom unteren bis zum oberen Ende des Prozessraums ragt.

68. Ein Verfahren gemäß Ausführungsform 65, dadurch gekennzeichnet, dass die Füllhöhe der Anfahrflüssigkeit AT wenigstens bis zum oberen Ende des Prozessraums ragt.

69. Ein Verfahren gemäß Ausführungsform 65, dadurch gekennzeichnet, dass die Füllhöhe der Anfahrflüssigkeit AT über den Prozessraum hinaus bis in den Verteilerraum hineinragt und das Volumen desselben bis wenigstens zur Hälfte ausfüllt.

70. Ein Verfahren gemäß Ausführungsform 65, dadurch gekennzeichnet, dass die Füllhöhe der Anfahrflüssigkeit AT über den Prozessraum hinaus bis in den Verteilerraum hineinragt und das Volumen desselben vollständig ausfüllt.

71. Ein Verfahren gemäß einer der Ausführungsformen 1 bis 70, dadurch gekennzeichnet, dass der Wärmeübertrager W ein Rohrbündelwärmeübertrager ist.

72. Ein Verfahren gemäß einer der Ausführungsformen 1 bis 71, dadurch gekennzeichnet, dass die Temperatur der Suspension S -25 bis +14°C beträgt.

73. Ein Verfahren gemäß einer der Ausführungsformen 1 bis 71, dadurch gekennzeichnet, dass die Temperatur der Suspension S -5 bis +12°C beträgt.

74. Ein Verfahren gemäß einer der Ausführungsformen 1 bis 71, dadurch gekennzeichnet, dass die Temperatur der Suspension S +4 bis +9°C beträgt.

75. Ein Verfahren gemäß einer der Ausführungsformen 1 bis 74, dadurch gekennzeichnet, dass die in der Suspension S enthaltene Mutterlauge ≥ 70 Gew.-%, oder ≥ 80 Gew.-% an Acrylsäure enthält.

76. Ein Verfahren gemäß einer der Ausführungsformen 1 bis 75, dadurch gekennzeichnet, dass die in der Suspension S enthaltene Mutterlauge ≤ 99 Gew.-% an Acrylsäure enthält.

77. Ein Verfahren gemäß einer der Ausführungsformen 1 bis 76, dadurch gekennzeichnet, dass von dem durch die Filterrohre aus der Waschkolonne herausgeführten Ablaugestrom SM* als Quelle QT* mit der Pumpe P3 ein Teilstrom als Steuerlaugestrom SL* durch die Förderverbindungen C1, C2 über den Verteilerraum und durch die Durchgänge U hindurch in den Prozessraum der Waschkolonne geführt wird.

78. Ein Verfahren gemäß einer der Ausführungsformen 1 bis 77, dadurch gekennzeichnet, dass die Förderverbindungen E2 und C2 vor dem Zuführraum der hydraulischen Waschkolonne als koaxiale Rohrleitungen ausgeführt sind, wobei die innen geführte Rohrleitung in Strömungsrichtung vor dem Zuführraum ausläuft, und nur die äußere Rohrleitung als gemeinsame Förderverbindung E2, C2 bis zum Verteilerraum weitergeführt wird.

79. Ein Verfahren gemäß Ausführungsform 78, dadurch gekennzeichnet, dass sich der Querschnitt der innen geführten Rohrleitung zu ihrem Auslauf hin verjüngt.

80. Ein Verfahren gemäß Ausführungsform 78 oder 79, dadurch gekennzeichnet, dass in der innen geführten Rohrleitung die Suspension S strömt.

81. Ein Verfahren gemäß einer der Ausführungsformen 1 bis 80, dadurch gekennzeichnet, dass während der Inbetriebnahme sowohl der Druck $P_K$ als auch der Druck Pv gemessen wird.

82. Ein Verfahren gemäß einer der Ausführungsformen 1 bis 81, dadurch gekennzeichnet, dass der Differenzdruck $P_D$ mit einem Differenzdruckmanometer bestimmt wird.

83. Ein Verfahren gemäß einer der Ausführungsformen 1 bis 82, dadurch gekennzeichnet, dass ab Beginn der Zufuhr des Stroms ST* der Suspension S bis zum Erreichen des Zeitpunktes $t_S$ der Wärmeaustauscher W außer Betrieb ist.

84. Ein Verfahren gemäß einer der Ausführungsformen 1 bis 83, dadurch gekennzeichnet, dass ab Beginn der Zufuhr des Stroms ST* der Suspension S bis zum Erreichen des Zeitpunktes $t_S$ die Förderpumpe P1 außer Betrieb ist.

85. Ein Verfahren gemäß einer der Ausführungsformen 1 bis 84, dadurch gekennzeichnet, dass ab Beginn der Zufuhr des Stroms ST* der Suspension S bis zum Erreichen des Zeitpunktes $t_S$ die rotationsfähige Abtrageinrichtung nicht in Betrieb genommen ist.

86. Ein Verfahren gemäß einer der Ausführungsformen 1 bis 85, dadurch gekennzeichnet, dass ab Beginn der Zufuhr des Stroms ST* der Suspension S bis zum Erreichen des Zeitpunktes $t_S$ der Durchfluss des Auslasses A gesperrt ist.

87. Ein Verfahren gemäß einer der Ausführungsformen 1 bis 86, dadurch gekennzeichnet, dass nach dem Zeitpunkt ts der Schmelzkreis und die Abtrageinrichtung in Betrieb genommen und der Durchfluss des Auslasses A geöffnet wird, sowie dem Schmelzkreis ein molekularen Sauerstoff enthaltendes Gas zudosiert wird, in dem in einen Teilstrom des Schmelzkreises das molekularen Sauerstoff enthaltende Gas eingebracht, und anschließend der den molekularen Sauerstoff enthaltende Teilstrom wieder dem Schmelzkreis zugeführt wird.

88. Ein Verfahren gemäß Ausführungsform 87, dadurch gekennzeichnet, dass bevor der den molekularen Sauerstoff enthaltende Teilstrom wieder dem Schmelzkreis zugeführt wird, im Teilstrom nicht gelöstes Gas in einem Gasabscheider abgetrennt wird.

89. Ein Verfahren gemäß einer der Ausführungsformen 1 bis 88, dadurch gekennzeichnet, dass der Prozessraum der hydraulischen Waschkolonne einen zentralen Verdrängerkörper aufweist.

90. Ein Verfahren gemäß einer der Ausführungsformen 1 bis 89, dadurch gekennzeichnet, dass die Höhe der Durchgänge U 200 bis 1000 mm beträgt.

91. Ein Verfahren gemäß einer der Ausführungsformen 1 bis 90, dadurch gekennzeichnet, dass die Öffnungen der Durchgänge U, die in den Prozessraum oder in den Verteilerraum führen, eine Querschnittsfläche aufweisen, die, bezogen auf eine kreisrunde Form der Öffnung, einem Durchmesser von 15 bis 300 mm entsprechen.

92. Ein Verfahren gemäß einer der Ausführungsformen 1 bis 91, dadurch gekennzeichnet, dass das Verhältnis der Gesamtfläche aller dem Prozessraum zugewandten Öffnungen der Durchgänge U zur Gesamtfläche des Prozessraumquerschnitts 0,10 bis 0,60 beträgt.

93. Ein Verfahren gemäß einer der Ausführungsformen 1 bis 92, dadurch gekennzeichnet, dass die Anzahl der Filterrohre in der hydraulischen Waschkolonne 3 bis 200 beträgt.

94. Ein Verfahren gemäß einer der Ausführungsformen 1 bis 93, dadurch gekennzeichnet, dass der Innendurchmesser der Filterrohre 5 bis 200 mm beträgt.

95. Ein Verfahren gemäß einer der Ausführungsformen 1 bis 93, dadurch gekennzeichnet, dass der Innendurchmesser der Filterrohre 20 bis 80 mm beträgt.

96. Ein Verfahren gemäß einer der Ausführungsformen 1 bis 95, dadurch gekennzeichnet, dass die Suspension S nachfolgende Gehalte aufweist.

≥ 70 Gew.-% Acrylsäure,
bis zu 15 Gew.-% Essigsäure,
bis zu 5 Gew.-% Propionsäure,
bis zu 5 Gew.-% niedermolekulare Aldehyde,
bis zu 3 Gew.-% Diacrylsäure, und
bis zu 25 Gew.-% Wasser.

97. Ein Verfahren gemäß einer der Ausführungsformen 1 bis 96, dadurch gekennzeichnet, dass die Suspension S wenigstens 0,1 Gew.-% Wasser enthält.

98. Ein Verfahren gemäß einer der Ausführungsformen 1 bis 97, dadurch gekennzeichnet, dass sich an das Verfahren der Inbetriebnahme ein Trennverfahren der reinigenden Abtrennung von Acrylsäurekristallen aus der Suspension S ihrer Kristalle in Mutterlauge in der in Betrieb genommen hydraulischen Waschkolonne anschließt.

99. Ein Trennverfahren zur reinigenden Abtrennung von Acrylsäurekristallen aus einer Suspension S ihrer Kristalle in Mutterlauge mit einer Vorrichtung, die eine hydraulische Waschkolonne umfasst, dadurch gekennzeichnet, dass das Trennverfahren gemäß einem Verfahren der Ausführungsformen 1 bis 97 in Betrieb genommen worden ist.

100. Ein Verfahren gemäß Ausführungsform 98 oder 99, dadurch gekennzeichnet, dass sich ein weiteres Verfahren anschließt, bei dem abgetrenntes und aufgeschmolzenes Acrylsäurekristallisat einer Polymerisation mit sich oder anderen wenigstens einfach ethylenisch ungesättigten Verbindungen unterworfen wird.

Beispiele und Vergleichsbeispiele

Vergleichsbeispiel 1 (Verfahren der Inbetriebnahme einer hydraulischen Waschkolonne mit L/D = 4,7 bei einer mittleren Filterflächenbelastung von 119 $m^3/(m^2 \cdot h)$

Durch wie in der WO 08/090190 beschriebene durchgeführte fraktionierende Kondensation des Produktgasgemischs einer zweistufigen heterogen katalysierten Gasphasenpartialoxidation von Propylen der Reinheit "chemical grade" wurden über den Seitenabzug der Kondensationskolonne pro Stunde 1,5 t einer rohen Acrylsäure abgetrennt, die nachfolgende Gehalte aufwies:

96,1 Gew.-% Acrylsäure,
446 Gew.ppm Acrolein,
20 Gew.ppm Allylacrylat,
3764 Gew.ppm Diacrylsäure,
7460 Gew.ppm Essigsäure,
6719 Gew.ppm Furfural,
7131 Gew.ppm Benzaldehyd,
751 Gew.ppm Propionsäure,
91 Gew.ppm Phenothiazin,
247 Gew.ppm MEHQ, und
0,83 Gew.ppm Wasser.

Durch kontinuierliche Zugabe von 31 kg/h an Wasser zu der rohen Acrylsäure wurde deren Wassergehalt auf 2,8 Gew.-% erhöht. Diese "wässrige" rohe Acrylsäure wurde anschließend mit einer Temperatur von 20°C einem Suspensionskristallisator zugeführt. Als Suspensionskristallisator wurde ein Kühlscheibenkristallisator der Firma GMF (Niederlande) mit einem Fassungsvermögen von 2500 l verwendet. Der Kristallisator enthielt eine äquidistant beabstandete Anordnung von 7 gewischten Kühlscheiben, die einen einheitlichen Durchmesser von 1,25 m aufwiesen.

Durch die Kühlscheiben wurde als Kälteträger ein Strom eines Gemischs aus 70 Vol.-% Wasser und 30 Vol.-% Glykol geführt, wobei die Zuführtemperatur 0,5 bis 1 °C betrug. Die wässrige rohe Acrylsäure und der Kälteträger wurden über den Kristallisator betrachtet im Gegenstrom durch selbigen geführt. Die aus dem Suspensionskristallisator herausgeführte Suspension von Acrylsäurekristallen in Mutterlauge wies einen Kristallisationsgrad von 0,24 und eine Temperatur von 6,9 bis 7,0°C auf.

Die so erzeugte Kristallisatsuspension strömte über ein Überlaufwehr aus dem Suspensionskristallisator heraus in einen gerührten beheizbaren Auffangbehälter. In selbigem wurden die Kristalle der Suspension wieder aufgeschmolzen und die dabei resultierende "wässrige" rohe Acrylsäure wieder in die fraktionierende Kondensation zurückgeführt (oberhalb der Seitenabzugentnahme).

Auf diese Weise war ein Strom einer Acrylsäurekristallsuspension verfügbar, der zur Inbetriebnahme der nachfolgend beschriebenen hydraulischen Waschkolonne verwendet wurde. Ferner stand im Auffangbehälter wieder aufgeschmolzene Kristallsuspension als Anfahrflüssigkeit AT zur Verfügung (sie wies eine Temperatur von 17°C auf).

Die hydraulische Waschkolonne wies im Wesentlichen das Design gemäß Figur 1 dieser Schrift auf. Der Innendurchmesser D des kreiszylindrischen Prozessraums (B) betrug 263 mm. Die Stärke der Mantelwand war 5 mm. Fertigungsmaterial war Edelstahl (DIN Werkstoff 1.4571). Die Länge L des Prozessraums (B) betrug 1230 mm (gemessen von der Oberkante der als Abtrageinrichtung (16) eingesetzten Messerscheibe). Der Prozessraum (B) wies lediglich ein Filterrohr (6) auf, das aus demselben Edelstahl gefertigt war und im Zentrum des Prozessraumquerschnitts von oben nach unten verlief. Die Wanddicke des kreiszylindrischen Filterrohres (6) war 2 mm. Sein Außendurchmesser betrug 48 mm. Die Gesamtlänge des Filterrohres (6) (einschließlich Verdränger (38)) betrug 1225 mm. Die aktive Filterlänge (Höhe) betrug 60 mm. Die Oberkante des Filters F (7) war auf einer Filterrohrlänge von 965 mm (von oben nach unten gemessen). Dem Prozessraum (B) war ein Verteilerraum (A) vorgelagert, dessen Höhe 250 mm betrug. Prozessraum (B) und Verteilerraum (A) waren durch einen Boden B (32) der Stärke 250 mm voneinander getrennt (im Bodeninneren befand sich der Ablaugesammelraum (27)). Über den Boden B (32) gleichmäßig verteilt waren 3 Durchgänge U (26), die mit kreisrunden Öffnungen des Durchmessers 26 mm und über die Durchgangsstrecke konstantem Querschnitt die beiden Räume verbanden.

Zunächst wurde die hydraulische Waschkolonne (0) vollständig mit der 17°C warmen Anfahrflüssigkeit AT aus dem Auffangbehälter befüllt (Schmelzkreis (31) + Prozessraum (B) + Verteilerraum (A) + die Förderverbindungen E2 (34), C2 (36), C1 (35) + die Förderpumpe P3 (13)). Die Befüllung erfolgte durch ein T-Stück am Kristallschmelzekreis hindurch.

Anschließend wurde die Förderpumpe P3 (13) in Betrieb genommen und ihre Drehzahl so eingestellt, dass sie einen Strom von 400 kg/h der Anfahrflüssigkeit AT über die Förderverbindung C1 (35) ansaugte und im Kreis "Saugseite der Förderpumpe P3 - Druckseite der Förderpumpe P3 - Förderverbindung C2 (36) - Verteilerraum (A) - Prozessraum (B) - Filter F (7) - Filterrohr (16) - Förderverbindung C1 (35)" förderte (Auslass A (3) war gesperrt).

Dann wurde (bei weiterhin gesperrtem Auslass A (3) sowie nicht in Betrieb befindlicher Abtrageinrichtung (16) (Messerscheibe) und nicht in Betrieb befindlichem Schmelzkreis (31)) die Förderpumpe P2 (8) in Betrieb genommen und mit dieser dem Suspensionskristallisator über einen Entnahmestutzen 1000 kg/h der Suspension S von Acrylsäurekristallen in Mutterlauge entnommen und über die Förderverbindung E2 (34) zusätzlich zu dem oben genannten Strom von 400 kg/h in den Verteilerraum (A) der hydraulischen Waschkolonne (0) gepumpt. Bei so in Betrieb befindlichen Förderpumpen P2 (8) und P3 (13) erfolgte die Ausbildung des Kristallbetts im Prozessraum (B) der hydraulischen Waschkolonne (0) (760 kg/h Ablauge strömten aus dem Auslass (2) der Waschkolonnenvorrichtung). Damit einher ging ein zunächst paralleler Anstieg der mit den Membranmanometern M1 (Druck im Verteilerraum (A)) und M2 (Druck im Kristallschmelzeraum (C)) gemessenen Drucke.

Der durch das Filter F (7) strömende Ablaugestrom betrug dabei 1080 l/h, was einer (mittleren) Filterflächenbelastung von 119 $m^3/(m^2 \cdot h)$ entspricht. Nach $t_S=$ 14 min (gerechnet ab Beginn der Inbetriebnahme der Förderpumpe P2 (8)) begann der mit dem Membranmanometer M2 erfasste Druck $P_K$ plötzlich zu fallen, während der mit dem Membranmanometer M1 erfasste Druck Pv weiter anstieg, was einem erstmals fallenden Differenzdruck $P_D=P_K-P_V$ entsprach.

Unmittelbar nach erfolgtem Drucksprung wurden die Förderpumpen P2 (8) und P3 (13) (in dieser Reihenfolge) ausgeschaltet und die Förderpumpe P1 (11) sowie die Rotation der Messerscheibe (16) (in dieser Reihenfolge) in Betrieb genommen. Anschließend wurden die Förderpumpen P2 (8) (mit einer Förderleistung von 1000 kg/h) und die Förderpumpe P3 (13) (mit einer Förderleistung von 800 kg/h) wieder in Betrieb genommen, wobei sich das Kristallbett samt Aufbaufront nach unten in Bewegung setzte.

Dann wurde der Wärmeübertrager W (9) in Betrieb genommen sowie die Zudosierung von inhibierend wirkender Luft und 1,5 Gew.-% MEHQ aufweisender Reinschmelzelösung (Lösung von MEHQ in zuvor entsprechend abgetrenntem Reinprodukt) in den Schmelzkreis hinein. Durch nachfolgende partielle Öffnung des Durchflusses des Auslasses A (3), Inbetriebnahme der Regelung der Temperatur des Schmelzkreises (der zugehörige Temperaturfühler befand sich in Fließrichtung kurz hinter dem Ausgang des Wärmeübertragers W), Inbetriebnahme der Regelung der Position der Aufbaufront (25) (des Standes des Kristallbetts) gemäß WO 2006/111565 und Inbetriebnahme der Regelung der Waschfrontlage (37) (die zugehörige Solltemperatur war 11,0°C) wurde, wie in der Beschreibung beschrieben, das in Betrieb genommene Trennverfahren in einen stationären Betriebszustand überführt, bei dem die Aufbaufront (25) 690 bis 790 mm oberhalb der Messerscheibe (16) und die Waschfront (37) ca. 80 mm unterhalb der Unterkante des Filters F (7) war. Der dazu zugeführte Strom an Suspension S betrug 800 bis 1400 kg/h und der Steuerlaugestrom lag bei 400 bis 1600 kg/h. Über einen Zeitraum von 14 Tagen konnte das Trennverfahren im Wesentlichen störungsfrei weitergeführt werden.

Vergleichsbeispiel 2 (Verfahren der Inbetriebnahme einer hydraulischen Waschkolonne mit L/D= 4,7 bei einer mittleren Filterflächenbelastung von 67 m$^3$/(m$^2$·h)

Es wurde dieselbe hydraulische Waschkolonne wie im Vergleichsbeispiel 1 eingesetzt. Die Herstellung der Suspension S und die Erzeugung der Anfahrflüssigkeit AT erfolgte ebenfalls wie im Vergleichsbeispiel 1 beschrieben.

Das Verfahren der Inbetriebnahme unterschied sich vom Inbetriebnahmeverfahren in Vergleichsbeispiel 1 lediglich dadurch, dass die Förderpumpe P3 (13) bis zum Zeitpunkt ts auf eine Förderleistung von 200 kg/h und die Förderpumpe P2 (8) auf eine Förderleistung von 600 kg/h eingestellt war. Der durch das Filter F (7) strömende Ablaugestrom betrug dabei 610 1/h. Dem entspricht eine (mittlere) Filterflächenbelastung von 67 m$^3$/(m$^2$·h). Nach $t_S$= 27 min (gerechnet ab Beginn der Inbetriebnahme der Förderpumpe P2 (8)) begann der mit dem Membranmanometer M2 erfasste Druck $P_K$ plötzlich zu fallen, während der mit dem Membranmanometer M1 erfasste Druck Pv weiter anstieg, was einem erstmals fallenden Differenzdruck $P_D= P_K-P_V$ entsprach.

Anschließend wurde wie im Vergleichsbeispiel 1 weiterverfahren. Bei der Wiederinbetriebnahme der Förderpumpe P2 (8) wurde deren Förderleistung auf 1000 kg/h und die Förderpumpe P3 (13) auf eine Förderleistung von 800 kg/h eingestellt. Das in Betrieb genommene Trennverfahren wurde reibungslos in einen stationären Betriebszustand überführt, bei dem die Aufbaufront (25) 690 bis 790 mm oberhalb der Messerscheibe (16) und die Waschfront (37) ca. 80 mm unterhalb der Unterkante des Filters F (7) war. Der dazu gehörige Strom an Suspension S betrug 800 bis 1400 kg/h und der Steuerlaugestrom lag bei 400 bis 1600 kg/h. Über einen Zeitraum von 14 Tagen konnte das Trennverfahren im Wesentlichen störungsfrei weitergeführt werden.

Vergleichsbeispiel 3 (Verfahren der Inbetriebnahme einer hydraulischen Waschkolonne mit L/D= 1,07 bei einer mittleren Filterflächenbelastung von 92 m$^3$/(m$^2$·h)

Durch wie in der WO 08/090190 beschrieben durchgeführte fraktionierende Kondensation des Produktgasgemischs einer zweistufigen heterogen katalysierten Gasphasenpartialoxidation von Propylen der Reinheit "chemical grade" wurden über den Seitenabzug der Kondensationskolonne pro Stunde 75 t einer rohen Acrylsäure abgetrennt, die nachfolgende Gehalte aufwies:

| | |
|---|---|
| 96,7716 | Gew.-% Acrylsäure, |
| 0,8253 | Gew.-% Essigsäure, |
| 1,6640 | Gew.-% Wasser, |
| 0,0213 | Gew.-% Ameisensäure, |
| 0,0018 | Gew.-% Formaldehyd, |
| 0,0070 | Gew.-% Acrolein, |
| 0,0681 | Gew.-% Propionsäure, |
| 0,1642 | Gew.-% Furfurale, |
| 0,0027 | Gew.-% Allylacrylat, |
| 0,0012 | Gew.-% Allylformiat, |
| 0,0164 | Gew.-% Benzaldehyd, |
| 0,1052 | Gew.-% Maleinsäureanhydrid, |
| 0,3278 | Gew.-% Diacrylsäure, |
| 0,0050 | Gew.-% Phenothiazin, |
| 0,0180 | Gew.-% MEHQ, und |
| 0,0002 | Gew.-% molekularer Sauerstoff. |

Die via Seitenabzug aus der Kondensationskolonne herausgeführte rohe Acrylsäure wurde mehrstufig durch indirekten Wärmeaustausch (unter anderem wärmeintegriert gegen in die Kondensationskolonne rückgeführte, zuvor wie in diesem Vergleichsbeispiel 3 ausgeführt abgetrennte, Mutterlauge (Ablauge) auf eine Temperatur von 17°C abgekühlt. Dann wurden der abgekühlten rohen Acrylsäure 1230 kg/h Wasser zugemischt, die eine Temperatur von 22°C aufwiesen. Die dabei resultierende "wässrige" rohe Acrylsäure wurde anschließend in drei gleich starke Teilströme aufgeteilt und jeder der drei Teilströme in einen von drei baugleichen, parallel betriebenen Kühlscheibensuspensionskristallisatoren geführt (vgl. WO 2006/111565).

Bei diesen Kristallisatoren handelte es sich jeweils um einen Trog mit einem Fassungsvermögen von 65000 l, in welchem 24 gewischte kreisförmige Kühlscheiben im äquidistanten Abstand von $30 \pm 1$ cm hintereinander hängend angeordnet waren. Ihr Durchmesser betrug einheitlich 3,3 m. Durch die Kühlscheiben wurde als Kälteträger jeweils ein Strom eines Gemischs aus 65 Gew.-% Wasser und 35 Gew.-% Glykol geführt. Die wässrige rohe Acrylsäure und der Kälteträger wurden über den jeweiligen Suspensionskristallisator betrachtet im Gegenstrom durch selbigen geführt. Der Kälteträger wurde dabei jeweils in zwei gleich starke Teilströme aufgeteilt, von denen jeder nur durch die Hälfte der Kühlscheiben des jeweiligen Kristallisators strömte. Dabei wurde so verfahren, dass der jeweilige Teilstrom von der durch ihn durchströmten Kühlscheibe zur übernächsten Kühlscheibe weitergereicht wurde. Ein Teilstrom führte so durch die "geradzahlig" bezifferten Kühlscheiben, während der andere Teilstrom durch die "ungeradzahlig" bezifferten Kühlscheiben strömte (jeweils im Sinne einer Reihenschaltung; die Bezifferung der Kühlscheiben bei der in Strömungsrichtung des Kälteträgers ersten Kühlplatte mit "1" beginnend). Die Stärke des jeweiligen Teilstroms betrug (bezogen auf einen Kristallisator) 95 bis 105 t/h. Die Temperatur des Kälteträgers betrug beim Eintritt in die in Strömungsrichtung jeweils vorderste Kühlscheibe 2,5°C. Die Wanddicke der aus Edelstahl gefertigten Kühlflächen der Kühlscheiben war 4 mm. Durch das Wischen der Kühlscheiben wurde die Ausbildung kristalliner Ablagerungen auf den Kühlflächen unterdrückt.

Die aus den drei Suspensionskristallisatoren jeweils herausgeführte Suspension von Acrylsäurekristallen in Mutterlauge wies eine Temperatur von 7,0 bis 7,1°C und einen Kristallisationsgrad von 0,25 auf. Die Drehzahl der Kühlscheibenwischer betrug 5 Umdrehungen je Minute. Die Wischer waren in radialer Richtung segmentiert (4 Segmente). Als Wischermaterial wurde Ultra High Molecular Weight Polyethylene verwendet.

Im in Förderrichtung der sich ausbildenden Kristallsuspension hintersten Teil des jeweiligen Suspensionskristallisators (hinter der letzten Kühlscheibe) floss die gebildete Kristallsuspension jeweils über ein Überlaufwehr in einen allen drei Kristallisatoren gemeinsamen, gerührten Pufferbehälter (vgl. DE-A 10 2007 043759). Aus diesem Pufferbehälter heraus wurden 33 bis 37 t pro Stunde an Suspension S mit einer Temperatur von 7,4°C in den Verteilerraum einer bereits im stationären Betriebszustand befindlichen ersten hydraulischen Waschkolonne gepumpt, um sie in selbiger einem reinigenden Abtrennverfahren zu unterwerfen.

Ein dem entsprechend verbleibender Reststrom an Suspension S floss in einen beheizten und umgepumpten Auffangbehälter. Diesem Auffangbehälter wurde außerdem die in der ersten hydraulischen Waschkolonne abgetrennte Mutterlauge zugeführt. Ferner wurden im Auffangbehälter die Acrylsäurekristalle der ihm zugeführten Suspension S durch entsprechende Wärmezufuhr wieder aufgeschmolzen, so dass dem Auffangbehälter eine Anfahrflüssigkeit AT entnommen werden konnte, deren Temperatur 18°C betrug. Diese wurde zunächst in ihrem Gesamtmengenstrom oberhalb der Seitenabzugentnahme der rohen Acrylsäure in die Kondensationskolonne rückgeführt. Auf diese Weise stand sowohl eine Anfahrflüssigkeit AT als auch eine Suspension S zur Verfügung, mit der eine zweite hydraulische Waschkolonne in Betrieb genommen werden konnte, deren Design, bis auf das Differenzdruckmanometer M3, das nicht eingebaut war, demjenigen in Figur 2 dieser Schrift entsprach.

Der Innendurchmesser D des kreiszylindrischen Prozessraums (B) betrug 1400 mm. Die Stärke der Mantelwand betrug 10 mm. Fertigungsmaterial war Edelstahl (DIN Werkstoff 1.4571). Die Länge L des Prozessraums (B) betrug 1500 mm (gemessen von der Oberkante der als Abtrageinrichtung (16) eingesetzten Messerscheibe).

Der Prozessraum umfasste 54 baugleiche Filterrohre (6) (aus dem selben Werkstoff wie die Mantelwand gefertigt). Die Wanddicke der kreiszylindrischen Filterrohre (6) war 5 mm. Der Filterrohraußendurchmesser betrug 48 mm. Die Gesamtlänge eines Filterrohres (6) war 1497 mm (einschließlich Verdränger (38)). Davon entfielen 60 mm auf die Höhe der Filter F (6), die sich über den gesamten Filterrohrumfang erstreckten. Die Oberkante eines Filters F (7) war auf einer Filterrohrlänge von 1182 mm (von oben nach unten gemessen). Die Länge des Filterrohrverdrängers (38) erstreckte sich auf 250 mm. Der zentrale zylindrische Verdrängerkörper (43) im Prozessraum (B) hatte einen Außendurchmesser von 350 mm. Er war mit dem Boden B (32) verbunden und dadurch stehend (d.h. nicht rotierend) ausgeführt. Die Anordnung (Verteilung) der Filterrohre (6) im Boden B (32) sowie der Durchgänge U (26) entsprach der Lehre der EP-A 1 448 282. Die Anzahl der Durchgänge U (26) war 78, ihre Länge (vom Verteilerraum bis zum Prozessraum) betrug 600 mm. Sie weisen einen über ihre Länge konstanten kreisförmigen Querschnitt auf, dessen Durchmesser einheitlich 83 mm betrug. Die Höhe des Verteilerraums (A) betrug 1700 mm.

Zunächst wurde die hydraulische Waschkolonne (0) vollständig mit der 18°C aufweisenden Anfahrflüssigkeit AT aus dem Auffangbehälter befüllt (Schmelzkreis (31) + Prozessraum (B) + Verteilerraum (A) + die Förderverbindungen E2 (34), C2 (36), C1 (35) + die Förderpumpe P3 (13)). Die Befüllung erfolgte über den Spülflüssigkeitszuführraum (40) durch die Spülrohre (42) hindurch.

Anschließend wurde die Förderpumpe P3 (13) in Betrieb genommen und ihre Drehzahl so eingestellt, dass sie einen Strom von 30000 kg/h der Anfahrflüssigkeit AT über die Förderverbindung C1 (35) ansaugte und im Kreis "Saugseite der Förderpumpe P3 - Druckseite der Förderpumpe P3 - Förderverbindung C2 (36) - Verteilerraum (A) - Prozessraum (B) - Filter F (7) - Filterrohre (6) - Förderverbindung C1 (35)" förderte (Auslass A (3) war gesperrt).

Dann wurde (bei weiterhin gesperrtem Auslass A (3) sowie nicht in Betrieb befindlicher Abtrageinrichtung (16) (Messerscheibe) und nicht in Betrieb befindlichem Schmelzkreis (31)) die Förderpumpe P2 (8) in Betrieb genommen und mit dieser dem Pufferbehälter über einen Entnahmestutzen 25000 kg/h der Suspension S von Acrylsäurekristallen in Mutterlauge entnommen und mit einer Temperatur von 7,4°C und über die Förderverbindungen E1 (33), E2 (34) zusätzlich zu dem oben genannten Strom von 30000 kg/h in den Verteilerraum (A) der hydraulischen Waschkolonne (0) gepumpt. Bei so in Betrieb befindlichen Förderpumpen P2 (8) und P3 (13) erfolgte (vollzog sich) die Ausbildung des Kristallbetts im Prozessraum der hydraulischen Waschkolonne (0) (18750 kg/h Ablauge strömten aus dem Auslass (2) der Waschkolonnenvorrichtung). Damit einher ging ein zunächst paralleler Anstieg der mit den Membranmanometern M1 (Druck im Verteilerraum (A)) und M2 (Druck im Kristallschmelzeraum (C)) gemessenen Drucke. Der durch die Filter F (7) insgesamt strömende Ablaugestrom betrug dabei 45200 l/h, was einer (mittleren) Filterflächenbelastung von 92 m$^3$/(m$^2$·h) entsprach.

Nach $t_S$= 24 min (gerechnet ab Beginn der Inbetriebnahme der Förderpumpe P2 (8)) begann der mit dem Membranmanometer M2 erfasste Druck $P_K$ plötzlich zu fallen, während der mit dem Membranmanometer M1 erfasste Druck Pv weiter anstieg, was einem erstmals fallenden Differenzdruck $P_D$= $P_K$-$P_V$ entsprach.

Unmittelbar nach erfolgtem Drucksprung wurden die Förderpumpen P2 (8) und P3 (13) (in dieser Reihenfolge) ausgeschaltet und die Förderpumpe P1 (11) sowie die Rotation der Messerscheibe (16) (in dieser Reihenfolge) in Betrieb genommen. Anschließend wurden die Förderpumpen P2 (8) mit einer Förderleistung von 25000 kg/h und die Förderpumpe P3 (13) mit einer Förderleistung von 30000 kg/h wieder in Betrieb genommen, wobei sich das Kristallbett samt Aufbaufront nach unten in Bewegung setzte.

Dann wurde der Wärmeübertrager W (9) in Betrieb genommen sowie die Zudosierung von inhibierend wirkender Luft und 3 Gew.-% MEHQ aufweisender Reinschmelzelösung in den Schmelzkreis hinein. Durch nachfolgende partielle Öffnung des Durchflusses des Auslasses A (3), Inbetriebnahme der Regelung der Temperatur des Schmelzkreises (31) (der zugehörige Temperaturfühler befand sich in Fließrichtung kurz hinter dem Ausgang des Wärmeübertragers W), Inbetriebnahme der Regelung der Position der Aufbaufront gemäß WO 2006/111565 (des Standes des Kristallbetts) und Inbetriebnahme der Regelung der Waschfrontlage (die zugehörige Solltemperatur war 11,2°C) wurde, wie in der Beschreibung beschrieben, das Trennverfahren in einen Betriebszustand mit stationärer Lage von Waschfront und Aufbaufront überführt, bei dem die Aufbaufront 700 bis 1200 mm oberhalb der Messerscheibe (16) und die Waschfront ca. 100 mm unterhalb der Unterkante des Filters F (7) war.

Der dazu gehörige Strom an Suspension S betrug 30000 bis 32000 kg/h und der Steuerlaugestrom lag bei 0 (ausgeschaltete Förderpumpe 3) bis 8000 kg/h.

Dieser Betriebszustand konnte nur über einen Zeitraum von 6,5 Stunden aufrechterhalten werden. Dann brach die zur Druckabsicherung eingefügte Berstscheibe (48), die auf einen Ansprechdruck von 10 bar ausgelegt war. Die Druckmessung M1 zeigte bis dahin zwar ansteigende, aber deutlich unterhalb des vorgenannten Ansprechdrucks von 10 bar liegende Drucke im Bereich < 4,5 bar an.

Eine Analyse der zum Zeitpunkt des Berstscheibenbruchs vorliegenden Druckverhältnisse legte als Ursache des Bruchs einen Verschluss im System der Zuführung der Suspension S in die Waschkolonne nahe.

Vergleichsbeispiel 4 (Verfahren der Inbetriebnahme einer hydraulischen Waschkolonne mit UD= 1,07 bei einer mittleren Filterflächenbelastung von 115 m$^3$/(m2·h)).

Es wurde dieselbe hydraulische Waschkolonne wie im Vergleichsbeispiel 3 eingesetzt, jedoch mit dem Unterschied, dass diese jetzt zusätzlich mit dem Differenzdruckmanometer M3 ausgestattet war. Die Herstellung der Suspension S und die Erzeugung der Anfahrflüssigkeit AT erfolgte ebenfalls wie im Vergleichsbeispiel 3 beschrieben.

Das Verfahren der Inbetriebnahme unterschied sich vom Inbetriebnahmeverfahren in Vergleichsbeispiel 3 lediglich dadurch, dass die Förderpumpe P3 (13) bis zum Zeitpunkt ts auf eine Förderleistung von 40000 kg/h und die Förderpumpe P2 (8) auf eine Förderleistung von 28000 kg/h eingestellt war. Der durch die Filter F (7) insgesamt strömende Ablaugestrom betrug so 56600 l/h. Dem entspricht eine (mittlere) Filterflächenbelastung von 115 m$^3$/(m2·h).

Nach 19 Minuten (gerechnet ab Beginn der Inbetriebnahme der Förderpumpe P2 (8)) begann das Differenzdruckmanometer M3 eine beginnende und ansteigende Druckdifferenz anzuzeigen. Nach weiteren 4 Minuten war der Zeitpunkt $t_S$ erreicht. Der mit dem Membranmanometer M2 erfasste Druck $P_K$ begann plötzlich zu fallen, während der mit dem Membranmanometer M1 erfasste Druck Pv weiter anstieg, was einem erstmals fallenden Differenzdruck $P_D$= $P_K$-$P_V$ entsprach.

Anschließend wurde wie in Vergleichsbeispiel 3 weiterverfahren. Bei der Wiederinbetriebnahme der Förderpumpe P2 (8) wurde deren Förderleistung auf 28000 kg/h und die Förderleistung der Förderpumpe P3 (13) auf 30000 kg/h eingestellt, wobei sich das Kristallbett samt Aufbaufront in Bewegung setzte.

Das wie beschrieben in Betrieb genommene Trennverfahren wurde wie in Vergleichsbeispiel 3 in einen Betriebszustand mit stationärer Lage von Waschfront und Aufbaufront überführt, bei dem die Aufbaufront 700 bis 1200 mm oberhalb der Messerscheibe (16) und die Waschfront ca. 100 mm unterhalb der Unterkante der Filter F (7) war. Der dazugehörige Strom an Suspension S betrug 30000 bis 33000 kg/h und der Steuerlaugestrom lag bei 0 bis 8000 kg/h. Dieser Betriebszustand konnte nur über einen Zeitraum von 5 Stunden aufrechterhalten werden. Innerhalb dieses Zeitraums wies das Differenzdruckmanometer M3 einen zunehmenden Anstieg des Differenzdruckes aus. Dann brach die zur Druckabsicherung eingefügte Berstscheibe (4), die auf einen Ansprechdruck von 10 bar ausgelegt war. Das Membrandruckmanometer M1 zeigte bis dahin zwar ansteigende, aber deutlich unterhalb des vorgenannten Ansprechdrucks von 10 bar liegende Druck im Bereich < 6 bar an.

Der zeitliche Verlauf des mit dem Differenzdruckmanometer M3 verfolgten Differenzdrucks belegt, dass beim Aufbau des Kristallbetts selbiger vor Erreichen des Zeitpunkts ts in den Verteilerraum (A) hineingewachsen ist. Die stetige Zunahme des vorgenannten Differenzdrucks legt eine fortschreitende Verlegung des Verteilerraums (A) sowie eine damit einhergehend zunehmende Verdichtung des in selbigem befindlichen Kristallhaufwerks nahe.

Beispiel

(Erfindungsgemäße Inbetriebnahme einer hydraulischen Waschkolonne mit UD= 1,07 bei einer mittleren Filterflächenbelastung von 39 $m^3/(m^2 \cdot h)$).

Es wurde dieselbe hydraulische Waschkolonne wie im Vergleichsbeispiel 4 eingesetzt. Der Herstellung der Suspension S und die Erzeugung der Anfahrflüssigkeit AT erfolgte wie im Vergleichsbeispiel 3 beschrieben.

Das Verfahren der Inbetriebnahme unterschied sich vom Inbetriebnahmeverfahren in Vergleichsbeispiel 3 lediglich dadurch, dass die Förderpumpe P3 (13) bis zum Zeitpunkt ts außer Betrieb blieb und die Förderpumpe P2 (8) auf eine Förderleistung von 28000 kg/h eingestellt war. Der durch die Filter F insgesamt strömende Ablaugestrom betrug so 19000 l/h. Dem entspricht eine (mittlere) Filterflächenbelastung von 39 $m^3/(m^2 \cdot h)$. Bei alleinig laufender Förderpumpe P2 erfolgte nun der Aufbau des Kristallbetts im Prozessraum. Dies war wie bei den Inbetriebnahmeverfahren der Vergleichsbeispiele am zunächst parallelen Anstieg der mit den Manometern M1 und M2 gemessenen Drücke zu erkennen.

Nach 16 Minuten (gerechnet ab der Inbetriebnahme der Förderpumpe P2 (8)) war im Differenzdruckmembranmanometer M3 eine ansteigende Druckdifferenz zu verzeichnen. Nach weiteren 3 Minuten war der Zeitpunkt $t_S$ erreicht. Der mit dem Membranmanometer M2 erfasste Druck $P_K$ begann plötzlich zu fallen, während der mit dem Membranmanometer M1 erfasste Druck Pv weiter anstieg, was einem erstmals fallenden Differenzdruck $P_D= P_K-P_V$ entsprach.

Anschließend wurde wie in Vergleichsbeispiel 3 weiterverfahren. Bei der Wiederinbetriebnahme der Förderpumpe P2 (8) wurde deren Förderleistung auf 30000 kg/h eingestellt. Zusätzlich zur Wiederinbetriebnahme der Förderpumpe P2 (8) wurde unmittelbar danach jetzt auch die Förderpumpe P3 (13) mit einer Förderleistung von 20000 kg/h in Betrieb genommen, wobei sich das Kristallbett samt Aufbaufront in Bewegung setzte.

Das wie beschrieben in Betrieb genommene Trennverfahren wurde mit den in Vergleichsbeispiel 3 beschriebenen Maßnahmen nachfolgend in einen Betriebszustand mit stationärer Lage von Waschfront und Aufbaufront überführt, bei dem die Aufbaufront 700 bis 1200 mm oberhalb der Messerscheibe (16) und die Waschfront ca. 100 mm unterhalb der Filter F (7) war. Der dazu gehörige Strom an Suspension S betrug 30000 bis 35000 kg/h und der Steuerlaugestrom lag bei 0 bis 12000 kg/h.

Über einen Zeitraum von 21 Tagen wurde das Trennverfahren im Wesentlichen störungsfrei ausgeübt.

Mit Beginn der Wiederinbetriebnahme der Förderpumpe P2 (8) sowie der Inbetriebnahme der Steuerlaugpumpe P3 (18) begann der mit dem Differenzdruckmanometer M3 erfasste Differenzdruck wieder abzunehmen. Im weiteren Verlauf bildete sich der bis zum Zeitpunkt ts mit dem Differenzdruckmanometer M3 beobachtete Differenzdruckanstieg vollständig zurück.

Der zeitliche Verlauf des mit dem Differenzdruckmanometer M3 verfolgten Differenzdrucks belegt, dass beim Aufbau des Kristallbetts selbiges vor Erreichen des Zeitpunktes ts in den Verteilerraum (A) hineingewachsen ist. Die nachfolgende Zurückbildung des Differenzdruckanstiegs belegt, dass bei erfindungsgemäßer Inbetriebnahme diese augenscheinlich nicht vermeidbare Verlegung des Verteilerraums (A) mit Kristallisat jedoch eine reversible ist.

US Provisional Patent Application No. 61/252181, eingereicht am 16. Oktober, 2009 und US Provisional Patent Application No. 61/356078, eingereicht am 18. Juni, 2010 sind in die vorliegende Anmeldung durch Literaturhinweis eingefügt.

Im Hinblick auf die obengenannten lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben ausgeführt werden kann.

Patentansprüche

1. Ein Verfahren der Inbetriebnahme eines Trennverfahrens zur reinigenden Abtrennung von Acrylsäurekristallen aus einer Suspension S ihrer Kristalle in Mutterlauge mit einer Vorrichtung, die eine hydraulische Waschkolonne umfasst, die einen zu seiner von oben nach unten verlaufenden Längsachse rotationssymmetrischen Prozessraum aufweist, der von einer zylindrischen Mantelwand und zwei auf der Symmetrieachse einander gegenüberliegenden Enden begrenzt wird, wobei

- sich vom oberen Ende des Prozessraums parallel zu dessen Längsachse ein oder mehrere Filterrohre durch den Prozessraum erstrecken, die auf das dem oberen Ende gegenüberliegende untere Ende des Prozessraums zulaufen, und in der dem unteren Ende des Prozessraums zugewandten Hälfte des Prozessraums wenigstens ein Filter F, das die einzige direkte Verbindung zwischen dem jeweiligen Filterrohrinneren und dem Prozessraum bildet, aufweisen sowie außerhalb des Prozessraums aus der Waschkolonne herausgeführt werden,
- der Quotient Q = L/D aus dem Abstand L zwischen dem oberen und dem unteren Ende des Prozessraums und dem Durchmesser D des Prozessraums 0,3 bis 4 beträgt,
- sich am unteren Ende des Prozessraums nach unten gerichtet der Kristallschmelzeraum der Waschkolonne anschließt, wobei zwischen den beiden Räumen eine rotationsfähige Abtrageinrichtung integriert und durch den Kristallschmelzeraum ein Kristallschmelzekreis hindurchgeführt ist, der außer dem Kristallschmelzeraum

- eine außerhalb der Waschkolonne befindliche Förderpumpe P1, die eine Saug- und eine Druckseite aufweist,
- eine erste Förderverbindung G1, die vom Kristallschmelzeraum der Waschkolonne zur Saugseite der Förderpumpe P1 führt,
- eine zweite Förderverbindung G2, die von der Druckseite der Förderpumpe P1 in den Kristallschmelzeraum der Waschkolonne zurückführt und einen Auslass A aus dem Kristallschmelzekreis mit regelbarem Durchfluss aufweist, sowie
- einen Wärmeübertrager W, über den entweder die Förderverbindung G1 vom Kristallschmelzeraum zur Saugseite der Förderpumpen P1, oder die Förderverbindung G2 von der Druckseite der Förderpumpe P1 zum Kristallschmelzeraum geführt ist, umfasst,

- dem oberen Ende des Prozessraums nach oben gerichtet ein Verteilerraum vorgelagert ist, der vom Prozessraum wenigstens durch einen Boden B getrennt ist, welcher Durchgänge U aufweist, die auf der dem Prozessraum zugewandten Seite des Bodens B in den Prozessraum und auf der vom Prozessraum abgewandten Seite des Bodens B in den Verteilerraum führen,
- sich außerhalb der Waschkolonne eine Förderpumpe P2, die eine Saug- und eine Druckseite aufweist, und eine Quelle QS der Suspension S befinden, wobei
- eine erste Förderverbindung E1 von der Quelle QS zur Saugseite der Förderpumpe P2, und
- eine zweite Förderverbindung E2 von der Druckseite der Förderpumpe P2 in den Verteilerraum führt,
- sich außerhalb der Waschkolonne gegebenenfalls eine Förderpumpe P3, die eine Saug- und eine Druckseite aufweist, und eine Quelle QT einer Steuerlauge befinden, wobei
- eine erste Förderverbindung C1 von der Saugseite der Pumpe P3 zur Quelle QT, und
- eine zweite Förderverbindung C2 von der Druckseite der Pumpe P3 in den Verteilerraum und/oder in den zwischen seinem oberen Ende und den Filtern F der Filterrohre gelegenen Längsabschnitt des Prozessraums führt,

und wobei man bei der Durchführung des Trennverfahrens in seinem stationären Betrieb

- mit der Pumpe P2 kontinuierlich einen Strom ST der Suspension S von der Quelle QS durch die Förderverbindungen E1, E2 über den Verteilerraum und durch die Durchgänge U hindurch in den Prozessraum der Waschkolonne führt,
- gegebenenfalls mit der Pumpe P3 einen Strom SL der Steuerlauge von der Quelle QT durch die Förderverbindungen C1, C2 über den Verteilerraum und die Durchgänge U hindurch und/oder direkt in den Prozessraum der Waschkolonne führt,
- über die Filter F der Filterrohre insgesamt einen Strom SM umfassend Mutterlauge und gegebenenfalls Steuerlauge als Ablaugestrom in das Filterrohrinnere hinein- und über die Filterrohre aus der Waschkolonne herausführt, und diesen aus der Waschkolonne herausgeführten Ablaugestrom SM als die Quelle QT für die Steuerlauge verwendet,
- durch die Mutter- und gegebenenfalls Steuerlaugeführung im Prozessraum der Waschkolonne die Ausbildung eines Kristallbetts an Acrylsäurekristallen aufrechterhält, das eine dem oberen Ende des Prozessraums zugewandte Aufbaufront aufweist, an der sich kontinuierlich Kristalle des zugeführten Stroms ST der Suspension S ans Kristallbett anlagern,
- das Kristallbett durch die aus dem hydraulischen Strömungsdruckverlust der Mutter- und gegebenenfalls Steuerlaugeführung im Prozessraum resultierende Kraft von oben nach unten an den Filtern F vorbei auf die rotierende Abtrageinrichtung zu fördert,
- mit der rotierenden Abtrageinrichtung vom auf sie stoßenden Kristallbett Acrylsäurekristalle abträgt,

- den Strom der abgetragenen Acrylsäurekristalle durch die rotierende Abtrageinrichtung hindurch und/oder an der rotierenden Abtrageinrichtung vorbei in den sich in Förderrichtung des Kristallbetts an den Prozessraum anschließenden Kristallschmelzeraum fördert, und in dem durch den Kristallschmelzeraum geführten Kristallschmelzekreis durch Eintrag von Wärme mit dem Wärmeübertrager W zu einem Kristallschmelzestrom aufschmilzt, und

- den Durchfluss des Auslasses A so regelt, dass, bezogen auf die Stärke des vorgenannten Kristallschmelzestroms, vom Kristallschmelzeraum ausgehend ein Teilstrom an Kristallschmelze als Waschschmelzestrom durch die rotierende Abtrageinrichtung hindurch und/oder an der rotierenden Abtrageinrichtung vorbei gegen die Bewegungsrichtung des Kristallbetts in den Prozessraum zurückströmt, wo er im abwärts geförderten Kristallbett aufsteigt und dabei von den Kristallen die im Kristallbett verbliebene und mit diesem unter die Filter F geförderte Mutterlauge abwäscht und zurückdrängt, wobei sich im von den Filtern F bis zum unteren Ende des Prozessraums erstreckenden Längsabschnitt des Prozessraums im Kristallbett eine Waschfront ausbildet, die das Kristallbett von oben nach unten in eine Mutterlaugezone und in eine Waschschmelzezone aufteilt, und der restliche Teilstrom des vorgenannten Kristallschmelzestroms den Kristallschmelzekreis durch den Auslass A verlässt,

**dadurch gekennzeichnet, dass** man bei der Inbetriebnahme des Trennverfahrens zur erstmaligen Ausbildung des Kristallbetts im Prozessraum

- den den Kristallschmelzeraum umfassenden Kristallschmelzekreis und den Prozessraum der zuvor unbefüllten Waschkolonne mit einer Acrylsäure enthaltenden Anfahrflüssigkeit AT zunächst so befüllt, dass die Füllhöhe der Anfahrflüssigkeit AT im Prozessraum wenigstens die Abtrageinrichtung überragt,

- anschließend die Befüllung der Waschkolonne fortsetzt, indem man mit der Pumpe P2 einen Strom ST* der Suspension S von der Quelle QS durch die Förderverbindungen E1, E2 über den Verteilerraum und durch die Durchgänge U hindurch in den Prozessraum der Waschkolonne und von dem dabei durch die Filterrohre aus der Waschkolonne herausgeführten Ablaugestrom SM* als Quelle QT* gegebenenfalls mit der Pumpe P3 einen Teilstrom als Steuerlaugestrom SL* durch die Förderverbindungen C1, C2 über den Verteilerraum und die Durchgänge U hindurch und/oder direkt in den Prozessraum der Waschkolonne führt, und dies wenigstens so lange, bis der Zeitpunkt $t_S$ erreicht ist, bei dem der Differenzdruck $P_D = P_K - P_V$, wobei $P_K$ der an einer beliebig ausgewählten Stelle im Kristallschmelzeraum zu einem bestimmten Zeitpunkt der Zufuhr des Stroms ST* jeweils bestehende Druck und $P_V$ der zum jeweils gleichen Zeitpunkt an einer beliebig ausgewählten Stelle im Verteilerraum jeweils bestehende Druck ist, in Abhängigkeit von der Dauer der Zufuhr des Stroms ST* nicht mehr ansteigt oder konstant bleibt, sondern plötzlich abnimmt, und dies mit der Maßgabe, dass

- bis zum Zeitpunkt $t_S$ die mittlere Flächenbelastung der Filter F, berechnet als der arithmetische Mittelwert des während der Zufuhr des Stroms ST* durch die Filter F der Filterrohre zum jeweiligen Zeitpunkt insgesamt strömenden Ablaugestroms SM*, geteilt durch die Gesamtfläche aller Filter F, nicht mehr als 80 m$^3$/(m$^2$·h) beträgt,

- die Acrylsäure enthaltende Anfahrflüssigkeit AT eine solche ist, aus der beim Abkühlen bis zur einsetzenden Kristallisation die sich bei der Kristallisation bildenden Kristalle Acrylsäurekristalle sind, und

- zwischen der in Grad Celsius angegebenen Kristallbildungstemperatur $T^{KB}$ dieser Acrylsäurekristalle in der Anfahrflüssigkeit AT und der in Grad Celsius angegebenen Temperatur $T^S$ der Suspension S des Stroms ST* die Beziehung

$$T^{KB} \leq T^S + 15°C$$

erfüllt ist.

2. Verfahren nach Anspruch 1, **dadurch gekennzeichnet, dass** bis zum Zeitpunkt $t_S$ die mittlere Flächenbelastung der Filter F wenigstens 5 m$^3$/(m$^2$·h) beträgt.

3. Verfahren nach einem der Ansprüche 1 oder 2, **dadurch gekennzeichnet, dass** der Abstand L $\geq$ 0,5 m beträgt.

4. Verfahren nach einem der Ansprüche 1 bis 3, **dadurch gekennzeichnet, dass** der Abstand L $\leq$ 5 m beträgt.

5. Verfahren nach einem der Ansprüche 1 bis 4, **dadurch gekennzeichnet, dass** $T^{KB}$ nicht mehr als 20°C unterhalb von $T^S$ liegt.

6. Verfahren nach einem der Ansprüche 1 bis 5, **dadurch gekennzeichnet, dass** die Anfahrflüssigkeit AT von der Suspension S abgetrennte Mutterlauge ist.

7. Verfahren nach einem der Ansprüche 1 bis 6, **dadurch gekennzeichnet, dass** die Anfahrflüssigkeit AT diejenige Flüssigkeit ist, aus der durch Abkühlen die Suspension S erzeugt wird.

8. Verfahren nach einem der Ansprüche 1 bis 7, **dadurch gekennzeichnet, dass** wenigstens während 50% des Zeitraums, gerechnet vom Beginn der Zufuhr des Stroms ST* der Suspension S bis zum Erreichen des Zeitpunkts $t_s$, die zum jeweiligen Zeitpunkt bestehende Flächenbelastung der Filter F nicht mehr als 80 $m^3/(m^2 \cdot h)$ beträgt.

9. Verfahren nach einem der Ansprüche 1 bis 8, **dadurch gekennzeichnet, dass** während des gesamten Zeitraums, gerechnet vom Beginn der Zufuhr des Stroms ST* der Suspension S bis zum Erreichen des Zeitpunkts ts, die zum jeweiligen Zeitpunkt bestehende Flächenbelastung der Filter F nicht mehr als 80 $m^3/(m^2 \cdot h)$ beträgt.

10. Verfahren nach einem der Ansprüche 1 bis 9, **dadurch gekennzeichnet, dass** wenigstens während 50% des Zeitraums, gerechnet vom Beginn der Zufuhr des Stroms ST* der Suspension S bis zum Erreichen des Zeitpunkts ts, die zum jeweiligen Zeitpunkt bestehende Flächenbelastung der Filter F wenigstens 5 $m^3/(m^2 \cdot h)$ oder wenigstens 10 $m^3/(m^2 \cdot h)$ beträgt.

11. Verfahren nach einem der Ansprüche 1 bis 10, **dadurch gekennzeichnet, dass** während des gesamten Zeitraums, gerechnet vom Beginn der Zufuhr des Stroms ST* der Suspension S bis zum Erreichen des Zeitpunkts ts, der arithmetische Mittelwert M des dem Prozessraum der Waschkolonne insgesamt zugeführten Stroms an Flüssigkeit, geteilt durch die freie Querschnittsfläche des Prozessraums 1 bis 30 $m^3/(m^2 \cdot h)$ beträgt.

12. Verfahren nach einem der Ansprüche 1 bis 11, **dadurch gekennzeichnet, dass** wenigstens während 50% des Zeitraums, gerechnet vom Beginn der Zufuhr des Stroms ST* der Suspension S bis zum Erreichen des Zeitpunkts $t_S$, der dem Prozessraum der Waschkolonne zum jeweiligen Zeitpunkt insgesamt zugeführte Strom an Flüssigkeit, geteilt durch die freie Querschnittsfläche des Prozessraums 1 bis 30 $m^3/(m^2 \cdot h)$ oder 5 bis 25 $m^3/(m^2 \cdot h)$ beträgt.

13. Verfahren nach einem der Ansprüche 1 bis 12, **dadurch gekennzeichnet, dass** der Gehalt der Suspension S an Acrylsäure ≥ 70 Gew.-% beträgt.

14. Verfahren nach einem der Ansprüche 1 bis 13, **dadurch gekennzeichnet, dass** der Kristallisationsgrad der Suspension S ≥ 0,10 beträgt.

15. Verfahren nach einem der Ansprüche 1 bis 14, **dadurch gekennzeichnet, dass** die Längstausdehnung der in der Suspension S enthaltenen Acrylsäurekristalle mehrheitlich 50 bis 1600 $\mu$m beträgt.

16. Verfahren nach einem der Ansprüche 1 bis 15, **dadurch gekennzeichnet, dass** das Öffnungsverhältnis OV der Abtrageinrichtung ≥ 0,01 beträgt.

17. Verfahren nach einem der Ansprüche 1 bis 16, **dadurch gekennzeichnet, dass** das Öffnungsverhältnis OV der Abtrageinrichtung ≤ 0,9 beträgt.

18. Verfahren nach einem der Ansprüche 1 bis 17, **dadurch gekennzeichnet, dass** die Abtrageinrichtung eine Durchgangsöffnungen aufweisende Messerscheibe ist.

19. Verfahren nach einem der Ansprüche 1 bis 18, **dadurch gekennzeichnet, dass** man den Kristallschmelzekreis und den Prozessraum der zuvor unbefüllten Waschkolonne mit einer Acrylsäure enthaltenden Anfahrflüssigkeit AT zunächst so befüllt, dass die Füllhöhe der Anfahrflüssigkeit AT im Prozessraum wenigstens die Filter F überragt.

20. Verfahren nach einem der Ansprüche 1 bis 19, **dadurch gekennzeichnet, dass** die Temperatur der Suspension S -25 bis +14°C beträgt.

21. Verfahren nach einem der Ansprüche 1 bis 20, **dadurch gekennzeichnet, dass** die in der Suspension S enthaltene Mutterlauge ≥ 70 Gew. an Acrylsäure enthält.

22. Verfahren nach einem der Ansprüche 1 bis 21, **dadurch gekennzeichnet, dass** von dem durch die Filterrohre aus der Waschkolonne herausgeführten Ablaugestrom SM* als Quelle QT* mit der Pumpe P3 ein Teilstrom als Steuerlaugestrom SL* durch die Förderverbindungen C1, C2 über den Verteilerraum und durch die Durchgänge U hindurch in den Prozessraum der Waschkolonne geführt wird.

23. Verfahren nach einem der Ansprüche 1 bis 22, **dadurch gekennzeichnet, dass** während der Inbetriebnahme sowohl der Druck $P_K$ als auch der Druck Pv gemessen wird.

24. Verfahren nach einem der Ansprüche 1 bis 23, **dadurch gekennzeichnet, dass** der Differenzdruck $P_D$ mit einem Differenzdruckmanometer bestimmt wird.

**25.** Verfahren nach einem der Ansprüche 1 bis 24, **dadurch gekennzeichnet, dass** nach dem Zeitpunkt ts der Schmelzkreis und die Abtrageinrichtung in Betrieb genommen und der Durchfluss des Auslasses A geöffnet wird, sowie dem Schmelzkreis ein molekularen Sauerstoff enthaltendes Gas zudosiert wird, in dem in einen Teilstrom des Schmelzkreises das molekularen Sauerstoff enthaltende Gas eingebracht, und anschließend der den molekularen Sauerstoff enthaltende Teilstrom wieder dem Schmelzkreis zugeführt wird.

**26.** Verfahren nach einem der Ansprüche 1 bis 25, **dadurch gekennzeichnet, dass** die Anzahl der Filterrohre in der hydraulischen Waschkolonne 3 bis 200 beträgt.

**27.** Verfahren nach einem der Ansprüche 1 bis 26, **dadurch gekennzeichnet, dass** die Suspension S nachfolgende Gehalte aufweist.

$\geq$ 70 Gew.-% Acrylsäure,
bis zu 15 Gew.-% Essigsäure,
bis zu 5 Gew.-% Propionsäure,
bis zu 5 Gew.-% niedermolekulare Aldehyde,
bis zu 3 Gew.-% Diacrylsäure, und
bis zu 25 Gew.-% Wasser.

**28.** Trennverfahren zur reinigenden Abtrennung von Acrylsäurekristallen aus einer Suspension S ihrer Kristalle in Mutterlauge mit einer Vorrichtung, die eine hydraulische Waschkolonne umfasst, **dadurch gekennzeichnet, dass** das Trennverfahren gemäß einem Verfahren der Ansprüche 1 bis 27 in Betrieb genommen worden ist.

**29.** Verfahren nach Anspruch 28, **dadurch gekennzeichnet, dass** sich ein weiteres Verfahren anschließt, bei dem abgetrenntes und aufgeschmolzenes Acrylsäurekristallisat einer Polymerisation mit sich oder anderen wenigstens einfach ethylenisch ungesättigten Verbindungen unterworfen wird.

**Claims**

**1.** A process for starting up a separating process for purifying removal of acrylic acid crystals from a suspension S of crystals thereof in mother liquor with an apparatus which comprises a hydraulic wash column which has a process space which is rotationally symmetric with respect to its longitudinal axis running from the top downward and is bounded by a cylindrical outer wall and two opposite ends on the axis of symmetry, in which

- one or more filter tubes extend through the process space from the upper end of the process space parallel to the longitudinal axis thereof, which run toward the lower end of the process space opposite the upper end, and have, in the half of the process space toward the lower end of the process space, at least one filter F which constitutes the only direct connection between the particular filter tube interior and the process space, and are conducted out of the wash column outside the process space,
- the quotient Q = L/D of the distance L between the upper and lower ends of the process space and the diameter D of the process space is 0.3 to 4,
- the lower end of the process space is followed in the downward direction by the crystal melt space of the wash column, a rotatable removal device being integrated between the two spaces and a crystal melt circuit which is conducted through the crystal melt space comprising, outside the crystal melt space,
- a delivery pump P1 which is outside the wash column and has a suction side and a pressure side,
- a first delivery connection G1 which leads from the crystal melt space of the wash column to the suction side of the delivery pump P1,
- a second delivery connection G2 which leads from the pressure side of the delivery pump P1 back into the crystal melt space of the wash column and has an outlet A from the crystal melt circuit with regulable flow, and
- a heat transferer W, through which either the delivery connection G1 from the crystal melt space to the suction side of the delivery pump P1 or the delivery connection G2 from the pressure side of the delivery pump P1 to the crystal melt space is conducted,
- connected upstream of the upper end of the process space in the upward direction is a distributor space which is separated from the process space at least by one end B which has passages U which lead into the process space on the side of the end B facing the process space and into the distributor space on the side of the end B facing away from the process space,
- a delivery pump P2 which has a suction side and a pressure side and a source QS of the suspension S are present outside the wash column,
- a first delivery connection E1 leading from the source QS to the suction side of the delivery pump P2, and
- a second delivery connection E2 leading from the pressure side of the delivery pump P2 into the distributor space,
- a delivery pump P3 which has a suction side and a pressure side and a source QT of a control liquor are optionally present outside the wash column,

- a first delivery connection C1 leading from the suction side of the pump P3 to the source QT, and
- a second delivery connection C2 leading from the pressure side of the pump P3 into the distributor space and/or into the longitudinal section of the process space between the upper end thereof and the filters F of the filter tubes,

and in which, in the course of performance of the separating process, in steady-state operation thereof,

- the pump P2 is used to continuously conduct a stream ST of the suspension S from the source QS through the delivery connections E1, E2 via the distributor space and through the passages U into the process space of the wash column,
- optionally, the pump P3 is used to conduct a stream SL of the control liquor from the source QT through the delivery connections C1, C2 via the distributor space and the passages U and/or directly into the process space of the wash column,
- overall, a stream SM comprising mother liquor and optionally control liquor is conducted as waste liquor stream into the filter tube interior via the filters F of the filter tubes, and out of the wash column via the filter tubes, and this waste liquor stream SM conducted out of the wash column is used as the source QT for the control liquor,
- the conduction of mother liquor and optionally control liquor in the process space of the wash column maintains the development of a crystal bed of acrylic acid crystals which has a buildup front facing the upper end of the process space, at which crystals of the stream ST of the suspension S supplied are added continuously onto the crystal bed,
- the crystal bed is conveyed from the top downward past the filters F toward the rotating removal device by the force which results from the hydraulic flow pressure drop of the conduction of mother liquor and optionally control liquor in the process space,
- the rotating removal device removes acrylic acid crystals from the crystal bed which meets it,
- the stream of the acrylic acid crystals removed is conveyed through the rotating removal device and/or past the rotating removal device into the crystal melt space which follows downstream of the process space in conveying direction of the crystal bed, and melted in the crystal melt circuit conducted through the crystal melt space as a result of introduction of heat with the heat transferer W to give a crystal melt stream, and
- the flow through the outlet A is regulated such that, based on the flow rate of the aforementioned crystal melt stream, proceeding from the crystal melt space, a substream of crystal melt flows as wash melt stream through the rotating removal device and/or past the rotating removal device against the direction of movement of the crystal bed back into the process space, where it ascends within the crystal bed conveyed downward and in doing so washes the mother liquor off the crystals and forces it back, said mother liquor remaining in the crystal bed having been conveyed with the latter under the filters F, which forms, in the longitudinal section of the process space which extends from the filters F to the lower end of the process space, in the crystal bed, a wash front which divides the crystal bed, from the top downward, into a mother liquor zone and into a wash melt zone, and the remaining substream of the aforementioned crystal melt stream leaves the crystal melt circuit through the outlet A,

wherein, in the course of startup of the separating process for first development of the crystal bed in the process space,

- the crystal melt circuit comprising the crystal melt space, and the process space of the previously unfilled wash column, are at first filled with an acrylic acid-comprising startup liquid AT such that the fill height of the startup liquid AT in the process space overtops at least the removal device,
- then the filling of the wash column is continued by using the pump P2 to conduct a stream ST* of the suspension S from the source QS through the delivery connections E1, E2 via the distributor space and through the passages U into the process space of the wash column and optionally using the pump P3 to conduct a substream of the waste liquor stream SM* conducted out of the wash column through the filter tubes as source QT*, as control liquor stream SL* through the delivery connections C1, C2 via the distributor space and the passages U and/or directly into the process space of the wash column, at least until the time $t_s$ is attained at which the pressure difference $P_D = P_K - P_V$, where $P_K$ is the pressure existing at any desired point in the crystal melt space at a particular time in the supply of the stream ST* and $P_V$ is the pressure which exists at the same time at any desired point in the distributor space, no longer rises or remains constant as a function of the duration of the supply of the stream ST*, but decreases suddenly, with the proviso that
- until the time $t_s$, the mean superficial velocity on the filters F, calculated as the arithmetic mean of the total waste liquor flow SM* which flows at the particular time through the filters F of the filter tubes during the supply of the stream ST*, divided by the total area of all filters F, is not more than 80 m$^3$/ (m$^2$ ·h),

- the acrylic acid-comprising startup liquid AT is one from which, in the course of cooling until crystallization sets in, the crystals which form in the course of crystallization are acrylic acid crystals, and

- between the crystal formation temperature T$^{KB}$, reported in degrees Celsius, of these acrylic acid crystals in the startup liquid AT and the temperature T$^S$, reported in degrees Celsius, of the suspension S of the stream ST*, the following relationship is satisfied:

$$T^{KB} \leq T^S + 15°C .$$

2. The process according to claim 1, wherein the mean superficial velocity on the filters F until the time $t_s$ is not more than 5 m$^3$/(m$^2$ · h).

3. A process according to either of claims 1 and 2, wherein the distance L is ≥ 0.5 m.

4. A process according to any of claims 1 to 3, wherein the distance L is ≤ 5 m.

5. A process according to any of claims 1 to 4, wherein T$^{KB}$ is not more than 20°C below T$^S$.

6. A process according to any of claims 1 to 5, wherein the startup liquid AT is mother liquor removed from the suspension S.

7. A process according to any of claims 1 to 6, wherein the startup liquid AT is that liquid from which the suspension S is obtained by cooling.

8. A process according to any of claims 1 to 7, wherein, at least over 50% of the period calculated from the commencement of supply of the stream ST* of the suspension S until the time $t_s$ is reached, the superficial velocity on the filters F which exists at the particular time is not more than 80 m$^3$/(m$^2$ · h).

9. A process according to any of claims 1 to 8, wherein, over the entire period calculated from the commencement of supply of the stream ST* of the suspension S until the time $t_s$ is reached, the superficial velocity on the filters F which exists at the particular time is not more than 80 m$^3$/(m$^2$· h).

10. A process according to any of claims 1 to 9, wherein, at least over 50% of the period calculated from the commencement of supply of the stream ST* of the suspension S until the time $t_s$ is reached, the superficial velocity on the filters F which exists at the particular time is at least 5 m$^3$/(m$^2$·h) or at least 10 m$^3$/(m$^2$·h).

11. A process according to any of claims 1 to 10, wherein, over the entire period calculated from the commencement of supply of the stream ST* of the suspension S until the time $t_s$ is reached, the arithmetic mean M of the total flow of liquid supplied to the process space of the wash column, divided by the free cross-sectional area of the process space, is 1 to 30 m$^3$/(m$^2$·h).

12. A process according to any of claims 1 to 11, wherein, at least over 50% of the period calculated from the commencement of supply of the stream ST* of the suspension S until the time $t_s$ is reached, the total flow of liquid supplied to the process space of the wash column at the particular time, divided by the free cross-sectional area of the process space, is 1 to 30 m$^3$/(m$^2$·h) or 5 to 25 m$^3$/(m$^2$·h).

13. A process according to any of claims 1 to 12, wherein content of acrylic acid in the suspension S is ≥70% by weight.

14. A process according to any of claims 1 to 13, wherein the degree of crystallization of the suspension S is ≥ 0.10.

15. A process according to any of claims 1 to 14, wherein the longest dimension of the majority of acrylic acid crystals present in the suspension S is 50 to 1600 μm.

16. A process according to any of claims 1 to 15, wherein the orifice ratio OV of the removal device its ≥ 0.01.

17. A process according to any of claims 1 to 16, wherein the orifice ratio OV of the removal device is ≤ 0.9.

18. A process according to any of claims 1 to 17, wherein the removal device is a bladed disk having passage orifices.

19. A process according to any of claims 1 to 18, wherein the crystal melt circuit and the process space of the previously unfilled wash column are at first filled with an acrylic acid-comprising startup liquid AT such that the fill height of the startup liquid AT in the process space overtops at least the filters F.

20. A process according to any of claims 1 to 19, wherein the temperature of the suspension S is -25 to +14°C.

21. A process according to any of claims 1 to 20, wherein the mother liquor present in the suspension S comprises ≥ 70% by weight of acrylic acid.

22. A process according to any of claims 1 to 21, wherein a substream of the waste liquor stream SM* conducted out of the wash column through the filter tubes as source QT* is conducted with the pump P3 as control liquor stream SL* through the delivery connections C1, C2 via the distributor space and through the passages U into the process space of the wash column.

23. A process according to any of claims 1 to 22, wherein both the pressure $P_K$ and the pressure $P_V$ are measured during the startup.

24. A process according to any of claims 1 to 23, wherein the pressure difference $P_D$ is determined with a pressure difference manometer.

25. A process according to any of claims 1 to 24, wherein, after the time $t_s$, the melt circuit and the removal device are put into operation and the flow through the outlet A is opened, and a molecular oxygen-comprising gas is metered into the melt circuit, by introducing the molecular oxygen-comprising gas into a substream of the melt circuit, and then supplying the substream comprising the molecular oxygen back to the melt circuit.

26. A process according to any of claims 1 to 25, wherein the number of filter tubes in the hydraulic wash column is 3 to 200.

27. A process according to any of claims 1 to 26, wherein the suspension S has the following contents:

≥ 70% by weight of acrylic acid,
up to 15% by weight of acetic acid,
up to 5% by weight of propionic acid,
up to 5% by weight of low molecular weight aldehydes,
up to 3% by weight of diacrylic acid, and
up to 25% by weight of water.

28. A separating process for purifying removal of acrylic acid crystals from a suspension S of crystals thereof in mother liquor with an apparatus which comprises a hydraulic wash column, wherein the separating process has been put into operation by a process of claims 1 to 27.

29. A process according to claim 28, which is followed by a further process in which removed and molten acrylic acid crystals are subjected to a polymerization with acrylic acid itself or other, at least monoethylenically unsaturated compounds.

**Revendications**

1. Procédé de mise en marche d'un procédé de séparation pour la séparation lavante de cristaux d'acide acrylique d'une suspension S de ses cristaux dans une liqueur mère avec un dispositif comprenant une colonne de lavage hydraulique, qui comprend une chambre de traitement symétrique par rotation par rapport à son axe longitudinal passant du haut vers le bas, qui est délimitée par une paroi d'enveloppe cylindrique et deux extrémités opposées l'une à l'autre sur l'axe de symétrie,

- un ou plusieurs tubes filtrants s'étendant dans la chambre de traitement de l'extrémité supérieure de la chambre de traitement parallèlement à son axe longitudinal, qui se dirigent vers l'extrémité inférieure de la chambre de traitement opposée à l'extrémité supérieure et qui comprennent, dans la moitié de la chambre de traitement orientée vers l'extrémité inférieure de la chambre de traitement, au moins un filtre F, qui constitue la seule connexion directe entre l'intérieur respectif des tubes filtrants et la chambre de traitement, et qui sortent de la colonne de lavage à l'extérieur de la chambre de traitement,
- le quotient Q = L/D entre la distance L entre l'extrémité supérieure et l'extrémité inférieure de la chambre de traitement et le diamètre D de la chambre de traitement étant de 0,3 à 4,
- la chambre de fusion de cristaux de la colonne de lavage s'ensuivant vers le bas à l'extrémité inférieure de la chambre de traitement, un dispositif de déchargement rotatif étant intégré entre les deux chambres et un circuit de masse fondue cristalline traversant la chambre de fusion de cristaux, qui comprend en dehors de la chambre de fusion de cristaux,

- une pompe de transport P1 se trouvant à l'extérieur de la colonne de lavage, qui comprend un côté d'aspiration et un côté de refoulement,
- une première jonction de transport G1, qui conduit de la chambre de fusion de cristaux de la colonne de lavage vers le côté d'aspiration de la pompe de transport P1,
- une seconde jonction de transport G2, qui recycle du côté de refoulement de la pompe de transport P1 dans la chambre de fusion de cristaux de la colonne de lavage et comprend une sortie A du circuit de masse fondue cristalline à débit réglable, et
- un échangeur de chaleur W, par lequel passe soit la jonction de transport G1 de la chambre de fusion de cristaux vers le côté d'aspiration de la pompe de transport P1, soit la jonction de transport G2 du côté de refoulement de la pompe de transport P1 vers la chambre de fusion de cristaux,

- une chambre de distribution étant située en amont de l'extrémité supérieure de la chambre de traitement vers le haut, qui est séparée de la chambre de traitement par au moins un plateau B qui comprend des passages U, qui conduisent du côté du plateau B orienté vers la chambre de traitement dans la chambre de traitement et du côté du plateau B opposé à la chambre de traitement dans la chambre de distribution,
- une pompe de transport P2, qui comprend un côté d'aspiration et un côté de refoulement, et une source QS de la suspension S se trouvant à l'extérieur de la colonne de lavage,
- une première jonction de transport E1 conduisant de la source QS vers le côté d'aspiration de la pompe de transport P2, et
- une seconde jonction de transport E2 conduisant du côté de refoulement de la pompe de transport P2 dans la chambre de distribution,
- une pompe de transport P3, qui comprend un côté d'aspiration et un côté de refoulement, et une source QT d'une ligueur de contrôle se trouvant à l'extérieur de la colonne de lavage,
- une première jonction de transport C1 conduisant du côté d'aspiration de la pompe P3 vers la source QT, et
- une seconde jonction de transport C2 conduisant du côté de refoulement de la pompe P3 vers la chambre de distribution et/ou vers la section longitudinale de la chambre de traitement située entre son extrémité supérieure et les filtres F des tubes filtrants,
et, lors de la réalisation du procédé de séparation en son mode stationnaire,
- un courant ST de la suspension S étant conduit en continu par la pompe P2 de la source QS par le biais des jonctions de transport E1, E2 au travers de la chambre de distribution et des passages U dans la chambre de traitement de la colonne de lavage,
- un courant SL de la liqueur de contrôle étant éventuellement conduit par la pompe P3 de la source QT par le biais des jonctions de transport C1, C2 au travers de la chambre de distribution et des passages U et/ou directement dans la chambre de traitement de la colonne de lavage,
- un courant SM comprenant au total la liqueur mère et éventuellement la liqueur de contrôle en tant que courant de liqueur usagée étant introduit par le biais des filtres F des tubes filtrants à l'intérieur des tubes filtrants et déchargés de la colonne de lavage par le biais des tubes filtrants, et ce courant de liqueur usagée SM déchargé de la colonne de lavage étant utilisé en tant que source QT pour la liqueur de contrôle,
- la formation d'un lit cristallin de cristaux d'acide acrylique étant maintenue par l'introduction de la liqueur mère et éventuellement de la liqueur de contrôle dans la chambre de traitement de la colonne de lavage, qui comprend un front de formation orienté vers l'extrémité supérieure de la chambre de traitement, sur lequel des cristaux du courant ST de la suspension S introduit s'accumulent en continu sur le lit cristallin,
- le lit cristallin étant transporté par la force résultant de la perte de charge d'écoulement hydraulique de l'introduction de liqueur mère et éventuellement de liqueur de contrôle dans la chambre de traitement du haut vers le bas au-delà des filtres F sur le dispositif de déchargement rotatif,
- des cristaux d'acide acrylique étant déchargés par le dispositif de déchargement rotatif du lit cristallin poussé sur celui-ci,
- le courant de cristaux d'acide acrylique déchargés étant transporté au travers du dispositif de déchargement rotatif et/ou au-delà du dispositif de déchargement rotatif dans la chambre de fusion de cristaux suivant la chambre de traitement dans la direction de transport du lit cristallin, et fondu en un courant de masse fondue cristalline dans le circuit de masse fondue cristalline traversant la chambre de fusion de cristaux par apport de chaleur avec l'échangeur de chaleur W, et
- le débit de la sortie A étant ajustée de manière à ce que, par rapport à la puissance du courant de masse fondue cristalline susmentionné, un courant partiel de masse fondue cristalline reflue à partir de la chambre de fusion de cristaux en tant que courant de masse fondue de lavage au travers du dispositif de déchargement rotatif et/ou au-delà du dispositif de déchargement rotatif contre la direction de mouvement du lit cristallin dans la chambre de traitement, où il monte dans le lit cristallin transporté en aval et ainsi lave et refoule des cristaux la liqueur mère restant dans le lit cristallin et transportée avec celui-ci sous le filtre F, un front de lavage se formant dans le lit cristallin dans la section longitudinale de la chambre de traitement s'étendant des filtres F jusqu'à l'extrémité inférieure de la chambre de traitement, qui divise le lit cristallin du haut vers le bas en une zone de liqueur mère et en une zone de masse fondue de lavage, et le courant partiel restant du courant de masse fondue cristalline susmentionné quittant le circuit de masse fondue cristalline par la sortie A,
**caractérisé en ce que** lors de la mise en marche du procédé de séparation pour la première formation du lit cristallin dans la chambre de traitement,

- le circuit de masse fondue cristalline comprenant la chambre de fusion de cristaux et la chambre de traitement de la colonne de lavage auparavant non remplie sont tout d'abord remplis avec un liquide de démarrage AT contenant de l'acide acrylique, de manière à ce que la hauteur de remplissage du liquide de démarrage AT dans la chambre de traitement dépasse au moins le dispositif de déchargement,

- puis le remplissage de la colonne de lavage est poursuivi par conduction avec la pompe P2 d'un courant ST* de la suspension S de la source QS par le biais des jonctions de transport E1, E2 au travers de la chambre de distribution et des passages U dans la chambre de traitement de la colonne de lavage et éventuellement avec la pompe P3 d'un courant partiel du courant de liqueur usagée SM* déchargé par les tubes filtrants de la colonne de lavage en tant que source QT* en tant que courant de liqueur de contrôle SL* par le biais des jonctions de transport C1, C2 au travers de la chambre de distribution et des passages U et/ou directement dans la chambre de traitement de la colonne de lavage, et ceci au moins jusqu'à atteindre le temps $t_s$ auquel la différence de pression $P_D = P_K - P_V$, $P_K$ étant la pression existant à chaque fois à un emplacement quelconque de la chambre de fusion de cristaux à un temps déterminé de l'introduction du courant ST* et $P_V$ étant la pression existant à chaque fois au même temps à un emplacement quelconque de la chambre de distribution, n'augmente plus ou ne reste plus constante en fonction de la durée de l'introduction du courant ST*, mais chute plutôt soudainement, et ceci à condition que

- jusqu"au temps $t_s$, la charge surfacique moyenne des filtres F, calculée en tant que moyenne arithmétique du courant de liqueur usagée SM* traversant au total les filtres F des tubes filtrants jusqu'au temps respectif pendant l'introduction du courant ST*, divisée par la surface totale de tous les filtres F, ne soit pas supérieure à 80 m$^3$/(m$^2$·h),

- le liquide de démarrage AT contenant de l'acide acrylique soit tel que les cristaux qui se forment lors de la cristallisation lors du refroidissement jusqu'au début de la cristallisation soient des cristaux d'acide acrylique, et

- la relation suivante entre la température de formation de cristaux $T^{KB}$ donnée en degrés Celsius de ces cristaux d'acide acrylique dans le liquide de démarrage AT et la température $T^S$ de la suspension S du courant ST* donnée en degrés Celsius :

$$T^{KB} \leq T^S + 15 \ °C$$

soit satisfaite.

2. Procédé selon la revendication 1, **caractérisé en ce que** la charge surfacique moyenne des filtres F jusqu'au temps $t_s$ est d'au moins 5 m$^3$/(m$^2$·h).

3. Procédé selon l'une quelconque des revendications 1 ou 2, **caractérisé en ce que** la distance L est $\geq$ 0,5 m.

4. Procédé selon l'une quelconque des revendications 1 à 3, **caractérisé en ce que** la distance L est $\leq$ 5 m.

5. Procédé selon l'une quelconque des revendications 1 à 4, **caractérisé en ce que** $T^{KB}$ n'est pas située plus de 20 °C en dessous de $T^S$.

6. Procédé selon l'une quelconque des revendications 1 à 5, **caractérisé en ce que** le liquide de démarrage AT est la liqueur mère séparée de la suspension S.

7. Procédé selon l'une quelconque des revendications 1 à 6, **caractérisé en ce que** le liquide de démarrage AT est le liquide à partir duquel la suspension S est formée par refroidissement.

8. Procédé selon l'une quelconque des revendications 1 à 7, **caractérisé en ce que** pendant au moins 50 % de la période, calculée à partir du début de l'introduction du courant ST* de la suspension S jusqu'à l'atteinte du temps $t_s$, la charge surfacique des filtres F existant au temps respectif n'est pas supérieure à 80 m$^3$/(m$^2$·h).

9. Procédé selon l'une quelconque des revendications 1 à 8, **caractérisé en ce que** pendant l'ensemble de la période, calculée à partir du début de l'introduction du courant ST* de la suspension S jusqu'à l'atteinte du temps $t_s$, la charge surfacique des filtres F existant au temps respectif n'est pas supérieure à 80 m$^3$/(m$^2$·h).

10. Procédé selon l'une quelconque des revendications 1 à 9, **caractérisé en ce que** pendant au moins 50 % de la période, calculée à partir du début de l'introduction du courant ST* de la suspension S jusqu'à l'atteinte du temps $t_s$, la charge surfacique des filtres F existant au temps respectif est d'au moins 5 m$^3$/(m$^2$·h) ou d'au moins 10 m$^3$/(m$^2$·h).

11. Procédé selon l'une quelconque des revendications 1 à 10, **caractérisé en ce que** pendant l'ensemble de la période, calculée à partir du début de l'introduction du courant ST* de la suspension S jusqu'à l'atteinte du temps $t_s$, la moyenne arithmétique M du courant de liquide introduit au total dans la chambre de traitement de la colonne de lavage, divisée par la surface de section libre de la chambre de traitement, est de 1 à 30 m$^3$/(m$^2$·h).

12. Procédé selon l'une quelconque des revendications 1 à 11, **caractérisé en ce que** pendant au moins 50 % de la période, calculée à partir du début de l'introduction du courant ST* de la suspension S jusqu'à l'atteinte du temps $t_s$, le courant de liquide introduit au total dans la chambre de traitement de la colonne de lavage au temps respectif, divisé par la surface de section libre de la chambre de traitement, est de 1 à 30 m$^3$/(m$^2$·h) ou de 5 à 25 m$^3$/(m$^2$·h).

13. Procédé selon l'une quelconque des revendications 1 à 12, **caractérisé en ce que** la teneur de la suspension S en acide acrylique est $\geq$ 70 % en poids.

14. Procédé selon l'une quelconque des revendications 1 à 13, **caractérisé en ce que** le degré de cristallisation de la suspension S est $\geq$ 0,10.

15. Procédé selon l'une quelconque des revendications 1 à 14, **caractérisé en ce que** la dimension longitudinale des cristaux d'acide acrylique contenus dans la suspension S est majoritairement de 50 à 1 600 $\mu$m.

16. Procédé selon l'une quelconque des revendications 1 à 15, **caractérisé en ce que** le rapport d'ouverture OV du dispositif de déchargement est $\geq$ 0,01.

17. Procédé selon l'une quelconque des revendications 1 à 16, **caractérisé en ce que** le rapport d'ouverture OV du dispositif de déchargement est $\leq$ 0,9.

18. Procédé selon l'une quelconque des revendications 1 à 17, **caractérisé en ce que** le dispositif de déchargement est un disque coupant comprenant des ouvertures de passage.

19. Procédé selon l'une quelconque des revendications 1 à 18, **caractérisé en ce que** le circuit de masse fondue cristalline et la chambre de traitement de la colonne de lavage auparavant non remplie sont tout d'abord remplis avec un liquide de démarrage AT contenant de l'acide acrylique de manière à ce que la hauteur de remplissage du liquide de démarrage AT dans la chambre de traitement dépasse au moins les filtres F.

20. Procédé selon l'une quelconque des revendications 1 à 19, **caractérisé en ce que** la température de la suspension S est de -25 à +14 °C.

21. Procédé selon l'une quelconque des revendications 1 à 20, **caractérisé en ce que** la liqueur mère contenue dans la suspension S contient $\geq$ 70 % en poids d'acide acrylique.

22. Procédé selon l'une quelconque des revendications 1 à 21, **caractérisé en ce qu'**à partir du courant de liqueur usagée SM* déchargé par les tubes filtrants de la colonne de lavage en tant que source QT*, un courant partiel est conduit par la pompe P3 en tant que courant de liqueur de contrôle SL* par le biais des conduites de transport C1, C2 au travers de la chambre de distribution et des passages U dans la chambre de traitement de la colonne de lavage.

23. Procédé selon l'une quelconque des revendications 1 à 22, **caractérisé en ce qu'**aussi bien la pression $P_K$ que la pression $P_V$ sont mesurées pendant la mise en marche.

24. Procédé selon l'une quelconque des revendications 1 à 23, **caractérisé en ce que** la pression différentielle $P_D$ est déterminée avec un manomètre de pression différentielle.

25. Procédé selon l'une quelconque des revendications 1 à 24, **caractérisé en ce qu'**après le temps $t_s$, le circuit de masse fondue et le dispositif de déchargement sont mis en marche et le passage de l'ouverture A est ouvert, et un gaz contenant de l'oxygène moléculaire est introduit dans le circuit de masse fondue par introduction du gaz contenant de l'oxygène moléculaire dans un courant partiel du circuit de masse fondue, puis réintroduction du courant partiel contenant l'oxygène moléculaire dans le circuit de masse fondue.

26. Procédé selon l'une quelconque des revendications 1 à 25, **caractérisé en ce que** le nombre de tubes filtrants dans la colonne de lavage hydraulique est de 3 à 200.

27. Procédé selon l'une quelconque des revendications 1 à 26, **caractérisé en ce que** la suspension S présente les teneurs suivantes :

$\geq$ 70 % en poids d'acide acrylique,
jusqu'à 15 % en poids d'acide acétique,
jusqu'à 5 % en poids d'acide propionique,
jusqu'à 5 % en poids d'aldéhydes de faible poids moléculaire,
jusqu'à 3 % en poids d'acide diacrylique et
jusqu'à 25 % en poids d'eau.

28. Procédé de séparation pour la séparation lavante de cristaux d'acide acrylique d'une suspension S de ses cristaux dans une liqueur mère avec un dispositif, qui comprend une colonne de lavage hydraulique, **caractérisé en ce que** le procédé de séparation a été mis en marche par un procédé selon les revendications 1 à 27.

29. Procédé selon la revendication 28, **caractérisé en ce qu'**un procédé supplémentaire s'ensuit, selon lequel les cristaux d'acide acrylique séparés et fondus sont soumis à une polymérisation avec eux-mêmes ou d'autres composés au moins monoéthyléniquement insaturés.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007043758 A
- DE 102007043748 A
- DE 102007004960 A
- DE 102007043759 A
- DE 102009000987
- WO 2006111565 A
- EP 1448282 A
- US 2009018347 A
- WO 03041832 A
- WO 0177056 A
- WO 0435514 A
- WO 0341833 A
- WO 029839 A
- DE 10036881 A
- WO 0255469 A
- WO 0378378 A
- DE 10228859 A
- DE 102008054587
- DE 102007028333 A
- DE 102007028332 A
- EP 1079194 A
- US 6382313 B
- EP 1232004 A
- WO 0132301 A
- WO 0341832 A
- DE 102006045089 A
- DE 10211290 A
- EP 492400 A
- WO 0209839 A
- WO 08090190 A
- WO 2004035514 A
- DE 10243625 A
- DE 10323758 A
- WO 2001077056 A
- US 61252181 B
- US 61356078 B